(19) **Europäisches Patentamt / European Patent Office / Office européen des brevets**

(11) **EP 4 736 775 A1**

(12) **EUROPEAN PATENT APPLICATION**
published in accordance with Art. 153(4) EPC

(43) Date of publication:
06.05.2026 Bulletin 2026/19

(21) Application number: 24832057.4

(22) Date of filing: 27.06.2024

(51) International Patent Classification (IPC):
A61B 7/04 (2006.01)          A61B 5/00 (2006.01)
A61B 7/02 (2006.01)          G01H 17/00 (2006.01)

(52) Cooperative Patent Classification (CPC):
A61B 7/02; A61B 5/00; A61B 7/04; G01H 9/00;
G01H 17/00

(86) International application number:
PCT/JP2024/023412

(87) International publication number:
WO 2025/005199 (02.01.2025 Gazette 2025/01)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 28.06.2023  JP 2023106205
22.11.2023  JP 2023198526
06.12.2023  JP 2023206277
07.12.2023  JP 2023207172
13.12.2023  JP 2023210285
18.12.2023  JP 2023213194
18.12.2023  JP 2023213195
19.12.2023  JP 2023214037
29.03.2024  JP 2024057616
10.04.2024  JP 2024063542
15.04.2024  JP 2024065612

(71) Applicant: Canon Kabushiki Kaisha
Tokyo, 146-8501 (JP)

(72) Inventors:
• KOJIMA, Keizo
  Tokyo 146-8501 (JP)
• SAMEJIMA, Keisuke
  Tokyo 146-8501 (JP)
• YAMANAKA, Hiromichi
  Tokyo 146-8501 (JP)
• WATANABE, Shinri
  Tokyo 146-8501 (JP)
• MIYASHITA, Takuya
  Tokyo 146-8501 (JP)
• YAMAGUCHI, Noritomo
  Tokyo 146-8501 (JP)
• SASAOKA, Osamu
  Tokyo 146-8501 (JP)
• NIHEI, Hironobu
  Tokyo 146-8501 (JP)

(74) Representative: TBK
Bavariaring 4-6
80336 München (DE)

(54) **ELECTRONIC DEVICE**

(57) An electronic device includes a diaphragm having a contact surface configured to contact a subject and a reflective medium on a surface of the diaphragm opposite the contact surface, the diaphragm being configured to elastically deform when pressed by the subject at the contact surface; a light-emitting diode; an aperture configured to narrow light emitted by the light-emitting diode; a photodetector having a light-receiving surface configured to receive light that passes through the aperture and is specularly reflected by the reflective medium; an output unit configured to output a signal based on light in an illuminated region formed on the light-receiving surface by the reflected light; and a housing enclosing the light-emitting diode, the aperture, and the photodetector, wherein the diaphragm is configured to form part of an exterior of the electronic device together with the housing, and wherein a boundary of the illuminated region, the boundary being defined by the light narrowed by the aperture and specularly reflected by the reflective medium, moves in accordance with displacement of the contact surface caused by elastic deformation of the diaphragm, thereby changing an area of the illuminated region on the light-receiving surface and changing an output of the output unit.

F I G. 2A

# FIG. 2B

# FIG. 2C

**Description**

TECHNICAL FIELD

The present invention relates to an electronic device.

BACKGROUND ART

Electronic devices such as electronic stethoscopes, which have sensors for measuring vibrations in target bodies and are capable of obtaining body sounds by the sensors, have become widespread in recent years. Patent Literature (PTL) 1 proposes an electronic stethoscope that obtains body sounds using a condenser microphone. PTL 2 proposes an auscultation apparatus that uses a vibration sensor in an obtainment unit that obtains body sounds.

CITATION LIST

PATENT LITERATURE

PTL 1: Japanese Patent Laid-Open No. 2022-119446
PTL 2: Japanese Patent Laid-Open No. 2017-47095

SUMMARY OF INVENTION

TECHNICAL PROBLEM

With a microphone having a size that fits in an auscultation apparatus such as that described in PTL 1, it is difficult to accurately detect vibrations produced by the target body in low-frequency bands around 10 Hz. In an auscultation apparatus that detects vibrations of a target body using a vibration sensor such as that described in PTL 2, a constant pressure is applied by pressing the vibration sensor against the surface of the target body, which reduces the amount of displacement of the surface of the target body and worsens the signal-to-noise ratio (SNR). With the conventional electronic devices, it has been difficult to accurately measure vibrations in a subject such as a target body. Some aspects of the present invention provide a technique for accurately measuring vibrations in a subject.

SOLUTION TO PROBLEM

According to some embodiments, an electronic device comprising: a diaphragm having a contact surface configured to contact a subject and a reflective medium on a surface of the diaphragm opposite the contact surface, the diaphragm being configured to elastically deform when pressed by the subject at the contact surface; a light-emitting diode; an aperture configured to narrow light emitted by the light-emitting diode; a photodetector having a light-receiving surface configured to receive light that passes through the aperture and is specularly reflected by the reflective medium; an output unit configured to output a signal based on light in an illuminated region formed on the light-receiving surface by the reflected light; and a housing enclosing the light-emitting diode, the aperture, and the photodetector, wherein the diaphragm is configured to form part of an exterior of the electronic device together with the housing, and wherein a boundary of the illuminated region, the boundary being defined by the light narrowed by the aperture and specularly reflected by the reflective medium, moves in accordance with displacement of the contact surface caused by elastic deformation of the diaphragm, thereby changing an area of the illuminated region on the light-receiving surface and changing an output of the output unit, is provided.

ADVANTAGEOUS EFFECTS OF INVENTION

According to the foregoing embodiment, displacement in a subject can be accurately measured.
Other features and advantages of the present invention will be apparent from the following description taken in conjunction with the accompanying drawings. Note that throughout the accompanying drawings, identical reference numerals denote identical or similar components.

BRIEF DESCRIPTION OF DRAWINGS

The accompanying drawings, which are incorporated in and constitute a part of the specification, illustrate embodiments of the invention and, together with the description, serve to explain principles of the invention.

FIG. 1A is a schematic diagram illustrating an example of the external appearance of an electronic stethoscope according to a first embodiment.

FIG. 1B is a schematic diagram illustrating an example of the external appearance of the electronic stethoscope according to the first embodiment.

FIG. 2A is a schematic diagram illustrating an example configuration of a chest piece according to the first embodiment.

FIG. 2B is a schematic diagram illustrating a variation on the chest piece according to the first embodiment.

FIG. 2C is a schematic diagram illustrating a variation on the chest piece according to the first embodiment.

FIG. 3A is a schematic diagram illustrating an example of operations of the chest piece according to the first embodiment.

FIG. 3B is a schematic diagram illustrating an example of operations of the chest piece according to the first embodiment.

FIG. 4A is a schematic diagram illustrating an example of movement of reflected light according to the first embodiment.

FIG. 4B is a schematic diagram illustrating an example of movement of reflected light according to the first embodiment.

FIG. 4C is a schematic diagram illustrating an example of movement of reflected light according to the first embodiment.

FIG. 4D is a schematic diagram illustrating an example of movement of reflected light according to the first embodiment.

FIG. 4E is a schematic diagram illustrating an example of movement of reflected light according to the first embodiment.

FIG. 4F is a schematic diagram illustrating an example of movement of reflected light according to the first embodiment.

FIG. 5 is a diagram illustrating a relationship between a displacement amount and a displacement signal according to the first embodiment.

FIG. 6 is a block diagram illustrating an example configuration of an electronic stethoscope according to the first embodiment.

FIG. 7A is a schematic diagram illustrating an example of operations of the chest piece according to the first embodiment.

FIG. 7B is a schematic diagram illustrating an example of operations of the chest piece according to the first embodiment.

FIG. 7C is a schematic diagram illustrating an example of operations of the chest piece according to the first embodiment.

FIG. 7D is a schematic diagram illustrating an example of operations of the chest piece according to the first embodiment.

FIG. 7E is a schematic diagram illustrating an example of a change in a light receiving range according to the first embodiment.

FIG. 8A is a schematic diagram illustrating a first variation on the chest piece according to the first embodiment.

FIG. 8B is a schematic diagram illustrating the first variation on the chest piece according to the first embodiment.

FIG. 8C is a schematic diagram illustrating a second variation on the chest piece according to the first embodiment.

FIG. 8D is a schematic diagram illustrating the second variation on the chest piece according to the first embodiment.

FIG. 8E is a diagram illustrating a relationship between a displacement amount and a displacement signal in the second variation.

FIG. 9A is a schematic diagram illustrating a third variation on the chest piece according to the first embodiment.

FIG. 9B is a schematic diagram illustrating the third variation on the chest piece according to the first embodiment.

FIG. 9C is a diagram summarizing the configuration of the first embodiment and each variation thereon.

FIG. 10 is a block diagram illustrating an example configuration of an electronic stethoscope according to a second embodiment.

FIG. 11A is a schematic diagram illustrating an example configuration of a chest piece according to the second embodiment.

FIG. 11B is a schematic diagram illustrating an example configuration of the chest piece according to the second embodiment.

FIG. 11C is a schematic diagram illustrating an example configuration of the chest piece according to the second embodiment.

FIG. 12A is a schematic diagram illustrating an example configuration of the chest piece according to the second embodiment.

FIG. 12B is a schematic diagram illustrating an example configuration of the chest piece according to the second embodiment.

FIG. 13A is a schematic diagram illustrating an example configuration of the chest piece according to the second embodiment.

FIG. 13B is a schematic diagram illustrating an example configuration of the chest piece according to the second embodiment.

FIG. 13C is a schematic diagram illustrating an example configuration of the chest piece according to the second embodiment.

FIG. 13D is a schematic diagram illustrating an example configuration of the chest piece according to the second embodiment.

FIG. 13E is a schematic diagram illustrating an example configuration of the chest piece according to the second embodiment.

FIG. 13F is a schematic diagram illustrating an example configuration of the chest piece according to the second embodiment.

FIG. 13G is a schematic diagram illustrating an example configuration of the chest piece according to the second embodiment.

FIG. 13H is a schematic diagram illustrating an example configuration of the chest piece according to the second embodiment.

FIG. 13I is a schematic diagram illustrating an example configuration of the chest piece according to the second embodiment.

FIG. 13J is a schematic diagram illustrating an example configuration of the chest piece according to the second embodiment.

FIG. 13K is a schematic diagram illustrating an example configuration of the chest piece according to the second embodiment.

FIG. 14A is a schematic diagram illustrating a variation on the chest piece according to the second embodiment.

FIG. 14B is a schematic diagram illustrating a variation on the chest piece according to the second embodiment.

FIG. 14C is a schematic diagram illustrating a variation on the chest piece according to the second embodiment.

FIG. 15A is a schematic diagram illustrating a variation on the chest piece according to the second embodiment.

FIG. 15B is a schematic diagram illustrating a variation on the chest piece according to the second embodiment.

FIG. 15C is a schematic diagram illustrating a variation on the chest piece according to the second embodiment.

FIG. 16A is a schematic diagram illustrating a variation on the chest piece according to the second embodiment.

FIG. 16B is a schematic diagram illustrating a variation on the chest piece according to the second embodiment.

FIG. 16C is a schematic diagram illustrating a variation on the chest piece according to the second embodiment.

FIG. 17A is a block diagram illustrating an example of the circuit configuration of the electronic stethoscope according to the second embodiment.

FIG. 17B is a diagram illustrating an effect achieved by the example of the circuit configuration of the electronic stethoscope according to the second embodiment.

FIG. 17C is a diagram illustrating an effect achieved by the example of the circuit configuration of the electronic stethoscope according to the second embodiment.

FIG. 17D is a diagram illustrating an effect achieved by the example of the circuit configuration of the electronic stethoscope according to the second embodiment.

FIG. 17E is a diagram illustrating an effect achieved by the example of the circuit configuration of the electronic stethoscope according to the second embodiment.

FIG. 17F is a diagram illustrating an effect achieved by the example of the circuit configuration of the electronic stethoscope according to the second embodiment.

FIG. 17G is a diagram illustrating an effect achieved by the example of the circuit configuration of the electronic stethoscope according to the second embodiment.

FIG. 17H is a diagram illustrating an effect achieved by the example of the circuit configuration of the electronic stethoscope according to the second embodiment.

FIG. 18 is a block diagram illustrating an example of the functional configuration of the electronic stethoscope according to the second embodiment.

FIG. 19A is a flowchart illustrating an example of operations by the electronic stethoscope according to the second embodiment.

FIG. 19B is a flowchart illustrating an example of operations by the electronic stethoscope according to the second embodiment.

FIG. 20 is a schematic diagram illustrating an example of operations by the electronic stethoscope according to the second embodiment.

FIG. 21 is a flowchart illustrating an example of operations by the electronic stethoscope according to the second embodiment.

FIG. 22 is a schematic diagram illustrating an example of operations by the electronic stethoscope according to the second embodiment.

FIG. 23 is a schematic diagram illustrating an example of a display by the electronic stethoscope according to the second embodiment.

FIG. 24A is a schematic diagram illustrating an example of a variation on the electronic stethoscope according to the second embodiment.

FIG. 24B is a schematic diagram illustrating an example of a variation on the electronic stethoscope according to the second embodiment.

FIG. 24C is a schematic diagram illustrating an example of a variation on the electronic stethoscope according to the second embodiment.

FIG. 25A is a schematic diagram illustrating an example of a variation on the electronic stethoscope according to the second embodiment.

FIG. 25B is a schematic diagram illustrating an example of a variation on the electronic stethoscope according to the second embodiment.

FIG. 26A is a state transition diagram illustrating an example of state transitions in the electronic stethoscope according to the second embodiment.

FIG. 26B is a flowchart illustrating an example of state transitions in the electronic stethoscope according to the second embodiment.

FIG. 26C is a flowchart illustrating an example of state transitions in the electronic stethoscope according to the second embodiment.

FIG. 27A is a schematic diagram illustrating an example of operations by the electronic stethoscope according to the second embodiment.

FIG. 27B is a schematic diagram illustrating an example of operations by the electronic stethoscope according to the second embodiment.

FIG. 28A is a schematic diagram illustrating an example configuration of an electronic stethoscope according to a third embodiment.

FIG. 28B is a schematic diagram illustrating an example configuration of the electronic stethoscope according to the third embodiment.

FIG. 28C is a schematic diagram illustrating an example configuration of the electronic stethoscope according to the third embodiment.

FIG. 28D is a schematic diagram illustrating an example configuration of the electronic stethoscope according to the third embodiment.

FIG. 29A is a schematic diagram illustrating an example configuration of the electronic stethoscope according to the third embodiment.

FIG. 29B is a schematic diagram illustrating an example configuration of the electronic stethoscope according to the third embodiment.

FIG. 29C is a schematic diagram illustrating an example configuration of the electronic stethoscope according to the third embodiment.

FIG. 29D is a schematic diagram illustrating an example configuration of the electronic stethoscope according to the third embodiment.

FIG. 30 is a schematic diagram illustrating an example configuration of the electronic stethoscope according to the third embodiment.

FIG. 31A is a schematic diagram illustrating an example of a variation on the electronic stethoscope according to the third embodiment.

FIG. 31B is a schematic diagram illustrating an example of a variation on the electronic stethoscope according to the third embodiment.

FIG. 31C is a schematic diagram illustrating an example of a variation on the electronic stethoscope according to the third embodiment.

FIG. 32A is a schematic diagram illustrating an example configuration of an electronic stethoscope according to a fourth embodiment.

FIG. 32B is a schematic diagram illustrating an example configuration of the electronic stethoscope according to the fourth embodiment.

FIG. 32C is a schematic diagram illustrating an example configuration of the electronic stethoscope according to the fourth embodiment.

FIG. 32D is a schematic diagram illustrating an example configuration of the electronic stethoscope according to the fourth embodiment.

FIG. 33A is a schematic diagram illustrating an example configuration of the electronic stethoscope according to the fourth embodiment.

FIG. 33B is a schematic diagram illustrating an example configuration of the electronic stethoscope according to the fourth embodiment.

FIG. 33C is a schematic diagram illustrating an example configuration of the electronic stethoscope according to the fourth embodiment.

FIG. 33D is a schematic diagram illustrating an example configuration of the electronic stethoscope according to the fourth embodiment.

FIG. 34A is a schematic diagram illustrating an example of a variation on the electronic stethoscope according to the fourth embodiment.

FIG. 34B is a schematic diagram illustrating an example of a variation on the electronic stethoscope according to the fourth embodiment.

FIG. 34C is a schematic diagram illustrating an example of a variation on the electronic stethoscope according to the fourth embodiment.

FIG. 34D is a schematic diagram illustrating an example of a variation on the electronic stethoscope according to the fourth embodiment.

FIG. 35A is a schematic diagram illustrating an example of a variation on the electronic stethoscope according to the fourth embodiment.

FIG. 35B is a schematic diagram illustrating an example of a variation on the electronic stethoscope according to the fourth embodiment.

FIG. 35C is a schematic diagram illustrating an example of a variation on the electronic stethoscope according to the fourth embodiment.

FIG. 35D is a schematic diagram illustrating an example of a variation on the electronic stethoscope according to the fourth embodiment.

FIG. 36A is a schematic diagram illustrating an example of a variation on the electronic stethoscope according to the fourth embodiment.

FIG. 36B is a schematic diagram illustrating an example of a variation on the electronic stethoscope according to the fourth embodiment.

FIG. 36C is a schematic diagram illustrating an example of a variation on the electronic stethoscope according to the fourth embodiment.

FIG. 36D is a schematic diagram illustrating an example of a variation on the electronic stethoscope according to the fourth embodiment.

FIG. 37A is a schematic diagram illustrating an example of a variation on the electronic stethoscope according to the fourth embodiment.

FIG. 37B is a schematic diagram illustrating an example of a variation on the electronic stethoscope according to the fourth embodiment.

FIG. 37C is a schematic diagram illustrating an example of a variation on the electronic stethoscope according to the fourth embodiment.

FIG. 37D is a schematic diagram illustrating an example of a variation on the electronic stethoscope according to the fourth embodiment.

DESCRIPTION OF EMBODIMENTS

Hereinafter, embodiments will be described in detail with reference to the attached drawings. Note, the following embodiments are not intended to limit the scope of the claimed invention. Multiple features are described in the embodiments, but limitation is not made to an invention that requires all such features, and multiple such features may be combined as appropriate. Furthermore, in the attached drawings, the same reference numerals are given to the same or similar configurations, and redundant description thereof is omitted.

First Embodiment

External Appearance of Electronic Auscultation Apparatus of First Embodiment

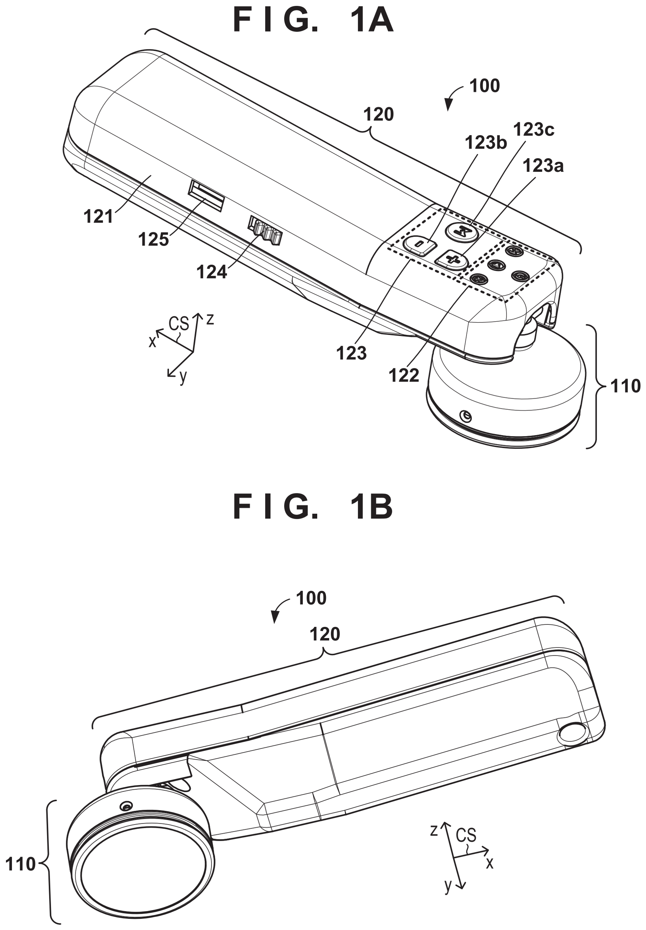

The external appearance of an electronic stethoscope 100 according to a first embodiment will be described with reference to FIGS. 1A to 9B. Note that each of the following drawings may use a coordinate system CS, which is a three-dimensional Cartesian coordinate system having an x-axis, a y-axis, and a z-axis, to indicate directions. In such descriptions, the positive direction in the z-axis may be expressed as the "upper" side, and the negative direction in the z-axis may be expressed as the "lower" side. FIG. 1A illustrates the external appearance of the electronic stethoscope 100 when viewed from one direction, and FIG. 1B illustrates the external appearance of the electronic stethoscope 100 when viewed from another direction. The electronic stethoscope 100 is an electronic device used as a diagnostic instrument for listening to internal sounds of target bodies such as humans or animals. The electronic stethoscope 100 is mainly used for listening to cardiac sounds and respiratory sounds.

As illustrated in FIG. 1A, the electronic stethoscope 100 includes a chest piece 110 and a grip 120. The chest piece 110 is a unit that, when making a diagnosis using the electronic stethoscope 100, contacts a surface of the target body, which is an example of a subject, measures minute vibrations (displacements) in the surface of the target body, and captures body sounds. The chest piece 110 detects the minute displacements in the surface of the compact target body via a diaphragm 206 (described later). As such, the chest piece 110 can also be referred to as a "displacement detection apparatus" or a "diaphragm displacement detection apparatus". Because it is used to detect vibrations on the surface of a target body, the chest piece 110 can also be referred to as a "body-surface vibration detection device".

The grip 120 is gripped by a user of the electronic stethoscope 100 (e.g., a doctor, a nurse, a public health nurse) when the diaphragm 206 is pressed to the surface of the target body. The user of the electronic stethoscope 100 will be called simply a "user" hereinafter. As illustrated in FIGS. 1A and 1B, the grip 120 has a rod shape, and the chest piece 110 is attached to one end thereof (in the negative direction of the x-axis in FIGS. 1A and 1B). The grip 120 is called a "grip", a "bar", a "handle", or the like. In the present embodiment, the grip 120 and the chest piece 110 are configurations that is pivotable relative to each other, but the present invention is not limited thereto, and the configuration may be such that the chest piece 110 is fixed to the grip 120, or such that the chest piece 110 serves as a grip.

The grip 120 has a housing 121. The grip 120 houses a battery and a circuit board within the housing 121. The battery stores operating power of the electronic stethoscope 100. The circuit board has circuit elements for controlling the operations of the electronic stethoscope 100. The grip 120 includes a display device 122, an operation unit 123, a power switch 124, and a connector 125 provided on an outer surface of the housing 121.

The display device 122 displays the state of the electronic stethoscope 100. For example, the display device 122 may include a plurality of indicators (four indicators, in the example in FIG. 1A). Each indicator is constituted by a light-emitting diode (LED). The plurality of indicators include an indicator that indicates whether the electronic stethoscope 100 is powered on or off. The plurality of indicators also include an indicator that indicates the current operating mode of the electronic stethoscope 100. The plurality of indicators also include an indicator that indicates whether the electronic stethoscope 100 is wirelessly connected to an external device. The plurality of indicators also include an indicator that indicates whether the chest piece 110 is pressed against the surface of a target body. As illustrated in FIG. 1A, the display device 122 is disposed on the outer surface of the housing 121, in the vicinity of the chest piece 110 in the one end in the x-axis direction, but on the side opposite from the chest piece 110. In the present embodiment, the "vicinity of the chest piece 110" means being closer to the chest piece 110 than the center of the grip 120. With such an arrangement, when the user grips the other end side of the grip 120 in the x-axis direction, the display device 122 disposed on one end side in the x-axis direction is not hidden by the user's hand. As a result, the user can easily view the display device 122. Note that the display device 122 does not need to include all of the aforementioned indicators, and the status of the electronic stethoscope 100 may be displayed by a liquid crystal panel or an electrostatic panel instead of or in addition to the plurality of indicators.

The operation unit 123 accepts operations by the user. In the present embodiment, the operation unit 123 includes a plurality of physical buttons (three buttons, in the example in FIG. 1A) for accepting settings for the electronic stethoscope 100. Specifically, the operation unit 123 includes volume adjustment buttons (a volume up button 123a and a volume down button 123b) for adjusting the volume of sounds that are output. By pressing the volume adjustment button, the electronic stethoscope 100 adjusts the gain of a signal output from a photodetector 204, and adjusts the volume of sounds output through an earphone. The operation unit 123 includes a mode switch button 123c for switching the operating mode of the electronic stethoscope 100. Pressing the mode switch button 123c switches the operating mode (described later). In other words, the mode switch button 123c receives an instruction regarding a mode transition of the electronic stethoscope 100 from the user. Note, however, that the operation unit 123 may include a touch panel instead of a plurality of physical buttons. The display device 122 and the operation unit 123 may be configured integrally as a touchscreen.

Like the display device 122, the operation unit 123 is disposed in the outer surface of the housing 121, in the vicinity of the chest piece 110 in the one end in the x-axis direction, but on the side opposite from the chest piece 110. With such an arrangement, the user can operate the operation unit 123 (e.g., with their thumb) while gripping the grip 120 when using the electronic stethoscope 100. The display device 122 is disposed in a position that is further away in from the center of the grip 120 than the operation unit 123 in the x-axis direction. With this arrangement, the user can keep the display device 122 visible even when operating the operation unit 123 while the electronic stethoscope 100 is in use.

The power switch 124 is a switch for switching the power of the electronic stethoscope 100 on and off. The connector 125 is a connector for receiving a cable or a connector of an external device. Power is supplied from the external device to a battery included in the grip 120 through the connector 125. The power switch 124 may be provided in the chest piece 110 instead of the grip 120. The connector 125 may be provided in the chest piece 110 instead of the grip 120. Furthermore, the electronic stethoscope 100 need not include the connector 125. In this case, the electronic stethoscope 100 may have a wireless charging function, or may be configured such that the battery can be replaced.

The electronic stethoscope 100 in the present embodiment includes both the chest piece 110 and the grip 120, but may instead be configured to include only the chest piece 110 and not the grip 120.

Configuration of Chest Piece of Electronic Auscultation Apparatus of First Embodiment

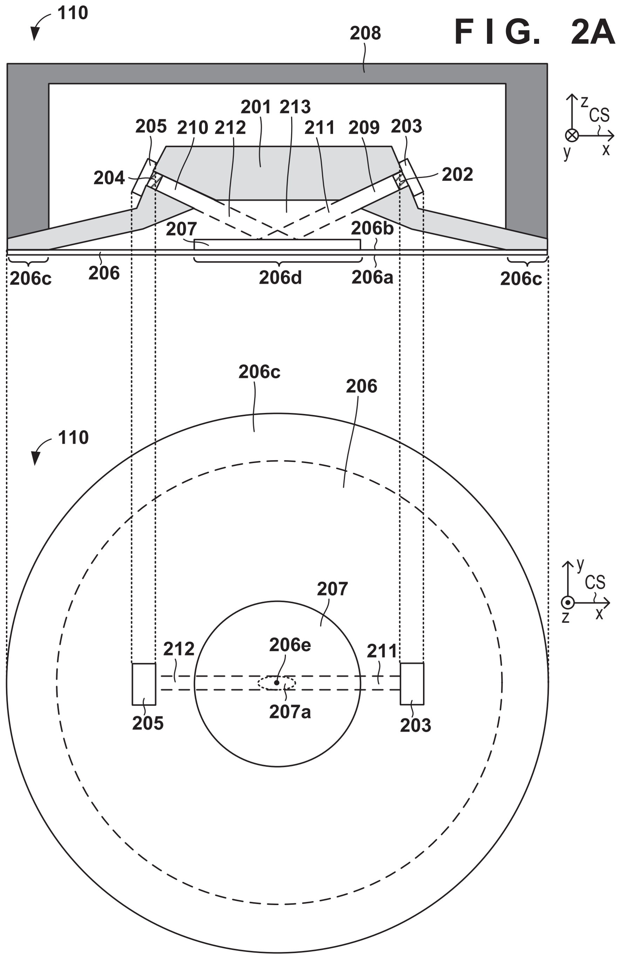

An example configuration of the chest piece 110 will be described with reference to FIG. 2A. The upper side of FIG. 2A is a cross-sectional view of the chest piece 110, and the lower side of FIG. 2A is a plan view of the chest piece 110. In the plan view, only a light-emitting circuit board 203, a light-receiving circuit board 205, the diaphragm 206, and a reflective medium 207 are illustrated, in order to clarify the positional relationship of the constituent elements.

The chest piece 110 includes a holding member 201, a light emitter 202, the light-emitting circuit board 203, the photodetector 204, the light-receiving circuit board 205, the diaphragm 206, the reflective medium 207, and a housing 208. The housing 208 houses the holding member 201, the light emitter 202, the light-emitting circuit board 203, the photodetector 204, the light-receiving circuit board 205, and the reflective medium 207. Because apertures 209 and 210 are formed in the holding member 201, the housing 208 also houses the apertures 209 and 210. Together with the housing 208, the diaphragm 206 forms part of the exterior of the electronic stethoscope 100. Note that the constituent elements of the chest piece 110 described here are merely examples, and a circuit board on which circuit elements for controlling the operations of the chest piece 110 are mounted may be provided in addition to the constituent elements illustrated in FIG. 2A.

The light emitter 202 is a light source that emits light. Power supplied to the light emitter 202 is supplied from a power source external to the chest piece 110 (the battery in the grip 120). In the present embodiment, the light emitter 202 uses a light-emitting diode (LED).

The light emitter 202 is mounted on the light-emitting circuit board 203. For example, peripheral circuitry for specifying a light emission intensity of the light emitter 202, a power terminal for receiving the supply of power from a power source external to the chest piece 110, and the like are mounted on the light-emitting circuit board 203. The light-emitting circuit board 203 may be a printed circuit board such as a flexible circuit board, a paper phenolic board, or a glass epoxy board. The light-emitting circuit board 203 including the light emitter 202 functions as a light-emitting unit.

The photodetector 204 generates an electrical signal that is based on an amount of light received using power supplied from a power source external to the chest piece 110 (the battery in the grip 120). Power supplied to the photodetector 204 is supplied from a power source external to the chest piece 110 (e.g., the battery in the grip 120). The photodetector 204 may be a phototransistor, a complementary metal oxide semiconductor (CMOS) sensor, or the like, for example. In the present embodiment, one photodetector 204 is provided, but the present invention is not limited thereto. A line sensor (photodetectors arranged in a $1 \times n$ arrangement ($n \geq 2$)), or an area sensor (photodetectors arranged in an m $\times$ n arrangement ($m \geq 2$, $n \geq 2$)) may be constituted by a plurality of photodetectors 204.

The photodetector 204 is mounted on the light-receiving circuit board 205. In addition to the photodetector 204, peripheral circuitry for reading out a signal from the photodetector 204, a signal terminal for outputting a signal to a device external to the chest piece 110, and a power terminal for receiving the supply of power from a power source external to the chest piece 110 are mounted on the light-receiving circuit board 205. The light-receiving circuit board 205 may be a printed circuit board such as a flexible circuit board, a paper phenolic board, or a glass epoxy board. The light-receiving circuit board 205 including the photodetector 204 functions as a light-receiving unit.

The holding member 201 holds the light-emitting circuit board 203 and the light-receiving circuit board 205. The light-emitting circuit board 203 and the light-receiving circuit board 205 are fixed to the holding member 201. These boards may be fixed to the holding member 201 using an adhesive, or using a fastening member such as a screw.

The diaphragm 206 has a contact surface 206a that contacts the surface of a target body, which is an example of a subject, and an inner surface 206b that the surface on the side opposite from the contact surface 206a. The diaphragm 206 is configured to elastically deform when pressed by the subject in contact with the contact surface 206a. The reflective medium 207 (described later) is provided on the inner surface 206b of the diaphragm 206. In the present embodiment, the diaphragm 206 uses a laminated plate of glass epoxy resin produced by impregnating glass fibers with epoxy resin and then thermosetting the resultant. The thickness is 230 $\mu$m. A ring-shaped rim for fixing the diaphragm 206 to the holding member 201 or the housing 208 is integrated with the diaphragm 206. In the present embodiment, the contact surface 206a and the inner surface 206b of the diaphragm 206 refer to the part of the diaphragm 206 that does not include the ring-shaped rim integrated with the diaphragm 206. The diaphragm 206 may have a multilayer structure. In this case, the layer of the multilayer structure diaphragm 206 that is furthest on the outside and that contacts the subject is defined as the contact surface 206a, and the layer that is furthest on the inside and that is provided with the reflective medium 207 is defined as the inner surface 206b. The diaphragm 206 may be constituted by a plurality of layers or members as long as the diaphragm 206 is a means that integrally vibrates with the subject in contact therewith. Additionally, in the present embodiment, even if the configuration is such that a separate cover is attached to the contact surface 206, that configuration is considered to be one form of the diaphragm 206 as long as the cover and the contact surface 206a are means for integrally vibrating with the subject. Although the contact surface 206a of the diaphragm 206 is exposed to the outside when the electronic stethoscope 100 is in use, the configuration may be such that a protective cover is attached to cover the contact surface 206a in order to suppress damage or deterioration of the contact surface 206a when not in use.

The diaphragm 206 is held by the holding member 201. The diaphragm 206 extends along an xy plane of the coordinate system CS. The diaphragm 206 is disposed so as to contact the surface of a target body, which is an example of the subject. The diaphragm 206 constitutes part of an exterior of the chest piece 110. The diaphragm 206 has the contact surface 206a disposed so as to contact the surface of the target body when the electronic stethoscope 100 is in use, and the inner surface 206b on the side opposite from the contact surface 206a.

The diaphragm 206 has a fixed part 206c, and is fixed to the holding member 201. The fixed part 206c is located on the outer periphery of the diaphragm 206. The inner periphery of the diaphragm 206 (i.e., the inner part of the fixed part 206c) is not fixed to the holding member 201. Accordingly, the diaphragm 206 is capable of vibrating in the z-axis direction with the fixed part 206c acting as a node. Specifically, when the chest piece 110 is in use, the diaphragm 206 vibrates, with the fixed part 206c acting as a node, in accordance with displacement in the surface of the target body. In this vibration, a center 206e of the diaphragm 206 acts as the antinode. The diaphragm 206 functions as a vibrating unit that vibrates with the subject.

The reflective medium 207 reflects the light emitted from the light emitter 202. The reflective medium 207 is affixed to the inner surface 206b of the diaphragm 206, and moves integrally with the diaphragm 206 in the z-axis direction in conjunction with the vibration of the diaphragm 206 that is in close contact with the surface of the target body. The reflective medium 207 has a circular outer edge in plan view. The reflective medium 207 may have a diameter of 15 mm to 20 mm. The outer edge of the reflective medium 207 may have another shape instead, however. The reflective medium 207 is disposed in a position that covers a region 206d including the center 206e of the circle of the diaphragm 206. Since the displacement of the diaphragm 206 varies most significantly at the center 206e, the displacement of the diaphragm 206 can be detected with a high level of sensitivity by reflecting light from the light emitter 202 in a region including the center 206e. Although the reflective medium 207 is disposed in a position covering the center 206e in the present embodiment, the reflective medium 207 may be disposed in a position covering a region that does not include the center 206e of the diaphragm 206. The reflective medium 207 is constituted by an aluminum vapor-deposited film, for example. The reflective medium 207 may be a sheet-shaped member affixed to the surface on the opposite side of the diaphragm 206 from the side on which the contact surface 206a is located.

The light emitter 202 emits light toward the inner surface 206b of the diaphragm 206 (specifically, the reflective medium 207). An upper surface of the reflective medium 207 reflects the light emitted from the light emitter 202.

In other words, the upper surface of the reflective medium 207 functions as a reflective surface. In the following descriptions, light being reflected at the upper surface of the reflective medium 207 (i.e., the reflective surface) will be referred to simply as light being reflected by the reflective medium 207. The reflective medium 207 reflects the light emitted from the light emitter 202 specularly (i.e., specular reflection). In the following descriptions, light directed from the light emitter 202 toward the reflective medium 207 will be referred to as "incident light 211", and the light after the incident light 211 is reflected will be referred to as "reflected light 212".

In the present embodiment, the reflective medium 207 is a member separate from the diaphragm 206. However, the reflective medium 207 may be configured as the same member as the diaphragm 206, and at least a part of the inner surface 206b of the diaphragm 206 may serve as the reflective medium. A coating layer may also be applied to the diaphragm 206, and the coating layer may constitute the reflective medium. For example, the entire inner surface 206b of the diaphragm 206 has a high reflectance such that enough light to be detected by the photodetector 204 can be reflected. Instead, however, the region of the inner surface 206b of the diaphragm 206 which the light emitted from the light emitter 202 reaches may have this high reflectance.

The light emitter 202 is disposed so that, in a state where the diaphragm 206 is not in contact with the surface of the target body, light is emitted toward a region 207a including a part of the reflective medium 207 that covers the center 206e of the diaphragm 206. In a state where the diaphragm 206 is not in contact with the surface of the target body, the diaphragm 206 is flat. The light emitter 202 emits light toward a specific region of the reflective medium 207 (e.g., the region 207a). In the present embodiment, as described above, an LED that emits diffuse light is used as the light emitter 202. Accordingly, the chest piece 110 has the aperture 209 for narrowing the light emitted from the light emitter 202. Due to the aperture 209, only some of the light emitted from the light emitter 202 is incident on the reflective medium 207. In the example illustrated in FIG. 2A, the part of the holding member 201 where the opening is formed corresponds to the aperture 209. Although the present embodiment describes a component that emits diffuse light as an example of the light emitter 202, a laser diode or the like that emits laser beam may instead be used as the light emitter 202, and the laser beam may be emitted toward the region 207a. If the light emitter 202 is a component that emits laser beam, the aperture 209 may be omitted. Additionally, although the portion of the holding member 201 where the opening is formed is described as an example of the aperture 209 in the present embodiment, a one-sided aperture may be used instead of an opening. In this case, for example, a light shield for narrowing one side (the upper side or the lower side) of the light emitted from the light emitter 202 is provided instead of the opening.

The photodetector 204 is disposed so as to receive the reflected light 212. Specifically, the photodetector 204 is disposed at a position where the amount of light received by the reflected light 212 changes due to the vibration of the diaphragm 206 in the z-axis direction. The photodetector 204 is disposed such that more of the reflected light 212 is received in a state where the diaphragm 206 is not in contact with the surface of the target body (i.e., in a state where the diaphragm 206 is flat) than when the diaphragm 206 is vibrating. In other words, the photodetector 204 outputs an electrical signal corresponding to the amount of reflected light 212 received, and the displacement amount of the diaphragm 206 can be determined on the basis of the electrical signal. The principles of this will be described later. The chest piece 110 has the aperture 210 for narrowing the light specularly reflected by the reflective medium 207. The aperture 210 suppresses situations where irregularly-reflected light is incident on the photodetector 204, and causes at least some of the light from the reflective medium 207 (i.e., primary reflected light) to reach the photodetector 204. In the example illustrated in FIG. 2A, the part of the holding member 201 where the opening is formed functions as the aperture 210. Note that although the portion of the holding member 201 where the opening is formed is described as an example of the aperture 210 in the present embodiment, a one-sided aperture may be used instead of an opening. In this case, for example, a light shield for narrowing one side (the upper side or the lower side) of the light from the reflective medium 207 is provided instead of the opening.

The housing 208 is attached to the upper surface of the outer periphery of the holding member 201. The housing 208 covers the light-emitting circuit board 203 and the light-receiving circuit board 205, and suppresses the entry of ambient sound into the housing 208. The outer edge of the diaphragm 206, the outer edge of the holding member 201, and the outer edge of the housing 208 substantially coincide with each other when the contact surface 206a of the diaphragm 206 is viewed in plan view. In the present embodiment, the housing 208 is formed of metal in the present embodiment, and the grounds of the circuit boards (e.g., the light-emitting circuit board 203 and the light-receiving circuit board 205) in the chest piece 110 are electrically connected to the housing 208. This stabilizes the ground potential.

An internal space 213 surrounded by the diaphragm 206 and the holding member 201 is formed by the diaphragm 206 being fixed to the holding member 201. The internal space 213 is sealed to suppress situations where the photodetector 204 receives light aside from that emitted by the light emitter 202. Additionally, the diaphragm 206 and the holding member 201 have light-shielding properties to suppress situations where the photodetector 204 receives light aside from that emitted by the light emitter 202. In the example illustrated in FIG. 2A, the fixed part 206c of the diaphragm 206 is fixed to the holding member 201. Instead, however, in the example illustrated in FIG. 2A, the fixed part 206c of the diaphragm 206 may be fixed to the housing 208.

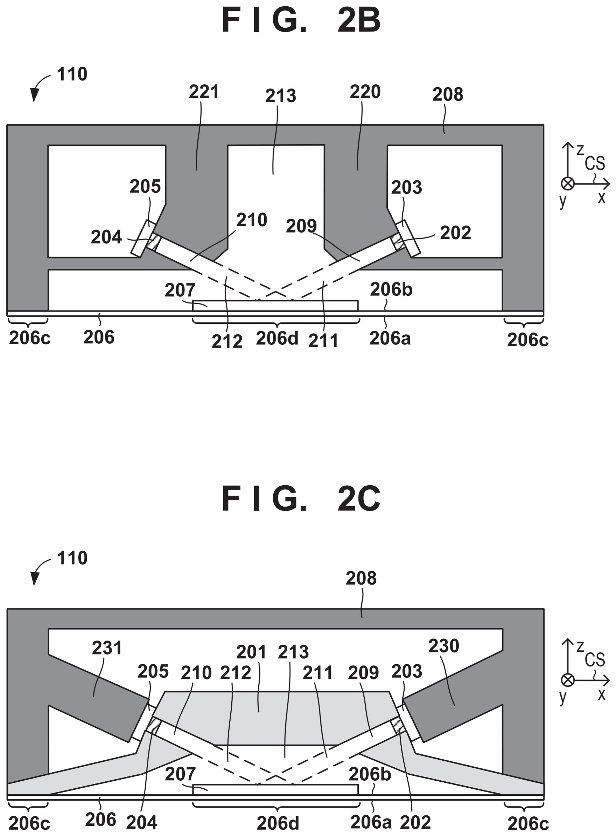

FIG. 2A illustrates an example of a configuration in which the light-emitting circuit board 203 and the light-receiving circuit board 205 are held by the holding member 201 provided in the housing 208. However, as illustrated in FIG. 2B, the configuration may be such that the light-emitting circuit board 203 formed integrally with the housing 208 holds a light-emitting unit 203, and a holding portion 221 formed integrally with the housing 208 holds the light-receiving circuit board 205. In such a configuration, the holding member 201 is omitted. A holding portion 220 has a light shield and functions as the aperture 209 that forms the incident light 211. The holding portion 221 has a light shield and functions as the aperture 210 that regulates the reflected light 212 that reaches the photodetector 204.

FIG. 2A illustrates an example of a configuration in which the light-emitting circuit board 203 and the light-receiving circuit board 205 are held by the holding member 201 provided in the housing 208. However, as illustrated in FIG. 2C, the configuration may be such that a member 230 extending from the housing 208 presses the light-emitting circuit board 203 toward the holding member 201, and the light-emitting circuit board 203 is held as a result. Additionally, illustrated in FIG. 2C, the configuration may be such that a member 231 extending from the housing 208 presses the light-receiving circuit board 205 toward the holding member 201, and the light-receiving circuit board 205 is held as a result.

Example of Operations by Electronic Auscultation Apparatus of First Embodiment

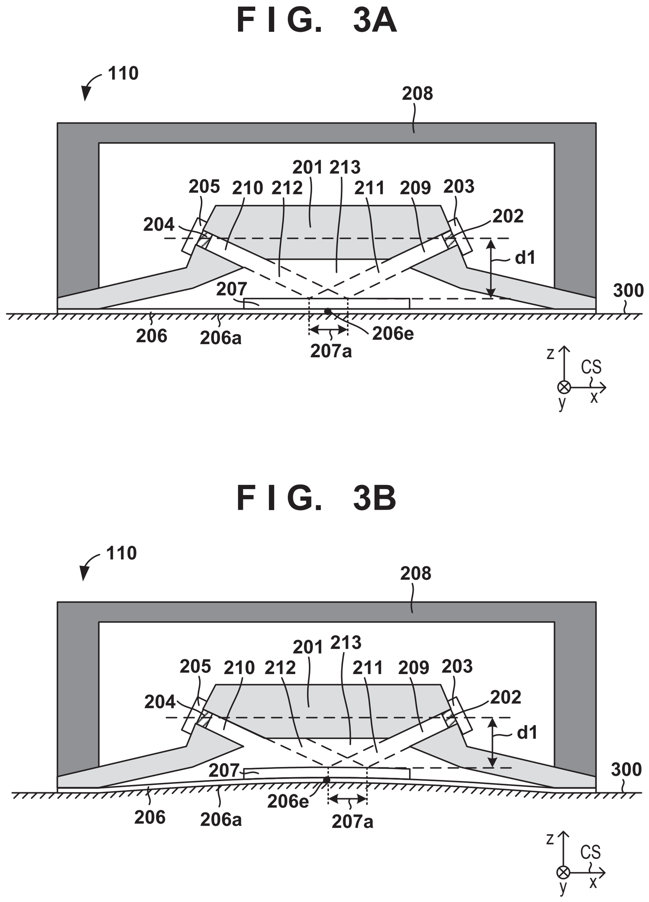

An example of operations by the chest piece 110 of the electronic stethoscope 100 in the first embodiment will be described with reference to FIGS. 3A and 3B. As illustrated in FIGS. 3A and 3B, the chest piece 110 is used when in a state of contact with a body surface 300 serving as the subject. In other words, when in use, the contact surface 206a of the diaphragm 206 of the chest piece 110 is in close contact with the body surface 300, which is an example of the subject. As a result, the body surface 300, the diaphragm 206, and the reflective medium 207 vibrate together. Here, the chest piece 110 detects displacement of the upper surface of the reflective medium 207 in the z-axis direction as displacement of the body surface 300 in the z-axis direction. The body surface 300 displaces in response to body movements such as the heartbeat or breathing of a human to which the body surface 300 belongs.

FIG. 3A is a cross-sectional view of the chest piece 110 when the diaphragm 206 is flat. As described above, the light emitter 202 and the photodetector 204 are disposed such that more of the reflected light 212 is received by the photodetector 204 when the diaphragm 206 is flat than when the diaphragm 206 is vibrating. The photodetector 204 amplifies and outputs photocurrent corresponding to the amount of received light. The peripheral circuitry of the light-receiving circuit board 205 generates, as a displacement signal, an output value obtained by converting the photocurrent output from the photodetector 204 into a voltage, and outputs the displacement signal to an external device. In the present embodiment, "displacement signal" refers to an output value of the photodetector 204 indicating the state, deformation, and the like of the diaphragm 206 at each time.

FIG. 3B is a cross-sectional view of the chest piece 110 when the body surface 300 is displaced upward. The distance between the light emitter 202 and the upper surface of the reflective medium 207 is represented by d1. The distance d1 decreases as the body surface 300 is displaced upward. Accordingly, the region 207a of the reflective medium 207 where the incident light 211 reaches moves so as to approach the light emitter 202, and the reflected light 212 also moves so as to approach the light emitter 202. As a result, the amount of the reflected light 212 that reaches the photodetector 204 decreases, and the value of the displacement signal generated by the light-receiving circuit board 205 decreases. In the state illustrated in FIG. 3B, none of the reflected light 212 reaches the photodetector 204, and thus the value of the displacement signal is ideally zero.

In this manner, in the chest piece 110, the light emitter 202 and the photodetector 204 are disposed such that the amount of light that reaches the photodetector 204 changes in response to movement of the body surface 300, the diaphragm 206, and the reflective medium 207. Because the reflective medium 207 displaces in conjunction with the displacement of the body surface 300, the displacement signal generated by the light-receiving circuit board 205 represents the displacement of the body surface 300.

Relationship between Displacement Amount of Diaphragm and Displacement Amount of Light-Receiving Position of Reflected Light in Electronic Auscultation Apparatus According to First Embodiment

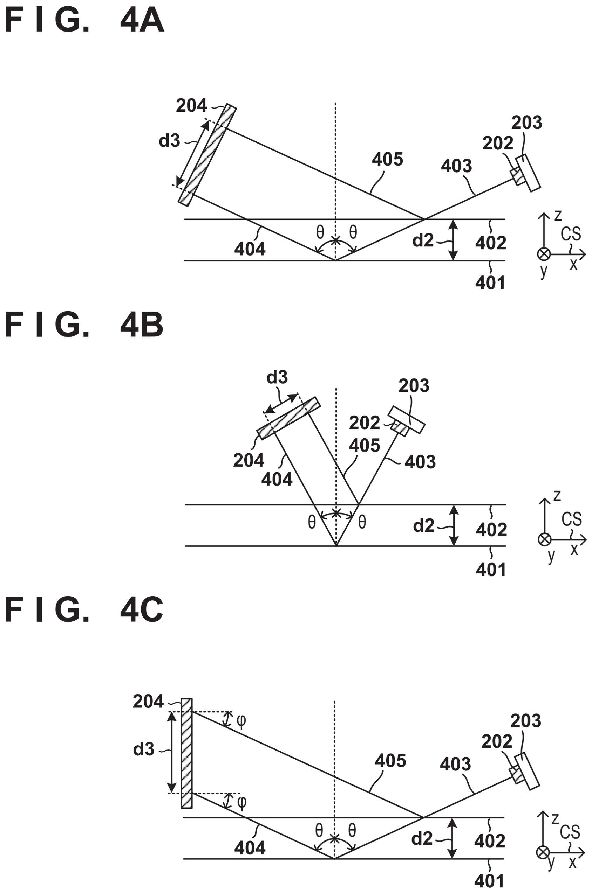

A relationship between the displacement amount of the body surface 300, the incident angle of the incident light 211, the incident angle of the reflected light 212, and the displacement amount at the position where the photodetector 204 receives the reflected light 212 will be described with reference to FIGS. 4A to 4F. In each of FIGS. 4A to 4F, a position 401 indicates a reference position of the upper surface of the reflective medium 207. In the present embodiment, the reference position is the upper surface of the reflective medium 207 when the diaphragm 206 is flat. A position 402 indicates a position to which the upper surface of the reflective medium 207 is displaced upward from the position 401 by a displacement amount d2. Because the displacement amount d2 of the reflective medium 207 is minute, the upper surface of the reflective medium 207 is assumed to be flat even when the upper surface of the reflective medium 207 is at the position 402.

In each of FIGS. 4A to 4F, an optical axis 403 indicates an optical axis of the incident light 211. The incident angle of the light emitted from the light emitter 202 and incident on the reflective medium 207 is represented by θ. The incident angle θ of the incident light 211 is defined by the angle formed between the optical axis 403 of the incident light 211 and the surface normal of the upper surface of the reflective medium 207. The optical axis of the reflected light 212 when the upper surface of the reflective medium 207 is at the position 401 is an optical axis 404. The optical axis of the reflected light 212 when the upper surface of the reflective medium 207 is at the position 402 is an optical axis 405. Because the incident light 211 is specularly reflected at the upper surface of the reflective medium 207, a reflection angle of the reflected light 212 is also θ. The optical axis 404 and the optical axis 405 are parallel to each other. An incident angle of the reflected light 212 on the photodetector 204 is represented by φ. The incident angle φ of the reflected light 212 is defined by the angle formed between the optical axis 404 or 405 of the reflected light 212 and the surface normal of a light-receiving surface of the photodetector 204. In FIGS. 4A and 4B, the incident angle φ is 0°, and thus φ is not indicated in the figures. The displacement amount at the position where the photodetector 204 receives the reflected light 212, when the upper surface of the reflective medium 207 is displaced from the position 401 to the position 402, is represented by d3. The displacement amount d3 may be defined by a displacement amount from the position where the photodetector 204 receives light along the optical axis 404 to a position where the photodetector 204 receives light along the optical axis 405. In the following descriptions, a ratio of the displacement amount d3 to the displacement amount d2 is expressed as a displacement gain G. In this case, the following relationship is established.

$$G = 2 \times \sin\theta/\cos\varphi \ldots \quad \text{(Formula 1)}$$

Accordingly, even if the displacement amount d2 of the reflective medium 207 is the same, the displacement gain G increases as the incident angle θ increases, and the displacement gain G also increases as the incident angle φ increases.

FIGS. 4A and 4B illustrate a case where the incident angle φ is 0°. In other words, the optical axes 404 and 405 are orthogonal to the light-receiving surface of the photodetector 204. In this case, Formula 1 becomes:

$$G = 2 \times \sin\theta... \quad \text{(Formula 2)}$$

As can be seen by comparing FIGS. 4A and 4B, the greater the incident angle $\theta$ is, the greater the displacement amount d3 becomes.

FIG. 4C illustrates a case where the light-receiving surface of the photodetector 204 is orthogonal to the upper surface of the reflective medium 207. In this case, $\varphi = 90° - \theta$ is satisfied, and Formula 1 becomes:

$$G = 2... \quad \text{(Formula 3)}$$

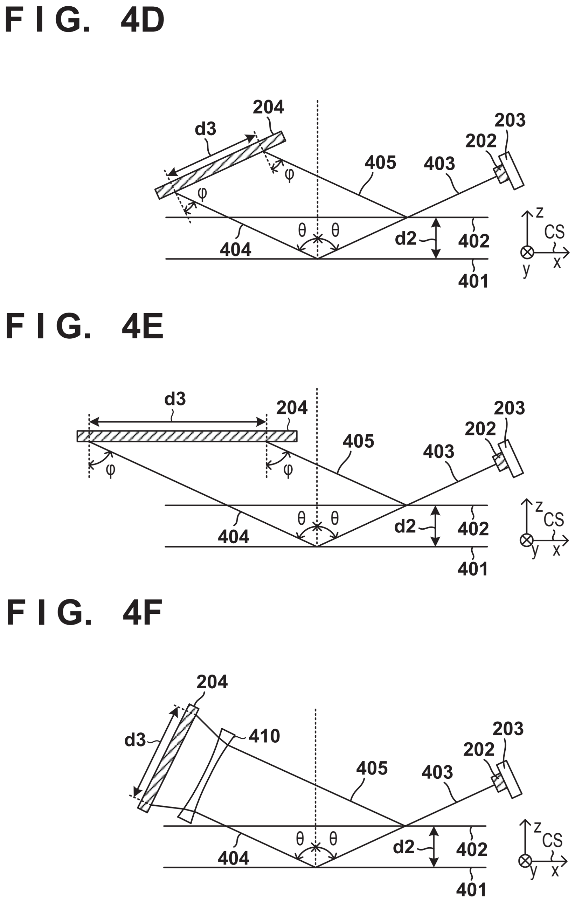

FIG. 4D illustrates a case where the light-receiving surface of the photodetector 204 is parallel to the optical axis 403. In this case, $\varphi = 2 \times \theta - 90°$ is satisfied, and Formula 1 becomes:

$$G = 1/\cos\theta... \quad \text{(Formula 4)}$$

FIG. 4E illustrates a case where the light-receiving surface of the photodetector 204 is parallel to the upper surface of the reflective medium 207. In this case, $\varphi = \theta$ is satisfied, and Formula 1 becomes:

$$G = 2\tan\theta... \quad \text{(Formula 5)}$$

Table 1 below shows the displacement gain G when the incident angle $\theta$ is changed with respect to the incident angle $\varphi$ in FIGS. 4A and 4C to 4E. In Table 1, decimals are rounded off to the nearest hundredth.

Table 1

| Incident angle $\theta$ | 30° | 45° | 60° | 70° |
|---|---|---|---|---|
| FIG. 4A (Displacement gain: $2\sin\theta$) | 1.00 | 1.41 | 1.73 | 1.88 |
| FIG. 4C (Displacement gain: 2) | 2.00 | 2.00 | 2.00 | 2.00 |
| FIG. 4D (Displacement gain: $1/\cos\theta$) | 1.15 | 1.41 | 2.00 | 2.92 |
| FIG. 4E (Displacement gain: $2\tan\theta$) | 1.15 | 2.00 | 3.46 | 5.49 |

The example configuration illustrated in FIG. 4F differs from that illustrated in FIG. 4A in that a lens 410 is further disposed in the optical path of the reflected light 212. The lens 410 is located between the reflective medium 207 and the photodetector 204. The lens 410 refracts the reflected light 212 in a direction away from the center of the photodetector 204. As a result, the displacement amount d3 increases even more, and thus the displacement gain G increases even more as well. In the example configuration illustrated in FIG. 4F, the lens 410 is added to the example configuration illustrated in FIG. 4A. The lens 410 may be added to any of the example configurations illustrated in FIGS. 4C to 4E.

Relationship between Displacement Amount of Surface of Living Body and Displacement Signal in Electronic Auscultation Apparatus According to First Embodiment

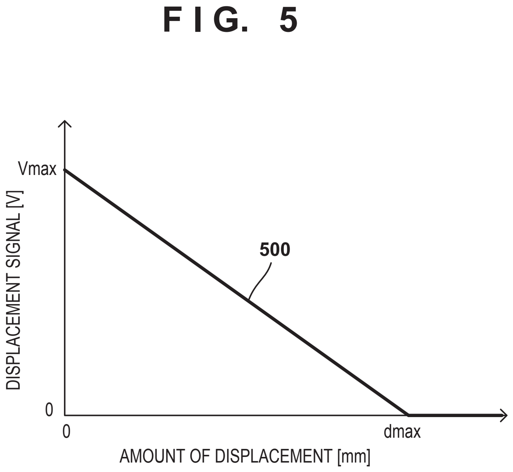

A relationship between the displacement amount of the body surface 300 and the displacement signal will be described with reference to FIG. 5. The displacement signal represents a voltage output from the light-receiving circuit board 205. A graph 500 in FIG. 5 represents the relationship between the displacement amount of the body surface 300 and the displacement signal. The horizontal axis of the graph 500 represents the displacement amount of the body surface 300, and represents the displacement signal generated by the light-receiving circuit board 205.

As described above, the displacement amount of the body surface 300 is equal to the displacement amount d2 of the upper surface of the reflective medium 207. As illustrated in FIGS. 3A and 3B, as the displacement amount d3 of the reflected light 212 increases, the reflected light 212 that reaches the photodetector 204 decreases monotonically and linearly. Accordingly, when the value of the displacement signal is represented by Sd,

$$Sd = Vmax - k \times d3... \quad \text{(Formula 6)}$$

Here, Vmax represents the value of the displacement signal when the displacement amount d3 of the reflected light 212 is zero, and k represents a proportionality coefficient determined by the amplification rate of an amplifier circuit of the light-receiving circuit board 205. By substituting Formula 1 in Formula 6, the following is obtained:

$$Sd = Vmax − 2k \times d2 \times \sin\theta/\cos\varphi... \quad (Formula\ 7)$$

Accordingly, the displacement signal Sd decreases monotonically and linearly as the displacement amount d2 of the body surface 300 increases, as indicated by the graph 500. The displacement amount at which the displacement signal Sd is zero is represented by dmax. When the displacement amount exceeds dmax, the reflected light 212 no longer reaches the photodetector 204, and thus the displacement signal Sd remains at zero even if the displacement amount d2 increases. Accordingly, the proportionality coefficient k, the incident angle $\theta$, and the incident angle $\varphi$ are set such that the displacement amount d2 is within a range of 0 to dmax in the range in which vibration of the diaphragm 206 is assumed to occur (referred to as the "operating range" of the diaphragm 206). As indicated by the graph 500, the light emitter 202 and the photodetector 204 are disposed such that the amount of light that reaches the photodetector 204 (the received light level) changes monotonically in response to the reflective medium 207 moving in one direction within the operating range of the diaphragm 206. Although the photodetector 204 is disposed such that the received light level decreases monotonically in the example in FIG. 5, the photodetector 204 may be disposed such that the received light level increases monotonically.

In Formula 7, $2k \times \sin\theta/\cos\varphi = k \times G$, which is the coefficient of d2, represents the sensitivity of the chest piece 110. The incident angle $\theta$ can take on a value in a range greater than 0° but less than 90°. The incident angle $\varphi$ can take on a value in a range greater than 0° but less than 90°. The greater the displacement gain G is, the higher the sensitivity of the chest piece 110 becomes. Accordingly, the chest piece 110 may be configured such that the displacement gain G is greater than 1, i.e., such that the displacement amount d3 is greater than the displacement amount d2. Furthermore, the chest piece 110 may be configured such that the displacement gain G is greater than 1.5, or such that the displacement gain G is greater than 2. For example, the chest piece 110 may satisfy a relationship of $45° \leq \theta < 90°$, and may further satisfy a relationship of $45° < \theta < 90°$. Specifically, the incident angle $\theta$ may be 45°, 60°, or 70°. The incident angle $\varphi$ may be 0°, 30°, 45°, 60°, or 70°.

In the present embodiment, the light emitter 202 and the photodetector 204 are disposed such that all of the reflected light 212 reaches the photodetector 204 (i.e., such that the displacement signal is Vmax) when the diaphragm 206 is flat. Instead, however, the light emitter 202 and the photodetector 204 may be disposed such that all of the reflected light 212 reaches the photodetector 204 when the diaphragm 206 is in a position displaced downward from the flat state.

The chest piece 110 according to the embodiment described above can accurately detect displacement of the body surface 300. Specifically, in the above-described chest piece 110, when the surface of a target body, which is an example of a subject, is in close contact with the diaphragm 206, a displacement signal is generated on the basis of the displacement amount d2 of the surface of the target body, which vibrates integrally with the diaphragm 206. Accordingly, the displacement of the body surface 300 can be accurately detected regardless of the frequency at which the body surface 300 vibrates. For example, displacement of the body surface 300 produced by low-frequency vibration of about 10 Hz can also be accurately detected. Such low-frequency vibrations are contained in sounds (e.g., heart sounds) emitted by vibrations transmitted from inside the body due to the heartbeat. In the chest piece 110, the displacement signal does not change unless the diaphragm 206 is displaced. As a result, no ambient sounds, or vibration or acceleration caused by movement of the chest piece 110, are detected as noise, and thus output characteristics having a high SNR can be obtained.

Hardware Configuration of Electronic Auscultation Apparatus of First Embodiment

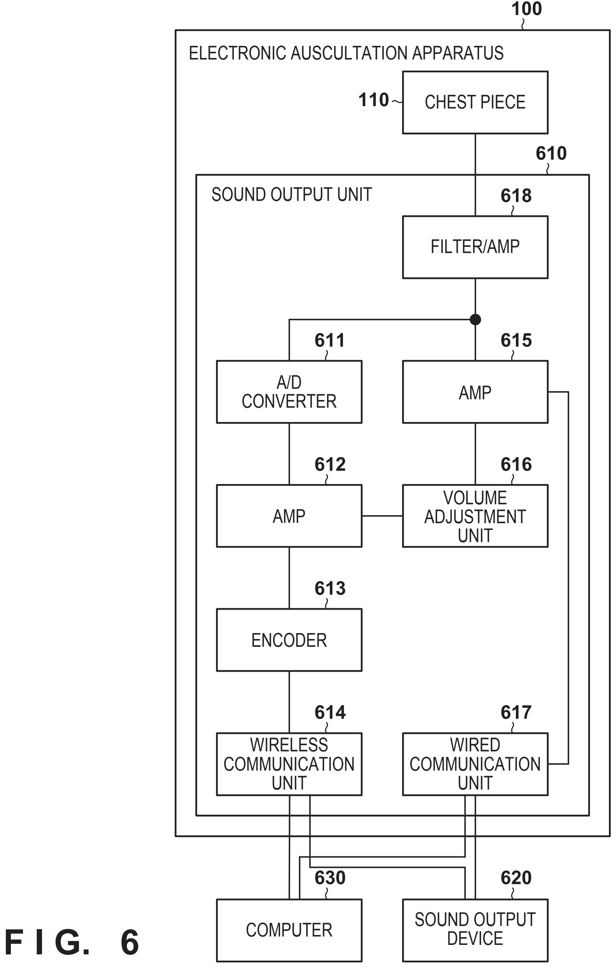

An example of the hardware configuration of the electronic stethoscope 100 according to the first embodiment will be used with reference to FIG. 6.

The electronic stethoscope 100 includes the above-described chest piece 110 and a audio output unit 610. The audio output unit 610 is implemented by a plurality of circuit elements mounted on a circuit board included in the grip 120. The plurality of circuit elements include a processor. The processor constituting the audio output unit 610 sends a sound signal based on the displacement signal generated by the chest piece 110 to an external audio output device. The sound signal transmitted by the audio output unit 610 represents the sound of a target body (e.g., a human) having the body surface 300, and will therefore also be referred to as a "biosignal". The sound signal is transmitted to a audio output device 620, such as an earphone, a headphone, or the like. The sound signal is also transmitted to a computer 630 (e.g., a personal computer, a smartphone, a tablet, or the like) at the same time as the sound signal is transmitted to the audio output device 620. A doctor, a nurse, or a public health nurse serving as the user can use the audio output device 620 or the computer 630 to listen to the body sound represented by the digitally-converted sound signal. The audio output device 620 is a wireless or wired earphone or headphone.

The audio output unit 610 has the constituent elements illustrated in FIG. 6. Because the audio output unit 610 conforms to the above-mentioned earphone or headphone, the sound signal can be communicated both through wireless communication and wired communication. Processing by which the audio output device 620 outputs the sound signal through wired communication will be described hereinafter. The displacement signal output from the chest piece 110 is filtered and amplified by a filter/amp 618 and supplied to each of an A/D converter 611 and an amp 615. The amp 615 further amplifies the output from the filter/amp 618 and supplies the output to a wired communication unit 617. The wired communication unit 617 provides the amplified sound signal to the audio output device 620. The wired communication unit 617 is, for example, a 3.5 mm AUX terminal or the like. The amplification gain of the amp 615 is adjusted by a volume adjustment unit 616. The audio output device 620 may be considered to constitute part of the electronic stethoscope 100. In this case, the electronic stethoscope 100 includes the chest piece 110, the grip 120, and the audio output device 620.

Processing by which the audio output device 620 outputs the sound signal through wireless communication will be described next. The A/D converter 611 digitizes the output from the filter/amp 618. The displacement signal in digital format is then amplified by an amp 612 and supplied to an encoder 613. The encoder 613 generates sound data for wireless communication by performing signal processing such as data compression and encoding on the amplified sound signal. The order of the processing by the amp 612 and the encoder 613 may reversed. A wireless communication unit 614, which conforms to a wireless communication standard such as Bluetooth (registered trademark), then provides the processed sound data to the audio output device 620. The amplification gain of the amp 612 is adjusted by the volume adjustment unit 616. Although the foregoing describes an example in which the electronic stethoscope 100 is capable of outputting the sound signal through both wireless communication and wired communication, the sound signal may be output by only one of these types of communication.

The transmission of the sound signal to the computer 630 is the same as the transmission of the sound signal to the audio output device 620. The computer 630 can also visually display waveform data generated on the basis of the sound signal. The waveform data may be generated by the computer 630, or may be generated by the electronic stethoscope 100. Some or all of the signal processing and the audio output processing by the electronic stethoscope 100 may be performed by an external device (e.g., the audio output device 620 or the computer 630).

The electronic stethoscope 100 according to the first embodiment described above can accurately detect displacement of the body surface 300 regardless of the frequency at which the body surface 300 vibrates. Accordingly, the electronic stethoscope 100 enables good auscultation of both relatively low-frequency body sounds at around 10 Hz, such as cardiac sounds emitted by the body due to heartbeats, and relatively high-frequency body sounds emitted by the body due to breathing.

Detailed Example of Operations by Electronic Auscultation Apparatus of First Embodiment

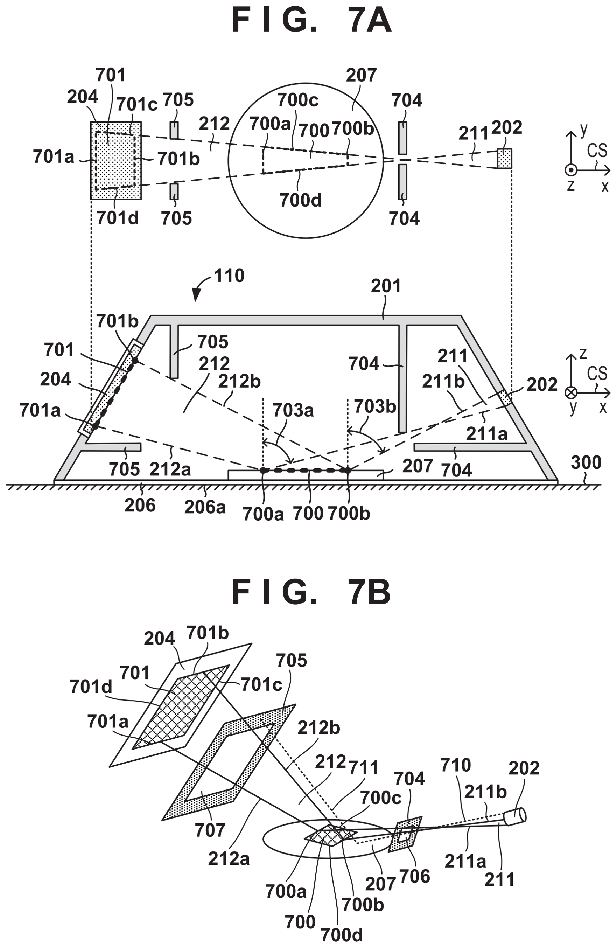

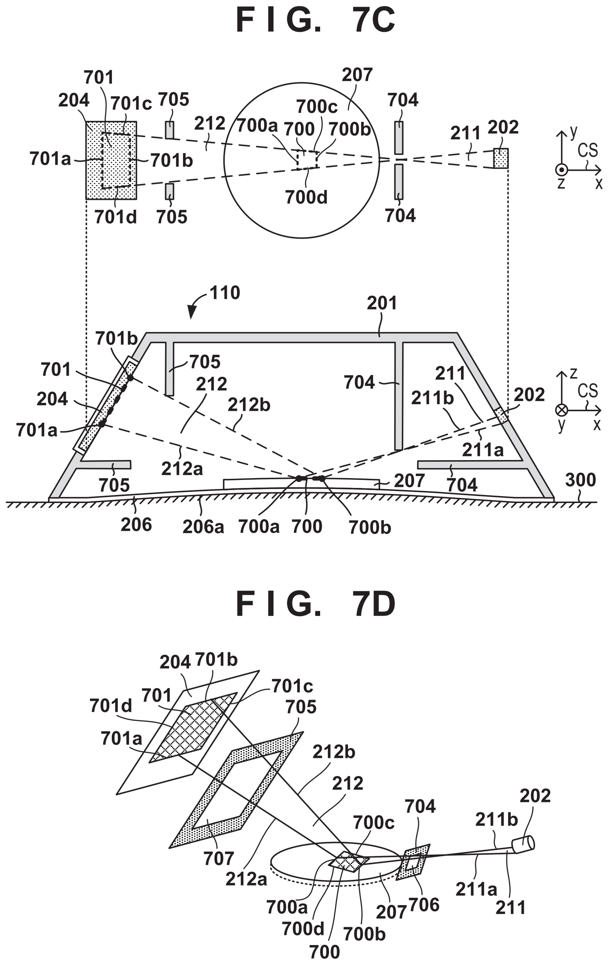

Operations of the chest piece 110 of the electronic stethoscope 100 in the first embodiment will be described in further detail with reference to FIGS. 7A to 7D. FIGS. 7A and 7B illustrate a state in which the diaphragm 206 is not pressed (i.e., is flat). FIGS. 7C and 7D illustrate a state in which the diaphragm 206 is pressed by the body surface 300. In each of FIGS. 7A and 7C, the lower side is a cross-sectional view of the chest piece 110, and the upper side is a plan view of the chest piece 110. In the plan view, only the light emitter 202, the photodetector 204, the reflective medium 207, a light shield 704, and a light shield 705 are illustrated in order to clarify the positional relationships of the constituent elements. FIGS. 7B and 7D are perspective views focusing on the light emitter 202, the photodetector 204, the reflective medium 207, the light shield 704, and the light shield 705.

In the chest piece 110 illustrated in FIGS. 7A to 7D, the light shield 704 in which an opening 706 is formed functions as the aperture 209 on the incident light 211 side, and the light shield 705 in which an opening 707 functions as the aperture 210 on the reflected light 212 side. In particular, the part of the light shield 704 on the upper side of the opening 706 corresponds to a first aperture. Accordingly, some of the light emitted by the light emitter 202 is shielded by the light shield 704 and does not reach the reflective medium 207. In addition, at least some of the light specularly reflected by the reflective medium 207 is blocked by the light shield 705, depending on the position of the reflective medium 207. In the present embodiment, both the openings 706 and 707 are rectangular. In the following descriptions, of the four sides of each of the openings 706 and 707, the side parallel to the diaphragm 206 and closer to the diaphragm 206 will be referred to as the "lower side", the side parallel to the diaphragm 206 and farther from the diaphragm 206 will be referred to as the "upper side", the side on the left as seen from the light emitter 202 will be referred to as the "left side", and the side on the right as seen from the light emitter 202 will be referred to as the "right side".

In FIGS. 7A to 7D, the incident light 211 and the reflected light 212 indicate light beams that reach the photodetector 204. In FIG. 7B, some light 710 emitted from the light emitter 202 passes through the opening 706 in the light shield 704 and is reflected by the reflective medium 207 to become light 711, but is blocked by the part of the light shield 705 that is higher than the reflected light 212 and does not reach the photodetector 204. The same applies in FIG. 7D.

As illustrated in FIGS. 7A and 7B, the part of the reflective medium 207 which the incident light 211 reaches while the diaphragm 206 is not pressed by the body surface 300 corresponds to an effective range 700. The effective range 700 is the part of the reflective medium 207 that reflects the light that reaches the photodetector 204. In a state where the diaphragm 206 is not pressed, the effective range 700 is equal to the range which the light from the light emitter 202 reaches. In the present embodiment, the effective range 700 is a rectangular region. The outer periphery of the effective range 700 is indicated as a boundary line of the effective range 700. The boundary line of the effective range 700 is located between the effective range 700 and a range other than the effective range 700. In the following descriptions, part of the boundary line will also be referred to as the boundary line.

Of the four line segments constituting the boundary line of the effective range 700, the line segment including the farthest position from the light emitter 202 in the x-axis direction will be referred to as a "far boundary line 700a". The part of the incident light 211 that reaches the far boundary line 700a is referred to as "far incident light 211a". "Far incident light 211a" means that the optical path from the light emitter 202 to the reflective medium 207 includes the longest part. The incident angle of the incident light 211 on the reflective medium 207 is a maximum value 703a at a position on the far boundary line 700a.

Of the four line segments constituting the boundary line of the effective range 700, the line segment including the nearest position from the light emitter 202 in the x-axis direction will be referred to as a "near boundary line 700b". The part of the incident light 211 that reaches the near boundary line 700b will be referred to as "near incident light 211b". "Near incident light 211b" means that the optical path from the light emitter 202 to the reflective medium 207 includes the shortest part. The incident angle of the incident light 211 on the reflective medium 207 is a minimum value 703b at a position on the near boundary line 700b. Of the light emitted from the light emitter 202, light not included between the far incident light 211a and the near incident light 211b is attenuated by being reflected by the light shield 704 multiples times.

Of the four line segments constituting the boundary line of the effective range 700, the two line segments other than the far boundary line 700a and the near boundary line 700b will be referred to as "side boundary lines 700c and 700d". The side boundary line 700c is located on the right side of the effective range 700 as seen from the light emitter 202, and the side boundary line 700d is located on the left side of the effective range 700 as seen from the light emitter 202.

As illustrated in FIGS. 7A and 7B, a region formed on the photodetector 204 by the reflected light 212 specularly reflected by the reflective medium 207 is indicated as an illuminated region 701. The illuminated region 701 is the part of the photodetector 204 where the light emitted from the light emitter 202 and specularly reflected by the reflective medium 207 reaches. Although scattered light other than the light specularly reflected by the reflective medium 207 can reach the photodetector 204, in the present embodiment, the illuminated region 701 defines the region formed by the specularly-reflected light as the illuminated region. The amount of light that reaches the photodetector 204 is proportional to the area of the illuminated region 701. In the present embodiment, the illuminated region 701 is a rectangular region. The outer periphery of the illuminated region 701 is indicated as a boundary line of the illuminated region 701. The boundary line of the illuminated region 701 is located between the illuminated region 701 and a region other than the illuminated region 701.

Of the four line segments constituting the boundary line of the illuminated region 701, the line segment formed by the light narrowed by the aperture 209 and specularly reflected by the reflective medium 207 will be referred to as a "lower boundary line 701a". Of the four line segments constituting the boundary line of the illuminated region 701, the line segment on the side opposite from the lower boundary line 701a will be referred to as an "upper boundary line 701b". The lower boundary line 701a is an example of a boundary line formed by the light narrowed by the aperture 209 and specularly reflected by the reflective medium 207. The lower boundary line 701a is a boundary line that moves in accordance with the displacement of the contact surface 206a (described later). In the present embodiment, the area of an illuminated region 709 changes due to the movement of the lower boundary line 701a, and the output of the photodetector 204 changes as a result. This makes it possible to accurately measure the displacement of the subject. The upper boundary line 701b is an example of a boundary line that does not move in accordance with the displacement of the contact surface 206a and whose length does not change even when the contact surface 206a displaces. Of the four line segments constituting the boundary line of the illuminated region 701, the two line segments other than the lower boundary line 701a and the upper boundary line 701b will be referred to as "side boundary lines 701c and 701d". The side boundary line 701c is located on the right side of the illuminated region 701 as seen from the light emitter 202, and the side boundary line 701d is located on the left side of the illuminated region 701 as seen from the light emitter 202. The side boundary line 701c and the side boundary line 701d are examples of boundary lines that do not move in accordance with the displacement of the contact surface 206a and whose length changes when the contact surface 206a displaces (described later).

Light passing through the opening 706 along the upper side of the opening 706 is specularly reflected by the reflective medium 207, and then reaches the lower boundary line 701a of the illuminated region 701 of the photodetector 204 without being shielded by the light shield 705. Accordingly, the upper side of the opening 706 defines the lower boundary line 701a of the illuminated region 701. On the other hand, light passing through the opening 706 along the lower side of the opening 706 is specularly reflected by the reflective medium 207, is shielded by the light shield 705, and does not reach the photodetector 204. Accordingly, the lower side of the opening 706 does not define the illuminated region 701. The near incident light 211b is therefore not narrowed by the aperture 209. Instead, however, the configuration may be such that the light passing through the opening 706 along the lower side of the opening 706 reaches the photodetector 204 without being shielded by the light shield 705 after being specularly reflected by the reflective medium 207. In this case, the lower side of the opening 706 defines the illuminated region 701. In such a configuration, the displacement signal in FIG. 5 is constant when the displacement amount of the diaphragm 206 is in a range from zero to a predetermined value. Then, when the lower side of the opening 706 no longer defines the illuminated region 701, the displacement signal begins to decrease monotonically.

After passing through the opening 706 and being specularly reflected by the reflective medium 207, the light passing through the opening 707 along the upper side of the opening 707 reaches the upper boundary line 701b of the illuminated region 701 of the photodetector 204. Accordingly, the upper side of the opening 707 defines the upper boundary line 701b of the illuminated region 701. In other words, the upper side of the opening 707 is an example of an aperture that narrows the light specularly reflected by the reflective medium 207. On the other hand, being shielded by the light shield 704, light does not pass through the part along the lower side of the opening 707. Accordingly, the lower side of the opening 707 does not define the illuminated region 701.

As illustrated in FIG. 7A, the side boundary line 701c and the side boundary line 701d of the illuminated region 701 are defined by the right side and the left side of the opening 707. Instead, however, the side boundary line 701c and the side boundary line 701d of the illuminated region 701 may be defined by the right side and the left side of the opening 706.

The reflected light of the far incident light 211a will be referred to as "lower end reflected light 212a". The lower end reflected light 212a is the reflected light 212 at the lowest position in the z-axis direction (i.e., the part near the diaphragm 206). The lower end reflected light 212a reaches the lower boundary line 701a of the illuminated region 701. The lower boundary line 701a is formed by the light narrowed by the aperture 209 and specularly reflected by the reflective medium 207. The lower end reflected light 212a is distanced from each side of the opening 705. In other words, the lower end reflected light 212a is not narrowed by the aperture 210. In the configuration in FIGS. 7A and 7B, the lower boundary line 701a includes the position of the illuminated region 701 closest to the diaphragm 206 in the normal direction (i.e., the z-axis direction) of the diaphragm 206 in a state where the diaphragm 206 is not pressed by the body surface 300. Furthermore, in the configuration in FIGS. 7A and 7B, the lower boundary line 701a includes a position of the illuminated region 701 which light having a maximum reflection angle at the reflective medium 207 reaches. The maximum value of the reflection angle is equal to the maximum value 703a of the incident angle. Furthermore, in the configuration in FIGS. 7A and 7B, the lower boundary line 701a includes the position furthest from the light emitter 202 in the plan view of the diaphragm 206 in the state where the diaphragm 206 is not pressed.

The reflected light of the near incident light 211b will be referred to as "upper end reflected light 212b". The upper end reflected light 212b is the reflected light 212 at the highest position in the z-axis direction (i.e., the part far from the diaphragm 206). The upper end reflected light 212b reaches the upper boundary line 701b of the illuminated region 701. In the configuration in FIGS. 7A and 7B, the upper boundary line 701b includes the position of the illuminated region 701 farthest from the diaphragm 206 in the normal direction of the diaphragm 206 (i.e., the z-axis direction) in the state where the diaphragm 206 is not pressed. Furthermore, in the configuration in FIGS. 7A and 7B, the upper boundary line 701b includes a position of the illuminated region 701 which light having a minimum reflection angle at the reflective medium 207 reaches. The minimum value of the reflection angle is equal to the minimum value 703b of the incident angle. Furthermore, in the configuration in FIGS. 7A and 7B, the upper boundary line 701b includes the position closest to the light emitter 202 in the plan view of the diaphragm 206 in the state where the diaphragm 206 is not pressed.

As illustrated in FIGS. 7C and 7D, the positions of the effective range 700, the far boundary line 700a, the near boundary line 700b, the illuminated region 701, the lower boundary line 701a, and the upper boundary line 701b change when the diaphragm 206 is pressed by the body surface 300. Of the reflected light 212, the part farthest from the light emitter 202 in the x-axis direction will be called the "lower end reflected light 212a". The lower end reflected light 212a reaches the lower boundary line 701a of the illuminated region 701. As described above, the lower boundary line 701a is defined by the upper side of the opening 706 in the aperture 209 on the light emitter 202 side. The lower boundary line 701a moves in accordance with the displacement of the contact surface 206a due to the elastic deformation of the diaphragm 206, and as a result, the area of the illuminated region 701 and the output of the photodetector 204 change (described later). The displacement amount of the lower boundary line 701a corresponds to the displacement amount d3 of the reflected light 212 illustrated in FIGS. 4A to 4F. In other words, the lower boundary line 701a is displaced by the displacement gain G relative to the displacement amount d2 of the diaphragm 206. The position where the part of the reflected light 212 farthest from the light emitter 202 (in a three-dimensional space independent of the x-axis direction) reaches the photodetector 204 is similarly displaced by the displacement gain G.

As illustrated in FIGS. 7A to 7D, the upper boundary line 701b is defined by the part of the light shield 705 higher than the reflected light 212, and is a boundary line that does not move in accordance with the displacement of the contact surface 206a and whose length does not change even when the contact surface 206a displaces. The part of the light shield 704 below the incident light 211 need not shield the light emitted from the light emitter 202. For example, the part of the light shield 704 below the incident light 211 need not be provided. Furthermore, the lower boundary line 701a is defined by the part of the light shield 704 higher than the incident light 211. Accordingly, the part of the light shield 705 lower than the reflected light 212 need not shield the light specularly reflected by the reflective medium 207. For example, the part of the light shield 705 below the reflected light 212 need not be provided.

Change in Reach Range of Reflected Light in Electronic Auscultation Apparatus According to First Embodiment

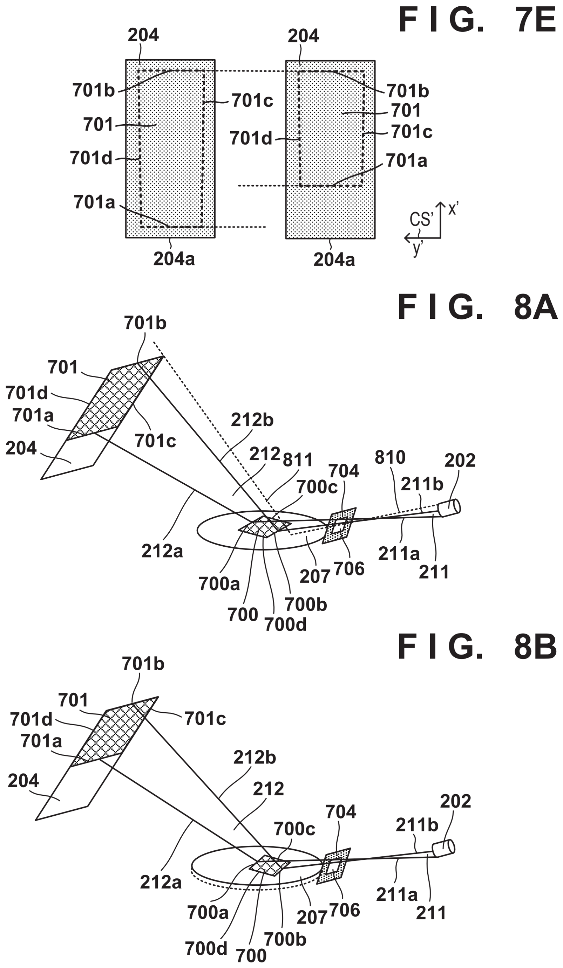

A change in the illuminated region 701 formed by the reflected light 212 reaching the light-receiving surface of the photodetector 204 will be described with reference to FIG. 7E. FIG. 7E is a plan view of the light-receiving surface of the photodetector 204. The left side of FIG. 7E illustrates the position of the illuminated region 701 in a state where the diaphragm 206 is not pressed. The right side of FIG. 7E illustrates the position of the illuminated region 701 in a state where the diaphragm 206 is pressed by the body surface 300.

A coordinate system CS' is indicated in FIG. 7E to show directions. The coordinate system CS' is a two-dimensional Cartesian coordinate system having an x'-axis and a y'-axis that are orthogonal to each other. The y'-axis matches the y-axis of the coordinate system CS. The x'-axis is parallel to an xz plane of the coordinate system CS. In the following descriptions, the positive direction in the x'-axis will be referred to as the "upper" side, and the negative direction in the x'-axis will be referred to as the "lower" side.

The surface of the photodetector 204 facing the internal space 213 is the light-receiving surface. The photodetector 204 detects the amount of light that has reached the light-receiving surface. As described above, in the present embodiment, the photodetector 204 is a single photodetector. A line sensor or an area sensor may be used instead of a single photodetector. The light-receiving surface may have a rectangular shape. Of the four sides of the light-receiving surface, the side parallel to the diaphragm 206 and closer to the diaphragm 206 will be referred to as a side 204a.

The area of the illuminated region 701 is defined by the lower boundary line 701a, the upper boundary line 701b, and the side boundary lines 701c and 701d. As illustrated in FIG. 7E, the lower boundary line 701a of the illuminated region 701 changes in the x'-axis direction in accordance with the displacement of the contact surface 206a. On the other hand, the upper boundary line 701b and the side boundary lines 701c and 701d substantially do not move in accordance with the displacement of the contact surface 206a. Accordingly, the area of the illuminated region 701 changes in accordance with the movement of the lower boundary line 701a. The length of the upper boundary line 701b does not change even when the contact surface 206a displaces. On the other hand, the lengths of the side boundary lines 701c and 701d change when the contact surface 206a displaces.

When the area of the illuminated region 701 changes, the signal output from the photodetector 204 also changes. Specifically, as the displacement amount of the contact surface 206a of the diaphragm 206 increases from the flat state, the shorter the distance between the lower boundary line 701a and the upper boundary line 701b (i.e., the lengths of the side boundary lines 701c and 701d) becomes, and the area of the illuminated region 701 decreases. As a result, as the displacement amount of the diaphragm 206 increases from the flat state, the less light the photodetector 204 receives. Accordingly, the signal output from the photodetector 204 also becomes smaller. As illustrated in FIG. 7E, the movement amount of the lower boundary line 701a associated with the movement of the reflective medium 207 is greater than the movement amount of the upper boundary line 701b associated with the movement of the reflective medium 207.

As illustrated in FIG. 7E, the change in the illuminated region 701 in the x'-axis direction is greater than the change in the illuminated region 701 in the y'-axis direction. Accordingly, in order to increase the dynamic range of the photodetector 204, the width of the photodetector 204 in the x'-axis direction is preferably greater than the width of the photodetector 204 in the y'-axis direction. More specifically, the width of the photodetector 204 in the x'-axis direction is preferably at least three times the width of the photodetector 204 in the y'-axis direction.

First Variation on Electronic Auscultation Apparatus of First Embodiment

A first variation on the chest piece 110 will be described with reference to FIGS. 8A and 8B. FIGS. 8A and 8B primarily illustrate the differences from FIGS. 7B and 7D. In the chest piece 110 according to the first variation, the light shield 705 is omitted. Accordingly, the chest piece 110 does not have the aperture 210.

Light passing through the opening 706 along the upper side of the opening 706 is specularly reflected by the reflective medium 207, and then reaches the lower boundary line 701a of the illuminated region 701 of the photodetector 204. Accordingly, the upper side of the opening 706 defines the lower boundary line 701a of the illuminated region 701. On the other hand, as illustrated in FIG. 8A, light 810 passing through the opening 706 along the lower side of the opening 706 is specularly reflected by the reflective medium 207 to become light 811, which reaches the outer side of the photodetector 204. Accordingly, the lower side of the opening 706 does not define the illuminated region 701. The same applies in FIG. 8B. The upper boundary line 701b of the illuminated region 701 is defined by a line segment forming an edge of a photosensitive area of the photodetector 204 (specifically, the upper side of the photosensitive area of the photodetector 204). Also, as illustrated in FIGS. 8A and 8B, the side boundary lines 701c and 701d of the illuminated region 701 are defined by a line segment forming an edge of the photosensitive area of the photodetector 204 (specifically, the right side and the left side of the photosensitive area of the photodetector 204). In other words, the side boundary lines 701c and 701d of the illuminated region 701 may instead be defined by the right side and the left side of the opening 706.

Second Variation on Electronic Auscultation Apparatus of First Embodiment

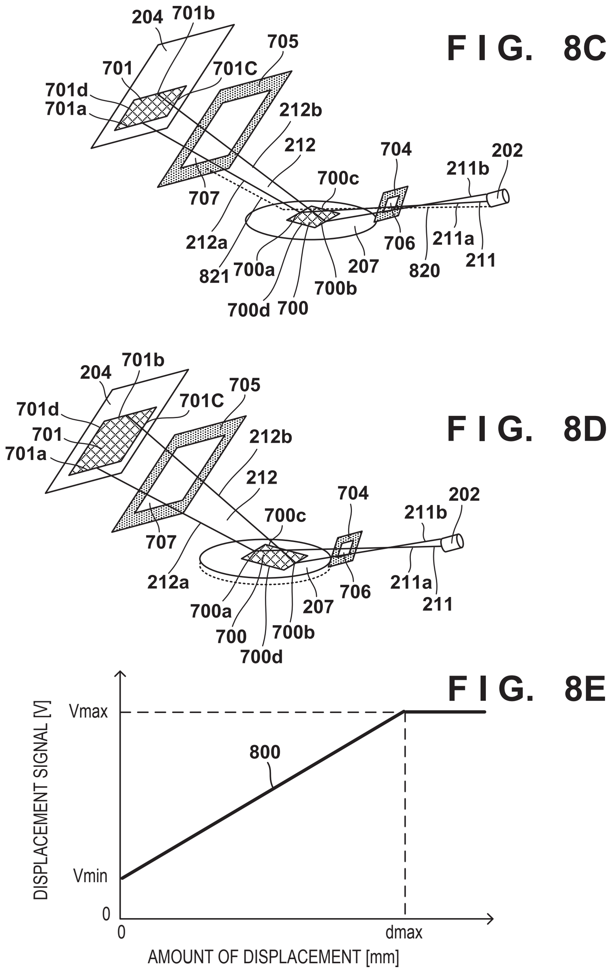

A second variation on the chest piece 110 will be described with reference to FIGS. 8C to 8E. FIGS. 8C to 8E primarily illustrate the differences from FIGS. 7B, 7D, and 5.

Light passing through the opening 706 along the lower side of the opening 706 is specularly reflected by the reflective medium 207, and then reaches the illuminated region 701 of the photodetector 204 without being shielded by the light shield 705. Accordingly, the lower side of the opening 706 defines the upper boundary line 701b of the illuminated region 701. On the other hand, as illustrated in FIG. 8C, light 820 passing through the opening 706 along the upper side of the opening 706 is specularly reflected by the reflective medium 207 to become light 821, which is shielded by the light shield 705 and does not reach the photodetector 204. Accordingly, the upper side of the opening 706 does not define the illuminated region 701. The same applies in FIG. 8D.

After passing through the opening 706 and being specularly reflected by the reflective medium 207, the light passing through the opening 707 along the lower side of the opening 707 reaches the lower boundary line 701a of the illuminated region 701 of the photodetector 204. Accordingly, the lower side of the opening 707 defines the lower boundary line 701a of the illuminated region 701. On the other hand, being shielded by the light shield 704, light does not pass through the part along the lower side of the opening 707. Accordingly, the upper side of the opening 707 does not define the illuminated region 701.

As illustrated in FIGS. 8C and 8D, the positions of the effective range 700, the far boundary line 700a, the near boundary line 700b, the illuminated region 701, the lower boundary line 701a, and the upper boundary line 701b change when the diaphragm 206 is pressed by the body surface 300. As described above, the upper boundary line 701b is defined by the lower side of the opening 706 in the aperture 209 on the light emitter 202 side. The upper boundary line 701b moves in accordance with the displacement of the contact surface 206a. On the other hand, the lower boundary line 701a and the side boundary lines 701c and 701d substantially do not move in accordance with the displacement of the contact surface 206a. The upper boundary line 701b is displaced by the displacement gain G relative to the displacement amount d2 of the diaphragm 206. The displacement gain G has the same value as in Table 1 above.

Relationship between Displacement Amount of Surface of Living Body and Displacement Signal in Electronic Auscultation Apparatus According to Second Variation

A relationship between the displacement amount of the body surface 300 and the displacement signal will be described with reference to FIG. 8E. The displacement signal represents a voltage output from the light-receiving circuit board 205. A graph 800 in FIG. 8E represents the relationship between the displacement amount of the body surface 300 and the displacement signal. The horizontal axis of the graph 800 represents the displacement amount of the body surface 300, and represents the displacement signal generated by the light-receiving circuit board 205.

When the displacement amount of the body surface 300 is zero, the displacement signal has a value based on the area of the illuminated region 701 illustrated in FIG. 8C. When the displacement amount of the contact surface 206a increases, the upper end reflected light 212b moves in a direction away from the diaphragm 206, and the area of the illuminated region 701 increases monotonically and linearly. In other words, of the light emitted by the light emitter, the amount of light that reaches the photodetector 204 increases monotonically and linearly. Accordingly, the displacement signal output from the photodetector 204 also increases monotonically and linearly. After that, once the upper end reflected light 212b reaches the light shield 705, the displacement signal becomes constant.

Third Variation on Electronic Auscultation Apparatus of First Embodiment

In the chest piece 110 according to the third variation, the light shield 705 is omitted. Accordingly, the chest piece 110 does not have the aperture 210. Light passing through the opening 706 along the lower side of the opening 706 is specularly reflected by the reflective medium 207, and then reaches the upper side of the illuminated region 701 of the photodetector 204. Accordingly, the lower side of the opening 706 defines the upper side of the illuminated region 701. On the other hand, light passing through the opening 706 along the upper side of the opening 706 is specularly reflected by the reflective medium 207, and then reaches the outer side of the photodetector 204. Accordingly, the upper side of the opening 706 does not define the illuminated region 701. The lower side of the illuminated region 701 is defined by the lower side of the photodetector 204. In addition, the left side and the right side of the illuminated region 701 are defined by the left side and the right side of the photodetector 204. Instead, however, the left side and the right side of the illuminated region 701 may be defined by the left side and the right side of the opening 706.

Fourth Variation on Electronic Auscultation Apparatus of First Embodiment

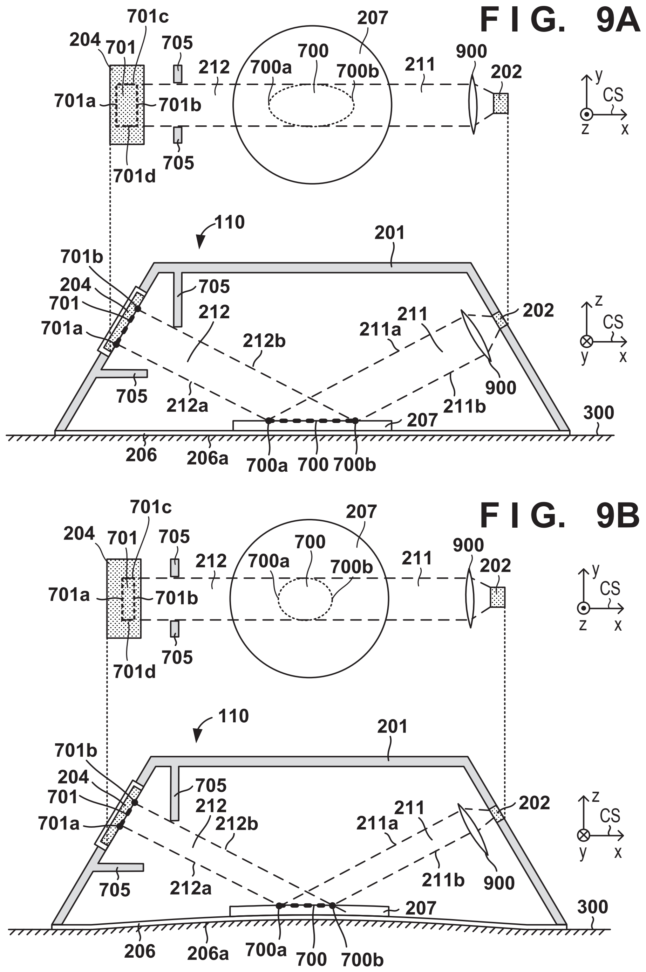

A fourth variation on the chest piece 110 will be described with reference to FIGS. 9A and 9B. FIGS. 9A and 9B primarily illustrate the differences from FIGS. 7A and 7C.

In the example in FIGS. 9A and 9B, the chest piece 110 further includes a lens 900 between the light emitter 202 and the reflective medium 207. The lens 900 converts the diffuse light emitted by the light emitter 202 into parallel light. Because this parallel light reaches the reflective medium 207, the aperture 209 on the incident light 211 side can be omitted from the chest piece 110.

Changes in the illuminated region 701 are the same as illustrated in FIGS. 7A and 7C. Accordingly, the range of light that reaches the photodetector 204 (i.e., the illuminated region 701) changes in the x'-axis direction as the reflective medium 207 moves.

Summary of First Embodiment and First to Fourth Variations

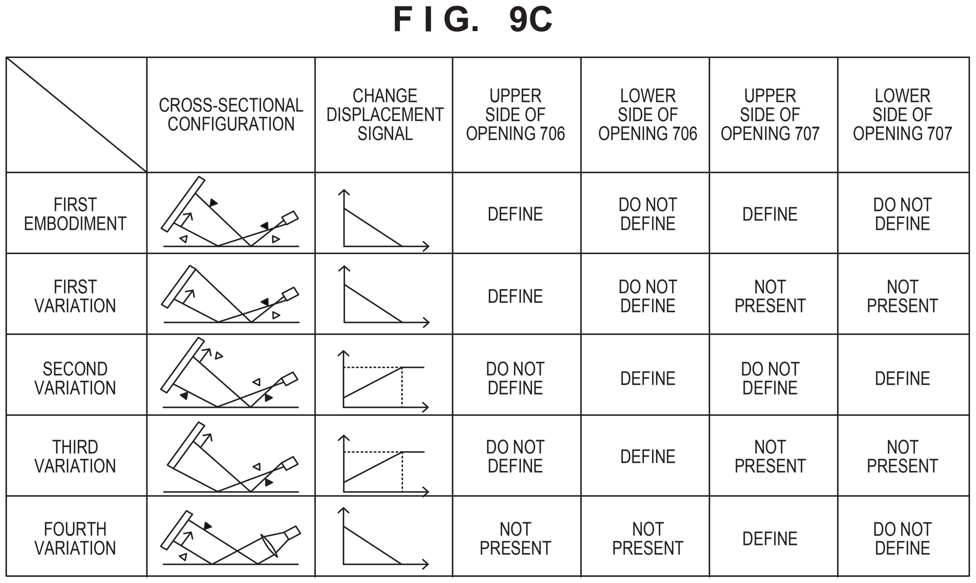

FIG. 9C is a diagram illustrating an overview of whether the upper side and the lower side of the openings 706 and 707 define the illuminated region 701 in the first embodiment and the first to fourth variations. The "cross-sectional configuration" in FIG. 9C indicates the cross-sectional configuration of the light emitter 202, the photodetector 204, the reflective medium 207, the opening 706, and the opening 707 of each configuration in a simplified manner. Of upper side and the lower side of the opening 706 and the opening 707, the sides that define the illuminated region 701 are indicated by black triangles, and the sides that do not define the illuminated region 701 are indicated by white triangles. The "change in displacement signal" in FIG. 9C indicates whether the displacement signal decreases monotonically as per the graph 500 in FIG. 5 or increases monotonically as per the graph 800 in FIG. 8E.

In the embodiment described above, the surface normal of the light-receiving surface of the photodetector 204 is inclined with respect to the z-axis direction (i.e., the normal direction of the diaphragm 206). Instead, however, the surface normal of the light-receiving surface of the photodetector 204 may coincide with the z-axis direction. In other words, the light-receiving surface is parallel to the diaphragm 206.

In the embodiment described above, the chest piece 110 includes only one light emitter 202. Instead, the chest piece 110 may include a plurality of light emitters 202. The light emitted by each of the plurality of light emitters 202 may be reflected by the reflective medium 207 and reach the photodetector 204. The photodetector 204 may detect a displacement of the diaphragm 206 on the basis of the total amount of this light. The chest piece 110 may include a plurality of photodetectors 204. Each of the plurality of photodetectors 204 may receive the light emitted from an individual light emitter 202 and reflected by the reflective medium 207. The displacement of the diaphragm 206 may be detected on the basis of the total amount of light received by the plurality of photodetectors 204.

In the embodiment described above, the electronic stethoscope 100 generates a displacement signal (i.e., a signal representing the displacement of the diaphragm 206) on the basis of an amount of light emitted from the light emitter 202, reflected by the reflective medium 207, and received by the photodetector 204. Instead, the electronic stethoscope 100 may detect the displacement signal on the basis of the light received by the photodetector 204 in a different manner. For example, the light emitter 202 may emit a laser beam toward the reflective medium 207. The laser beam reflected by the reflective medium 207 reaches the photodetector 204. The position where the laser beam reaches the photodetector 204 varies depending on the displacement of the diaphragm 206. Accordingly, the electronic stethoscope 100 may detect the displacement signal on the basis of the position of the light received by the photodetector 204. If the light emitter 202 emits a laser beam, the chest piece 110 need not include the apertures 209 and 210. If the light emitter 202 emits a laser beam, the photodetector 204 may be a two-dimensional area sensor or a one-dimensional line sensor.

Second Embodiment

Hardware Configuration of Electronic Auscultation Apparatus of Second Embodiment

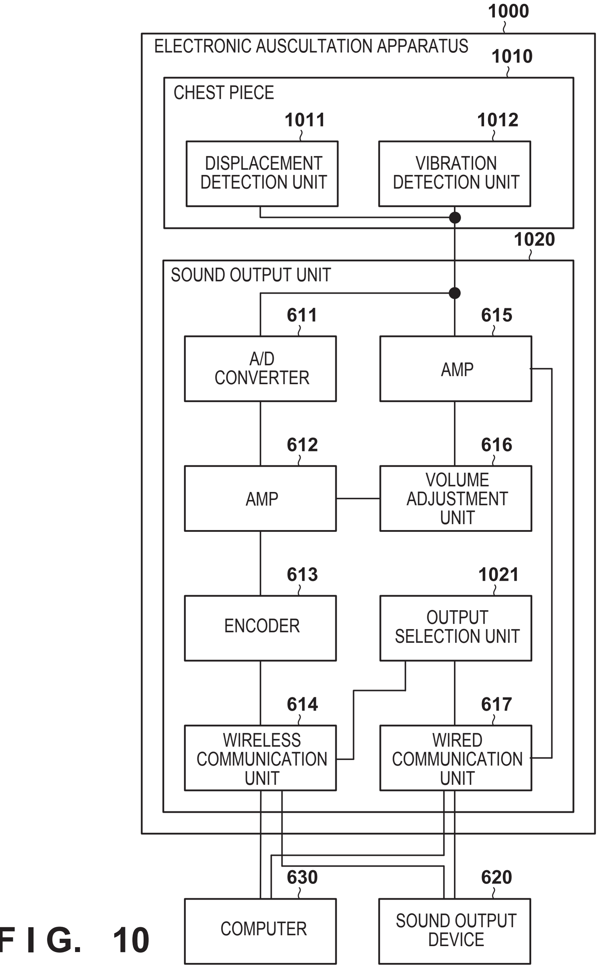

An example of the configuration of an electronic stethoscope 1000 according to a second embodiment will be described with reference to FIGS. 10 to 13K. The following will primarily describe the differences from the electronic stethoscope 100 according to the first embodiment. The external appearance of the electronic stethoscope 1000 is similar to that of the electronic stethoscope 100 described with reference to FIGS. 1A and 1B. The variations described in the first embodiment may also be applied to the second embodiment.

The hardware configuration of the electronic stethoscope 1000 will be described with reference to FIG. 10. FIG. 10 differs from FIG. 6 in that the electronic stethoscope 1000 includes a chest piece 1010 and a audio output unit 1020 instead of the chest piece 110 and the audio output unit 610 of the electronic stethoscope 100. The electronic stethoscope 1000 is constituted by the chest piece 1010, and the audio output unit 1020 included in the grip 120 (FIGS. 1A and 1B). The chest piece 1010 includes a displacement detection unit 1011 and a vibration detection unit 1012. As in the electronic stethoscope 100, the displacement detection unit 1011 is constituted by primary components including the light emitter 202, the photodetector 204, and the reflective medium 207. The displacement detection unit 1011 generates a displacement signal (i.e., a signal representing the displacement of the diaphragm 206) on the basis of an amount of light emitted from the light emitter 202, reflected by the reflective medium 207, and received by the photodetector 204. Instead, the displacement detection unit 1011 may detect the displacement signal on the basis of the light received by the photodetector 204 in a different manner. For example, the light emitter 202 may emit a laser beam toward the reflective medium 207. The laser beam reflected by the reflective medium 207 reaches the photodetector 204. The position where the laser beam reaches the photodetector 204 varies depending on the displacement of the diaphragm 206. Accordingly, the displacement detection unit 1011 may detect the displacement signal on the basis of the position of the light received by the photodetector 204. If the light emitter 202 emits a laser beam, the chest piece 1010 need not include the apertures 209 and 210. If the light emitter 202 emits a laser beam, the photodetector 204 may be a two-dimensional area sensor or a one-dimensional array sensor.

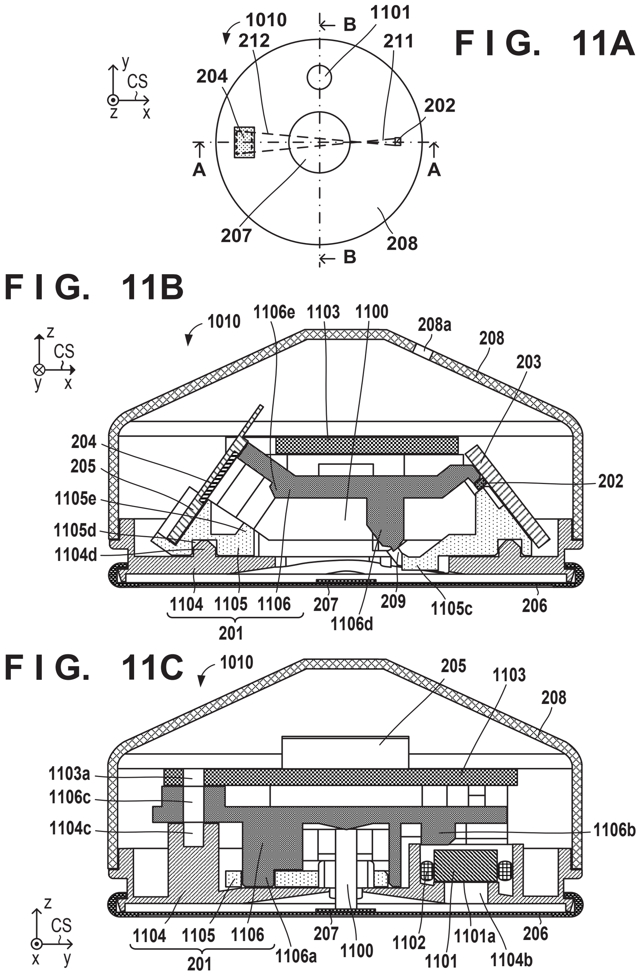

The vibration detection unit 1012 detects air vibrations produced by the displacement of the diaphragm 206. Because the vibration detection unit 1012 detects sounds produced by air vibrations, the vibration detection unit 1012 will also be called a "sound detection unit". The vibration detection unit 1012 is constituted by a condenser microphone 1101 (FIG. 11C), for example. The vibration detection unit 1012 generates a sound signal representing the detected air vibration. The sound signal may be an electrical signal (e.g., a voltage signal). The displacement signal generated by the displacement detection unit 1011 and the sound signal generated by the vibration detection unit 1012 are supplied to the audio output unit 1020.

The audio output unit 1020 performs the same signal processing as that performed by the audio output unit 610 of the electronic stethoscope 100 on the displacement signal and the sound signal supplied from the chest piece 1010, and transmits the resulting signals to the exterior of the audio output unit 1020 (e.g., the audio output device 620 or the computer 630). The frequency bands of high sensitivity can be different between the displacement detection unit 1011 and the vibration detection unit 1012. For example, the displacement detection unit 1011 can detect vibrations in low frequency bands (e.g., 100 Hz or less; cardiac sounds, for example) with a higher level of sensitivity than the vibration detection unit 1012. On the other hand, the vibration detection unit 1012 can detect vibrations in high frequency bands (e.g., 100 Hz or more; respiratory sounds, for example) with a higher level of accuracy than the displacement detection unit 1011. Accordingly, because the audio output unit 1020 can output both the displacement signal and the sound signal, the electronic stethoscope 1000 can accurately detect vibrations over a wide range of frequency bands.

The audio output unit 1020 includes an output selection unit 1021 in addition to the constituent elements included in the audio output unit 610 of the electronic stethoscope 100. The output selection unit 1021 selects which of the displacement signal and the sound signal supplied from the chest piece 1010 is to be output to the exterior. For example, when the transmission destination is the audio output device 620, the output selection unit 1021 outputs only the selected one of the displacement signal and the sound signal. If the transmission destination is the computer 630, the output selection unit 1021 may output only the selected one of the displacement signal and the sound signal, or may output both the displacement signal and the sound signal. In this manner, the audio output unit 1020 can selectively output the displacement signal and the sound signal to the exterior.

The operation unit 123 of the electronic stethoscope 1000 obtains a designation from the user as to which of the displacement signal and the sound signal to output. In the present embodiment, the operation unit 123 in FIG. 1A is provided with the mode switch button 123c. In response to the mode switch button 123c being pressed, the output selection unit 1021 switches the signal output to the external device between the displacement signal and the sound signal. For example, when listening to a cardiac sound, the user operates the mode switch button 123c provided in the operation unit 123 to designate a cardiac sound mode, which is one auscultation mode. On the other hand, when listening to a respiratory sound, the user operates the mode switch button 123c provided in the operation unit 123 to designate a respiratory sound mode, which is one auscultation mode. Information on the operating mode designated by the user is sent to the output selection unit 1021. On the basis of this operating mode information, the output selection unit 1021 selects whether to output the displacement signal representing the displacement detected by the displacement detection unit 1011 or the sound signal detected by the vibration detection unit 1012. The operation unit 123 is also provided with volume adjustment buttons 123a and 123b for adjusting the gain of the displacement signal output by the electronic stethoscope 100. The volume adjustment buttons 123a and 123b are used to adjust the volume of sounds output by the electronic stethoscope 100. Furthermore, the display device 122 of the electronic stethoscope 1000 is provided with an LED as an indicator to display whether the signal currently selected for output is the displacement signal or the sound signal. The user can visually confirm whether the current mode of operation is the cardiac sound mode or the respiratory sound mode on the basis of the illumination state of the LED. Note that the cardiac sound mode and the respiratory sound mode are examples of auscultation modes.

The output selection unit 1021 controls the wireless communication unit 614 and the wired communication unit 617, and determines the type of signal to be output to the exterior. Instead, the output selection unit 1021 may determine which of the displacement signal and the sound signal to supply to the A/D converter 611 and the amp 615. Furthermore, although the audio output unit 1020 includes the output selection unit 1021 in the example in FIG. 10, the chest piece 1010 may include the output selection unit 1021 instead. The output selection unit 1021 in the chest piece 1010 may determine the type of signal to be supplied to the audio output unit 1020.

Configuration of Chest Piece of Electronic Auscultation Apparatus of Second Embodiment

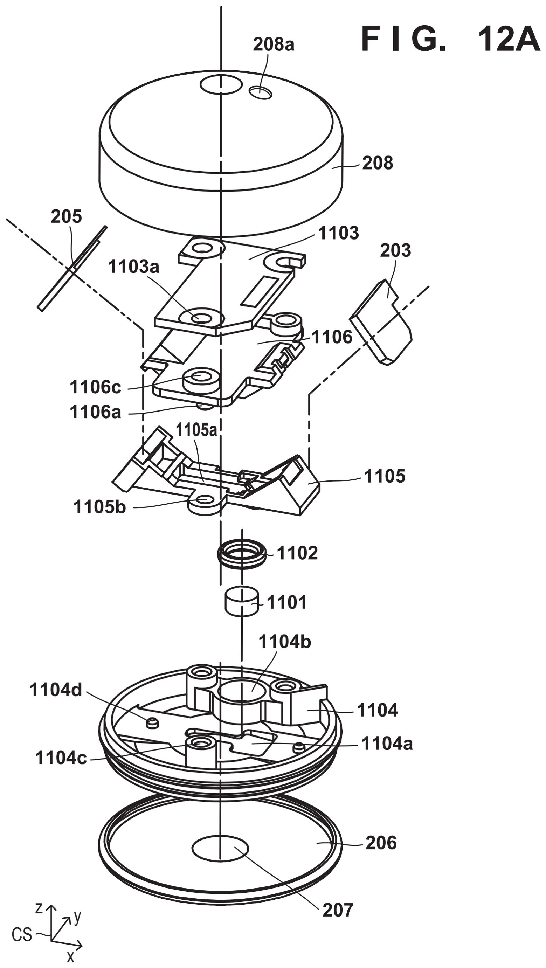

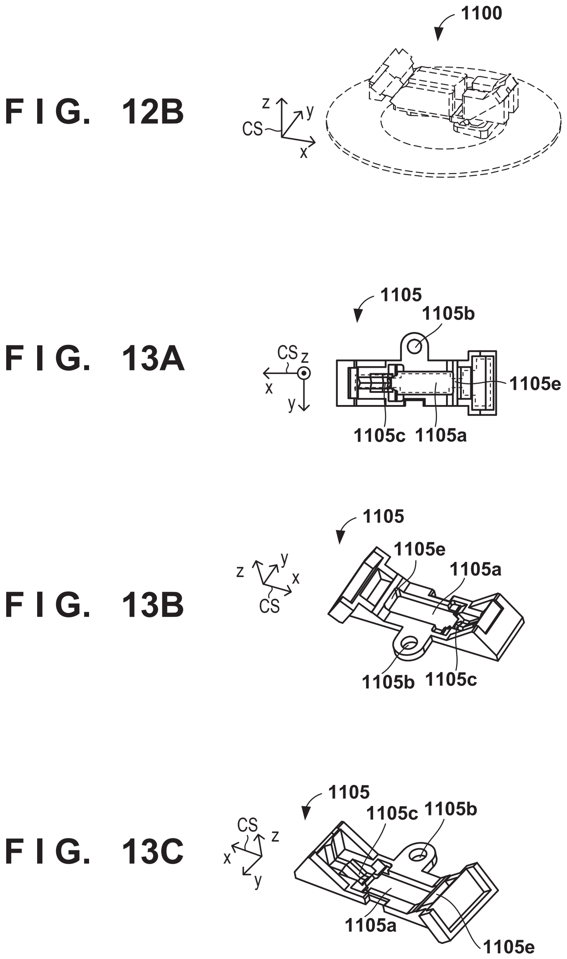

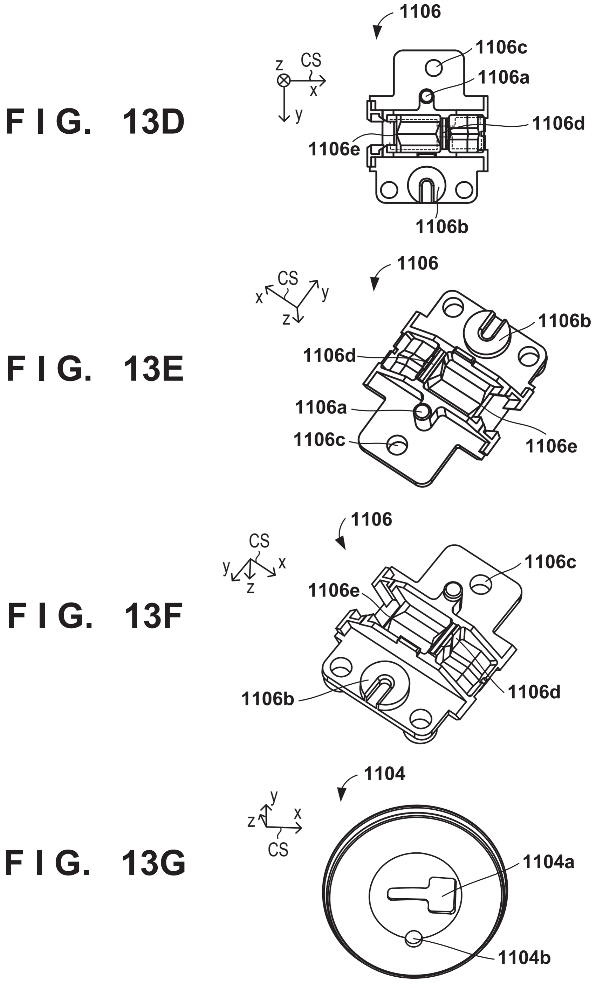

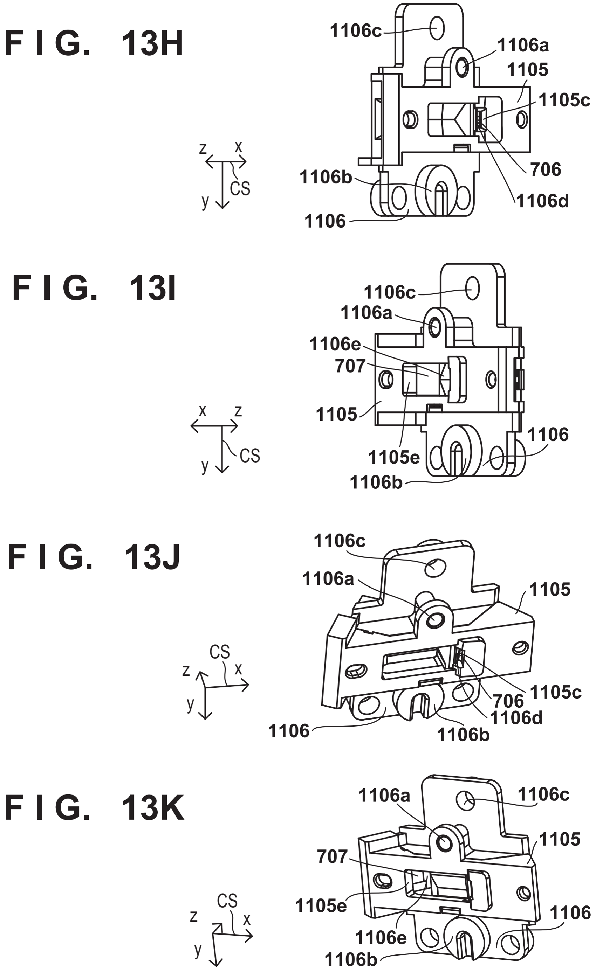

An example structure of the chest piece 1010 will be described with reference to FIGS. 11A to 13K. FIG. 11A is a plan view of the chest piece 1010. FIG. 11B is an enlarged cross-sectional view of the chest piece 1010 taken along line A-A in FIG. 11A. FIG. 11C is an enlarged cross-sectional view of the chest piece 1010 taken along line B-B in FIG. 11A. FIG. 12A is an exploded perspective view of the chest piece 1010, illustrating each constituent element separately. FIG. 12B is a perspective view illustrating the shape of a sealed space 1100. FIGS. 13A to 13C are perspective views of a lower holding member 1105 from various angles. FIGS. 13D to 13F are perspective views of an upper holding member 1106 from various angles. FIG. 13G is a perspective view of a base holding member 1104 from an angle. FIGS. 13H to 13K are perspective views of the lower holding member 1105 and the upper holding member 1106 in a coupled state, from various angles. In particular, FIG. 13H is a perspective view of the light emitter 202 seen through the opening 706 at an angle, and FIG. 13I is a perspective view of the photodetector 204 seen through the opening 707 at an angle. In FIG. 11A, the light emitter 202, the photodetector 204, the reflective medium 207, and the condenser microphone 1101 are illustrated in a visible manner to facilitate understanding, but these constituent elements are obscured by the housing 208. In the cross-sections in FIGS. 11B and 11C, the same constituent elements are given the same hatching, and different constituent elements are given different hatching, to facilitate understanding. The mechanism for attaching the chest piece 1010 to the grip 120 is not illustrated in FIGS. 11A to 13K.

The chest piece 1010 further includes the condenser microphone 1101, a sealing member 1102, and a relay board 1103 in addition to the constituent elements included in the chest piece 110 of the electronic stethoscope 100. As described above, the condenser microphone 1101 constitutes the vibration detection unit 1012.

The relay board 1103 is connected to the light-emitting circuit board 203 by a lead line (not shown). The relay board 1103 transmits a control signal for instructing light to be emitted to the light-emitting circuit board 203, and supplies power, over the lead line. The relay board 1103 is also connected to the light-receiving circuit board 205 by a lead line (not shown). The relay board 1103 receives the displacement signal from the light-receiving circuit board 205, and supplies power to the light-receiving circuit board 205, over the lead line. The relay board 1103 is also connected to the condenser microphone 1101 by a lead line (not shown). The relay board 1103 receives the sound signal from the condenser microphone 1101, and supplies power to the condenser microphone 1101, over the lead line. The relay board 1103 is also connected to the circuit board in the grip 120 by a cable (not shown). The relay board 1103 transmits the displacement signal and the sound signal to the circuit board in the grip 120, receives the control signal for controlling the operation of the chest piece 110 from the circuit board in the grip 120, and receives the supply of power over this cable. A hole 208a for passing cables connecting the relay board 1103 and the circuit board in the grip 120 is formed in the housing 208. Unless specified otherwise, "hole" herein refers to a through-hole that penetrates the member in which the hole is formed. On the other hand, "recess" means that the recess does not penetrate the member in which the recess is formed.

The holding member 201 is constituted by the base holding member 1104, the lower holding member 1105, and the upper holding member 1106. The diaphragm 206 is attached to the base holding member 1104 so as to cover a lower surface of the base holding member 1104. The outer periphery of the diaphragm 206 is bent so as to form a recess, and the diaphragm 206 is attached to the base holding member 1104 by fitting the projection of the outer periphery of the base holding member 1104 with the recess of the diaphragm 206. Instead, however, the diaphragm 206 may be attached to the base holding member 1104 using an adhesive, for example. Because a gap is present between the lower surface of the base holding member 1104 and the diaphragm 206, the diaphragm 206 can displace relative to the lower surface of the base holding member 1104. Additionally, the lower surface of the base holding member 1104 has a depression near the center thereof so as not to inhibit the displacement of the diaphragm 206.

The housing 208 is attached to the base holding member 1104 so as to cover the upper surface of the base holding member 1104. The base holding member 1104 has a thread or a thread groove in the outer periphery thereof, and the housing 208 has a thread groove or thread on the inner side of the lower end thereof. The thread and the thread groove fit with each other to attached the housing 208 to the base holding member 1104. Instead, however, the housing 208 may be attached to the base holding member 1104 using an adhesive, for example. The housing 208 covers the light emitter 202, the light-emitting circuit board 203, the photodetector 204, the light-receiving circuit board 205, and the condenser microphone 1101 attached to the holding member 201, and has a function of blocking sound so that ambient sounds are not detected as noise.

Two holding members (the lower holding member 1105 and the upper holding member 1106) are further disposed above the base holding member 1104. The lower holding member 1105 is located on the opposite side from the diaphragm 206 with respect to the base holding member 1104 in the z direction. A projection 1104d formed in the upper surface of the base holding member 1104 fits with a recess 1105d formed in the lower surface of the lower holding member 1105, which aligns the lower holding member 1105 with the base holding member 1104.

The upper holding member 1106 is disposed above the base holding member 1104 and the lower holding member 1105 in the z direction. The upper holding member 1106 is located on the opposite side from the diaphragm 206 with respect to the base holding member 1104. The lower holding member 1105 is located between the upper holding member 1106 and the base holding member 1104. A projection 1106a formed in the lower surface of the upper holding member 1106 fits with a hole 1105b formed in the lower holding member 1105, which aligns the upper holding member 1106 with the lower holding member 1105.

The relay board 1103 is disposed on the upper holding member 1106. A screw (not shown) passing through a hole 1103a formed in the relay board 1103 and a hole 1106c formed in the upper holding member 1106 is threaded into a screw hole 1104c formed in the base holding member 1104. In the example illustrated here, three screw holes are formed in the base holding member 1104, and screws passing through the relay board 1103 and the upper holding member 1106 are threaded into those screw holes. As a result, the lower holding member 1105, the upper holding member 1106, and the relay board 1103 are fixed to the base holding member 1104. The method of fixing the lower holding member 1105, the upper holding member 1106, and the relay board 1103 to the base holding member 1104 is not limited thereto, and the members may be fixed using an adhesive, for example.

The light emitter 202 is inserted into a hole formed by engaging the upper holding member 1106 with the lower holding member 1105. The photodetector 204 is disposed to cover a hole formed when the upper holding member 1106 is engaged with the lower holding member 1105. The condenser microphone 1101 is disposed in a hole 1104b formed in the base holding member 1104. The sealing member 1102 is disposed between the condenser microphone 1101 and the base holding member 1104 to cover a gap between those members, which improves the seal of the sealed space (described later). Additionally, a projection 1106b formed in the lower surface of the upper holding member 1106 is disposed above the hole 1104b of the base holding member 1104. The projection 1106b may press the condenser microphone 1101 downward (i.e., toward the diaphragm 206). The condenser microphone 1101 is disposed in a position that does not overlap with the optical path of the light from the light emitter 202 to the photodetector 204 via the reflective medium 207 (i.e., the incident light 211 and the reflected light 212) when the diaphragm 206 is viewed in plan view (i.e., from the positive side in the z-axis direction).

A T-shaped hole 1104a is formed near the center of the base holding member 1104. A hole 1105a is formed near the center of the lower holding member 1105. The incident light 211 reaches the reflective medium 207 from the light emitter 202 through the hole 1104a and the hole 1105a. The reflected light 212 similarly reaches the photodetector 204 from the reflective medium 207 through the hole 1104a and the hole 1105a. In this manner, no other constituent elements are disposed on the optical path of the light from the light emitter 202 to the photodetector 204 via the reflective medium 207 (i.e., the incident light 211 and the reflected light 212). The base holding member 1104 covers a region other than the region where the reflective medium 207 is fixed to the inner surface 206b of the diaphragm 206.

The upper holding member 1106 has a light shield 1106d that extends in a direction intersecting with the diaphragm 206 (an yz plane in the example in FIG. 11B). In particular, the part of the light shield 1106d on the upper side of the opening 706 corresponds to a first aperture. The lower holding member 1105 has a light shield 1105c that extends in a direction parallel to the diaphragm 206 (the xy plane in the example in FIG. 11B). The aperture 209 on the incident light 211 side is formed by the light shield 1106d and the light shield 1105c. In other words, each of the light shield 1106d and the light shield 1105c blocks some of the light emitted by the light emitter 202. Part of the light shield 1105c may enter into the hole 1104a of the base holding member 1104. This makes it possible to bring the aperture 209 closer to the reflective medium 207, and reduce the spread of the incident light 211. In addition, the upper holding member 1106 has a light shield 1106e that protrudes downward in the z direction at the position where the reflected light from the reflective medium 207 reaches. The lower holding member 1105 has a light shield 1105e at the position where the reflected light from the reflective medium 207 reaches. The aperture 210 on the reflected light 212 side is formed by the light shield 1106e and the light shield 1105e. In other words, each of the light shield 1106e and the light shield 1105e blocks some of the light specularly reflected by the reflective medium 207. The light shield 1106d provided in the upper holding member 1106 and the light shield 1105c provided in the lower holding member 1105 serve as an aperture that shields the incident light 211 and the reflected light 212, and also serve to narrow the sealed space. In other words, the light shield 1105c and the light shield 1106d reduce the volume of the sealed space (the internal space), which increases a volume change rate (described later), and as a result, the sensitivity of the condenser microphone 1101 can be improved.

In the second embodiment, the sealed space 1100 is defined by the diaphragm 206, the reflective medium 207, the base holding member 1104, the lower holding member 1105, the upper holding member 1106, the light emitter 202, the photodetector 204, and the condenser microphone 1101. Accordingly, the space facing the inner surface 206b of the diaphragm 206 is the sealed space. "Sealed space" refers to a space that is sealed against a specific substance. In other words, the sealed space is a space in which the inflow of a specific substance from an outside space or an outside environment, and the outflow of a specific substance to the outside space or the outside environment, are inhibited. Accordingly, there are no passages such as gaps or holes that connect the sealed space 1100 to the outside. The substances to be blocked by the seal may differ depending on the usage environment of the electronic stethoscope 1000. For example, if the electronic stethoscope 1000 is used at atmospheric pressure, the substance to be sealed is a gas (e.g., air), and if the electronic stethoscope 1000 is also used in water, the substance to be sealed can be a liquid (e.g., water).

Sealed Space within Chest Piece of Electronic Auscultation Apparatus of Second Embodiment

In FIGS. 11B and 11C, the space facing the inner surface 206b of the diaphragm 206 and surrounded by the hatched constituent elements is the sealed space 1100. The part of the lower holding member 1105 that defines the sealed space 1100 includes the light shield 1105c described above. The part of the upper holding member 1106 that defines the sealed space 1100 includes the light shield 1106d described above. When a gap is present between the light emitter 202 and the holding member 201, the sealed space 1100 is also defined by the light-emitting circuit board 203. Similarly, when a gap is present between the photodetector 204 and the holding member 201, the sealed space 1100 is also defined by the light-receiving circuit board 205. When a gap is present between the condenser microphone 1101 and the holding member 201, the sealed space 1100 is also defined by the sealing member 1102.

The sealed space 1100 need not be sealed when the electronic stethoscope 100 is not in use. For example, the diaphragm 206 and the base holding member 1104 need not be in close contact when the electronic stethoscope 100 is not in use, but may come into close contact, forming the sealed space 1100 as a result, when the diaphragm 206 is pressed against the surface of a target body.

A sound detection surface 1101a of the condenser microphone 1101 faces the sealed space 1100. As a result, the condenser microphone 1101 detects air vibrations in an internal space 212 produced by the displacement of the contact surface 206a of the diaphragm 206. The condenser microphone 1101 generates a sound signal expressing these air vibrations.

Specifically, the part of the diaphragm 206 that contacts the surface of the target body is constituted by a sheet-shaped flexible member. The diaphragm 206 separates the sealed space 1100 from the exterior of the chest piece 1010. The diaphragm 206 has a property of deforming under an external force applied to the diaphragm 206 (e.g., a force from the surface of a target body) and returning to its original shape when the external force is removed. By having this property, the air pressure of the sealed space 1100 changes in response to vibrations transmitted from the surface of the target body to the diaphragm 206. The sound detection surface 1101a of the condenser microphone 1101 vibrates in response to the air pressure of the sealed space 1100, and the condenser microphone 1101 converts the vibration of the sound detection surface 1101a into an electrical signal.

The condenser microphone 1101 can efficiently detect the sound signal in response to the displacement of the surface of the target body by having the inner surface 206b of the diaphragm 206 and the sound detection surface 1101a of the condenser microphone 1101 face the sealed space 1100.

The change in the air pressure of the sealed space 1100 in response to the vibration of the diaphragm 206 increases as the volume of the sealed space 1100 decreases. Accordingly, the sound detected by the condenser microphone 1101 also becomes louder. As such, in the present embodiment, the sealed space 1100 is configured such that the volume of the sealed space 1100 is smaller than the volume of the internal space of the chest piece 110. In other words, in the present embodiment, the sealed space 1110 does not face the housing 208, and is separated from a space facing the inner surface of the housing 208. However, it goes without saying that the space facing the inner surface of the housing 208 may be used as the sealed space.

Although the internal space other than the sealed space 1110 of the chest piece 110 is not sealed in the present embodiment, this internal space may be sealed if the electronic stethoscope 1000 is required to be waterproof or dustproof, for example. For example, as illustrated in FIG. 11B, the hole 208a for passing a cable through is formed in the housing 208. The gap between the hole 208a and the cable can be sealed with a sealing member to seal the internal space of the chest piece 110 against water or dust. In addition to the hole 208a, when screws are used to fix the housing 208 to the base holding member 1104, the gaps between the thread and the thread groove can similarly be sealed with a sealing member to improve the seal.

Although the space facing the inner surface 206b of the diaphragm 206 is the sealed space 1100 in the present embodiment, holes and gaps of sizes that do not reduce the sensitivity in the frequency band to be measured may be formed, for example.

In the present embodiment, the space facing the inner surface 206b of the diaphragm 206 is sealed using the base holding member 1104, the lower holding member 1105, and the upper holding member 1106. Instead, however, the space may be sealed by some of these holding members, or the space may be sealed using another member. Additionally, to improve the adhesion among the plurality of constituent elements defining the sealed space 1100, a cushioning member may be disposed between two adjacent constituent elements. For example, a cushioning member may be disposed in the gap between the contact parts of the light-emitting circuit board 203 and the holding member 201, the gap between the contact parts of the light-receiving circuit board 205 and the holding member 201, and the gap between the contact parts of the diaphragm 206 and the holding member 201. This makes it possible to increase the seal of the sealed space 1100.

Sound-Insulation Configuration of Electronic Auscultation Apparatus of Second Embodiment

The condenser microphone 1101 can detect not only air vibrations produced in the sealed space 1100, but also sound transmitted through the housing 208 from the exterior of the chest piece 1010. It is therefore preferable that the housing 208 be formed of a material having good sound insulation properties.

The sound transmission loss of the housing 208 is calculated by a function that takes the frequency of the sound, the incident angle of the sound, and the areal density of the material of the housing 208 as inputs. When the incident angle of the sound is vertical, the frequency of the sound is represented by f [Hz], and the areal density of the material of the housing 208 is represented by σ [kg/m²], sound transmission loss TL [db] of the housing 208 can be given as follows:

$$TL = 18 \times \log_{10} (\sigma \times f) - 44... \quad \text{(Formula 8)}$$

The areal density of the material of the housing 208 is calculated by multiplying the density of the material of the housing 208 by the thickness of the housing 208.

The areal density and transmission loss at various frequencies of the housing 208 when the housing 208 is formed of brass, stainless steel, aluminum, and polycarbonate ABS, respectively, at a thickness of 1.5 [mm], are shown in the following Table 2. Polycarbonate ABS is an example of a resin.

Table 2

| Material | Surface density [kg/m²] | Transmission loss at 50 Hz [dB] | Transmission loss at 100 Hz [dB] | Transmission loss at 200 Hz [dB] | Transmission loss at 500 Hz [dB] | Transmission loss at 800 Hz [dB] |
|---|---|---|---|---|---|---|
| Brass | 12.75 | 6.5 | 11.9 | 17.3 | 24.5 | 28.2 |
| Stainless steel | 11.085 | 5.4 | 10.8 | 16.2 | 23.4 | 27.1 |
| Aluminum | 4.05 | -2.5 | 2.9 | 8.4 | 15.5 | 19.2 |
| Polycarbonate ABS | 1.8 | -8.8 | -3.4 | 2 | 9.2 | 12.9 |

From Table 2, it can be seen that metal materials have better sound insulation properties than resin materials. It is therefore desirable that the housing 208 be constituted by a metal material. The areal density of the housing 208 may also be greater than the areal density of the diaphragm. Specifically, the areal density of the housing 208 may be at least 5 kg/m², and may furthermore be at least 10 kg/m². On the other hand, the holding member 201 may be constituted by a resin material to reduce the weight and cost of the chest piece 1010.

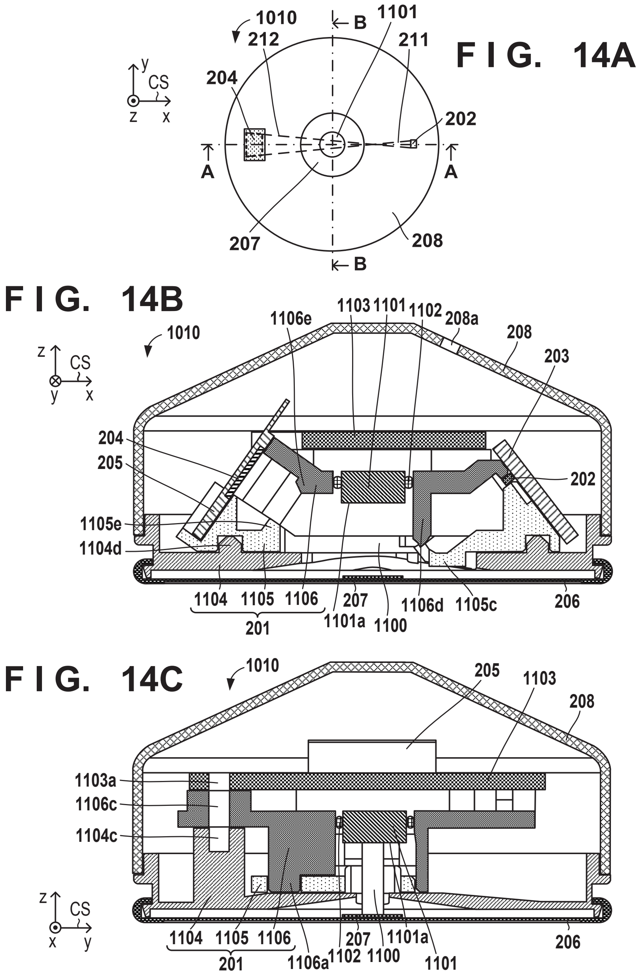

A plurality of variations on the chest piece 1010 will be described with reference to FIGS. 14A to 16C. The descriptions of each of the drawings in FIGS. 14A to 16C are similar to those of the drawings in FIGS. 11A to 11C, and thus redundant descriptions will be omitted.

The variation illustrated in FIGS. 14A to 14C differs from the foregoing descriptions of the second embodiment illustrated in FIGS. 11A to 11C in terms of the position of the condenser microphone 1101. The condenser microphone 1101 is disposed in a position that overlaps with the optical path of the light from the light emitter 202 to the photodetector 204 via the reflective medium 207 (i.e., the incident light 211 and the reflected light 212) when the diaphragm 206 is viewed in plan view (i.e., from the positive side in the z-axis direction). However, the condenser microphone 1101 is disposed above the optical path in the z-axis direction (the positive side of the z-axis) and therefore does not block the optical path. The condenser microphone 1101 is disposed between the light emitter 202 and the photodetector 204 in the x-axis direction. The sound detection surface 1101a of the condenser microphone 1101 faces the sealed space 1100 facing the inner surface 206b of the diaphragm 206. The volume of the sealed space 1100 can be reduced by disposing the condenser microphone 1101 in such a position, and the vibration of the diaphragm 206 can be accurately detected by the condenser microphone 1101.

Variation on Configuration of Chest Piece of Electronic Auscultation Apparatus of Second Embodiment

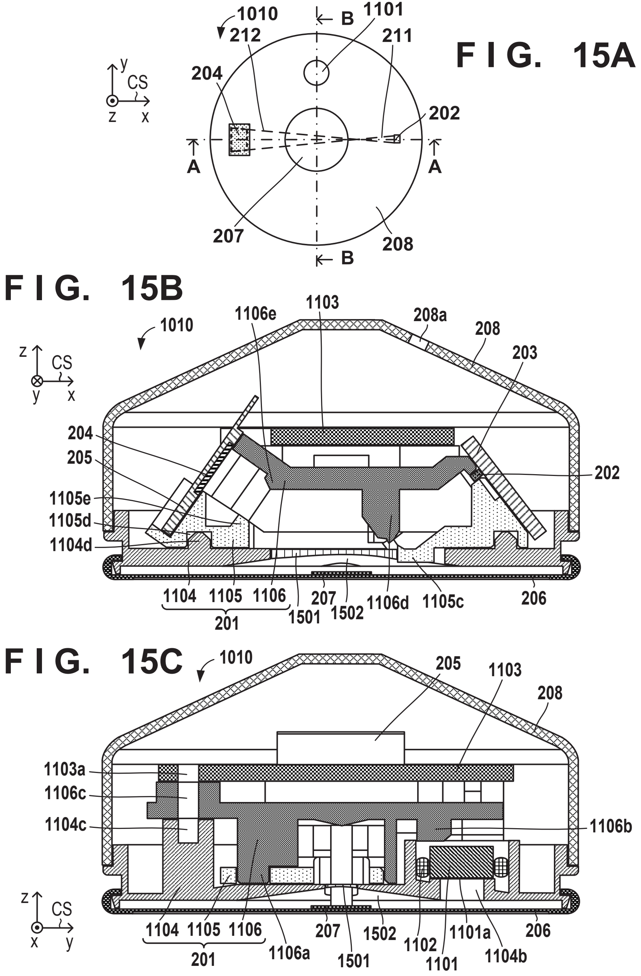

The variation illustrated in FIGS. 15A to 15C differs from the foregoing descriptions of the second embodiment illustrated in FIGS. 11A to 11C in that the chest piece 1010 further includes a light-transmitting member 1501. The light-transmitting member 1501 has a property of transmitting the light emitted by the light emitter 202. The light-transmitting member 1501 is formed of glass, acrylic, polystyrene, or the like, for example.

In the variation illustrated in FIGS. 15A to 15C, the hole 1104a in the base holding member 1104 is sealed by the light-transmitting member 1501 and the lower holding member 1105. Accordingly, a sealed space 1502 is defined by the diaphragm 206, the base holding member 1104, the lower holding member 1105, the light-transmitting member 1501, the condenser microphone 1101, and the sealing member 1102. The inner surface 206b of the diaphragm 206 faces the sealed space 1502. On the other hand, the light emitter 202 and the photodetector 204 do not face the sealed space 1502. The sound detection surface 1101a of the condenser microphone 1101 faces the sealed space 1502 facing the inner surface 206b of the diaphragm 206 in this variation as well.

The light-transmitting member 1501 is disposed in the optical path of the light from the light emitter 202 to the photodetector 204 via the reflective medium 207 (i.e., the incident light 211 and the reflected light 212). However, the light-transmitting member 1501 transmits light, and the displacement detection unit 1011 can therefore still detect the displacement of the diaphragm 206. The volume of the sealed space 1502 is smaller than the volume of the sealed space 1100, and thus the vibration of the diaphragm 206 can be detected more accurately by the condenser microphone 1101.

Other Variation on Configuration of Chest Piece of Electronic Auscultation Apparatus of Second Embodiment

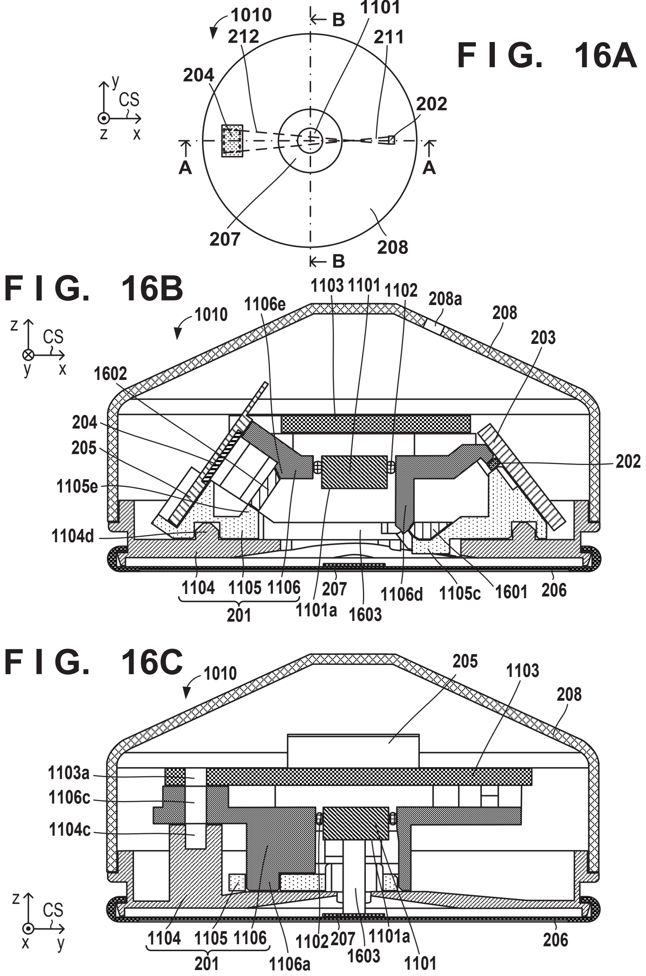

The variation illustrated in FIGS. 16A to 16C differs from the foregoing descriptions of the embodiments illustrated in FIGS. 14A to 14C in that the position of the condenser microphone 1101 is different, and the chest piece 1010 further includes light-transmitting members 1601 and 1602. The condenser microphone 1101 is disposed in a position that overlaps with the optical path of the light from the light emitter 202 to the photodetector 204 via the reflective medium 207 (i.e., the combined light of the incident light 211 and the reflected light 212) when the diaphragm 206 is viewed in plan view (i.e., from the positive side in the z-axis direction). The condenser microphone 1101 is disposed above the optical path (the positive side of the z-axis) and therefore does not block the optical path. For example, the condenser microphone 1101 may be disposed between the light emitter 202 and the photodetector 204 in the three-dimensional space.

The light-transmitting members 1601 and 1602 have a property of transmitting the light emitted by the light emitter 202. The light-transmitting members 1601 and 1602 are formed of glass, acrylic, polystyrene, or the like, for example.

In the variation illustrated in FIGS. 16A to 16C, the gap between the light shield 1106d and the light shield 1105c, which constitute the aperture 209 on the incident light side, is sealed by the light-transmitting member 1601. Likewise, the gap between the light shield 1106e and the light shield 1105e, which constitute the aperture 210 on the reflected light side, is sealed by the light-transmitting member 1602. Accordingly, a sealed space 1603 is defined by the diaphragm 206, the base holding member 1104, the lower holding member 1105, the light-transmitting member 1601, the light-transmitting member 1602, the condenser microphone 1101, and the sealing member 1102. The inner surface 206b of the diaphragm 206 faces the sealed space 1603. On the other hand, the light emitter 202 and the photodetector 204 do not face the sealed space 1603. The sound detection surface 1101a of the condenser microphone 1101 faces the sealed space 1603 facing the inner surface 206b of the diaphragm 206 in the variation illustrated in FIGS. 16A to 16C as well.

The light-transmitting member 1601 is disposed in the optical path of the light from the light emitter 202 to the reflective medium 207 (i.e., the incident light 211). The light-transmitting member 1601 is disposed in the optical path of the light from the reflective medium 207 to the photodetector 204 (i.e., the reflected light 212). However, the light-transmitting members 1601 and 1602 transmit light, and the displacement detection unit 1011 can therefore still detect the displacement of the diaphragm 206. The volume of the sealed space 1603 is smaller than the volume of the sealed space 1100, and thus the vibration of the diaphragm 206 can be detected more accurately by the condenser microphone 1101. Although the present embodiment describes an example in which the chest piece 1010 includes both the light-transmitting member 1601 and the light-transmitting member 1602, the configuration may include only one of these light-transmitting members.

Relationship between Volume of Sealed Space in Chest Piece of and Sensitivity of Microphone in Electronic Auscultation Apparatus of Second Embodiment

A relationship between the volume of the sealed space facing the inner surface 206b of the diaphragm 206 and the sensitivity of the condenser microphone 1101 will be described in detail with reference to Table 3 below.

Table 3

| Item | Volume [mm$^3$] | Volume change rate [%] |
|---|---|---|
| Volume change from 1 mm of displacement | 680 | - |
| Chest piece volume | 17,005 | 4 |
| Volume of sealed space 1100 | 2,085 | 33 |
| Volume of sealed space 1502 | 1,021 | 67 |

The upper limit value of the displacement in the operating range of the diaphragm 206 (i.e., the range in which vibration of the diaphragm 206 is assumed to occur) is assumed to be 1 mm. By displacing the diaphragm 206 from the flat state to the upper limit value of this displacement, the amount of the volume change in the internal space of the chest piece 1010 is, for example, 680 mm$^3$.

The volume of the internal space of the chest piece 1010 is, for example, 17,005 mm$^3$. Accordingly, the volume change rate of the internal space due to the change of the diaphragm 206 to the upper limit is 680/17,005 ≈ 4%. The volume of the sealed space 1100 illustrated in FIGS. 11A to 11C is, for example, 2,085 mm$^3$. Accordingly, the volume change rate of the sealed space 1100 due to the change of the diaphragm 206 to the upper limit is 680/2,085 ≈ 33%. The volume of the sealed space 1502 illustrated in FIGS. 15B and 15C is, for example, 1,021 mm$^3$. Accordingly, the volume change rate of the sealed space 1502 due to the change of the diaphragm 206 to the upper limit is 680/1,021 ≈ 67%. In this manner, by using only a part of the internal space of the chest piece 1010 as the sealed space facing the inner surface 206b of the diaphragm 206, the volume change rate of the sealed space increases, and the sensitivity of the condenser microphone 1101 increases as well. For example, the sealed space may be defined such that the volume change rate of the sealed space in response to the diaphragm 206 being displaced to the upper limit of the operating range is 30%.

Circuit Configuration of Electronic Auscultation Apparatus of Second Embodiment

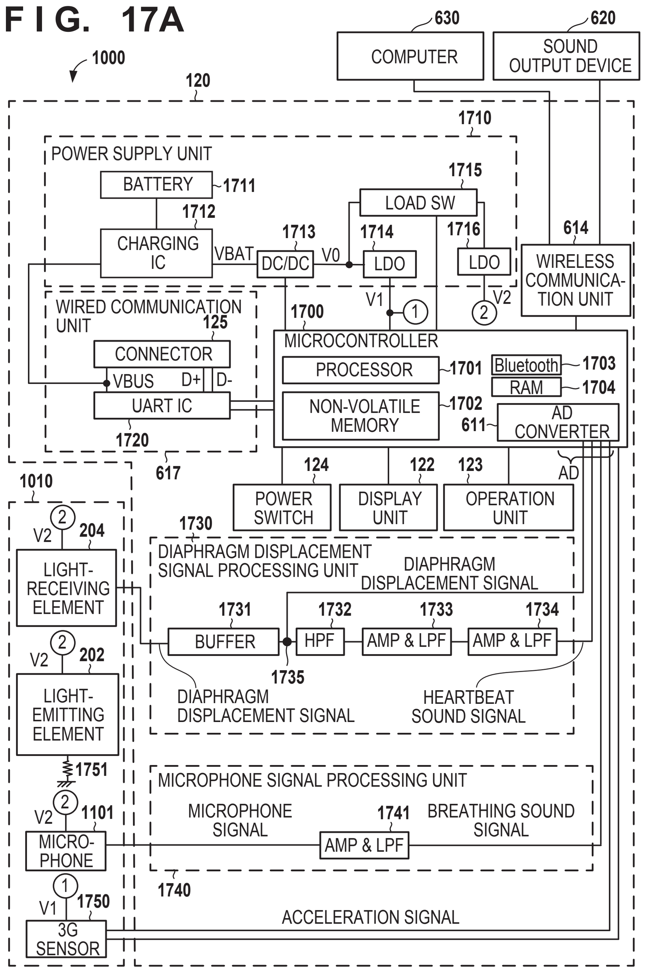

An example of the circuit configuration of the electronic stethoscope 1000 according to the second embodiment will be described next with reference to FIG. 17A. The circuit configuration illustrated in FIG. 17A is a diagram illustrating the hardware configuration in FIG. 10 in more detail.

As described above with reference to FIGS. 11A to 11C, the chest piece 1010 includes the light emitter 202, the photodetector 204, and the condenser microphone 1101. Furthermore, in the example in FIG. 17A, the chest piece 1010 includes a three-axis accelerometer 1750. The accelerometer 1750 is a sensor that measures acceleration in three dimensions. The accelerometer 1750 is a sensor for detecting movement (motion) of the electronic stethoscope 1000, and detects the electronic stethoscope 1000 being picked up by the user, for example. In other words, the accelerometer 1750 is used to determine whether the electronic stethoscope 1000 is being used. In the present embodiment, the accelerometer 1750 is included in the chest piece 1010, but may be included in the grip 120 instead of the chest piece 1010. In addition, if another configuration is employed to determine the usage state of the electronic stethoscope 1000, the accelerometer 1750 need not be included.

As described above with reference to FIG. 1A, the grip 120 includes the display device 122, the operation unit 123, the power switch 124, and the connector 125. Furthermore, the grip 120 includes a microcontroller 1700, a power supply unit 1710, a UART integrated circuit 1720, a diaphragm displacement signal processing unit 1730, and a microphone signal processing unit 1740. The plurality of circuit elements included in the grip 120 may be mounted on the same circuit board included in the grip 120, or may be distributed across a plurality of circuit boards.

The microcontroller 1700 is control means for controlling the overall operations of the electronic stethoscope 1000. In FIG. 17A, the electronic stethoscope 1000 includes one or more microcontrollers 1700. The microcontroller 1700 includes a processor 1701, a non-volatile memory 1702, a Bluetooth (registered trademark) circuit 1703, and a RAM 1704. The processor 1701 controls the operations of the electronic stethoscope 1000 by executing programs stored in the non-volatile memory 1702. The non-volatile memory 1702 is storage means for storing programs that define the operations of the electronic stethoscope 1000 and various types of configuration data, and keeps the stored content even when no power is supplied thereto from the exterior. The Bluetooth circuit 1703 is a control unit that controls the wireless communication unit 614 compliant with a Bluetooth wireless communication standard. The wireless communication unit 614 includes an antenna for performing wireless communication. Although the microcontroller 1700 has the Bluetooth circuit 1703 built in in FIG. 17A, the Bluetooth circuit 1703 may be outside the microcontroller 1700. The RAM 1704 is storage means for temporarily storing programs, various types of configuration data, and the like read out from the non-volatile memory 1702. The microcontroller 1700 is implemented by a plurality of circuit elements mounted on a circuit board included in the grip 120. The microcontroller 1700 transmits a sound signal based on the displacement signal generated by the photodetector 204 to an external audio output device via the wireless communication unit 614 or the wired communication unit 617. The audio output device 620 is a wireless or wired earphone or headphone, for example. The microcontroller 1700 can also transmit the sound signal to the audio output device 620, as well as to the computer 630 (e.g., a personal computer, a smartphone, a tablet, or the like). A doctor, a nurse, or a public health nurse can use the audio output device 620 or the computer 630 to listen to the body sound represented by the digitally-converted sound signal. The displacement signal output from the photodetector 204 is filtered and amplified by the diaphragm displacement signal processing unit 1730 (described later) and supplied to the A/D converter 611. The A/D converter 611 digitizes the output from the diaphragm displacement signal processing unit 1730. The displacement signal in digital format is then subjected to signal processing by the microcontroller 1700, such as data compression and encoding according to a format compliant with the communication standard, using an encoder, and converted into sound data for wireless communication. The conversion to sound data is, for example, conversion to Pulse Code Modulation (PCM) format. The wireless communication unit 614, which conforms to a wireless communication standard such as Bluetooth (registered trademark), then provides the sound data converted to PCM format to the audio output device 620. Having received the sound data, the audio output device 620 outputs a sound in accordance with the sound data. Although the foregoing describes an example in which the electronic stethoscope 1000 is capable of transmitting the sound data through both wireless communication and wired communication, the sound data may be transmitted by only one of these types of communication. The same applies to the transmission of the sound data to the computer 630 and the transmission of the sound data to the audio output device 620. The computer 630 can also visually display waveform data generated on the basis of the received sound data. The waveform data may be generated by the computer 630, or may be generated by the electronic stethoscope 1000. Some or all of the signal processing and the audio output processing by the electronic stethoscope 1000 may be performed by an external device (e.g., the audio output device 620 or the computer 630).

The UART integrated circuit 1720 is connected to the microcontroller 1700 and the connector 125 (specifically, the data terminal thereof). The UART integrated circuit 1720 performs UART-compliant communication. The UART integrated circuit 1720 and the connector 125 function as the wired communication unit 617. The microcontroller 1700 may be capable of wired communication with an external device through the UART integrated circuit 1720 and the connector 125. The UART integrated circuit 1720 may also be connected to the power terminal of the connector 125. A voltage VBUS may be applied to the UART integrated circuit 1720 through the power terminal of the connector 125 from an external device connected to the connector 125 (e.g., a charger or the computer 630). The UART integrated circuit 1720 may capable of operating using the voltage VBUS as an operating voltage.

The power supply unit 1710 includes a battery 1711, a charging integrated circuit 1712, a boost converter 1713, a voltage regulator 1714, a load switch 1715, and a voltage regulator 1716. The power supply unit 1710 supplies power to a plurality of circuit elements included in the electronic stethoscope 1000. The power supply unit 1710 may supply power at a plurality of different voltages. Instead, however, the power supply unit 1710 may supply power at a single voltage, and may step the voltage down in the stage before each circuit element such that the appropriate operating voltage is obtained.

The battery 1711 stores electrical energy used by the electronic stethoscope 1000. The battery 1711 may have a function for blocking current flowing to the battery 1711 when the current flowing to the battery 1711 exceeds a threshold. The charging integrated circuit 1712 is an integrated circuit (IC) that controls charging of and discharging from the battery 1711. For example, the charging integrated circuit 1712 charges the battery 1711 using electrical energy supplied from an external device such as a charger or the computer 630 connected to the connector 125. The charging integrated circuit 1712 also supplies electrical energy stored in the battery 1711 to the boost converter 1713. The voltage provided by the charging integrated circuit 1712 will be referred to as a voltage VBAT. The voltage VBAT is 3.7 V, for example.

The boost converter 1713 boosts a DC voltage to a DC voltage of another value. The boost converter 1713 is also called a DC/DC converter. The boost converter 1713 boosts the voltage VBAT supplied from the charging integrated circuit 1712 to a voltage V0. The voltage V0 is 6.8 V, for example. The voltage regulator 1714 generates and outputs a voltage of a specific value. The voltage regulator 1714 may be a linear regulator, and will also be referred to as a low-dropout regulator (LDO). The voltage regulator 1714 generates an operating voltage for some of the circuit elements of the electronic stethoscope 1000. The voltage generated by the voltage regulator 1714 will be referred to as a voltage V1. The voltage regulator 1714 may generate an operating voltage for the microcontroller 1700, and for example, the voltage V1 is 3.3 V. The operating voltage of the accelerometer 1750 is also the voltage V1. In the example in FIG. 17A, the voltage V1 is applied to both the microcontroller 1700 and the accelerometer 1750. Power is supplied to the microcontroller 1700 and the accelerometer 1750 from the voltage regulator 1714 of the power supply unit 1710. The voltage regulator 1714 outputs the voltage V1 when a voltage higher than the voltage V1 is applied to the input terminal thereof. Accordingly, the voltage regulator 1714 outputs the voltage V1 when the voltage V0 is supplied from the boost converter 1713.

The load switch 1715 is a switch that switches on (a conductive state) and off (a non-conductive state) in response to a control signal from the microcontroller 1700. The voltage regulator 1716 generates and outputs a voltage of a specific value. The voltage regulator 1716 may be a linear regulator or an LDO. The voltage regulator 1716 generates an operating voltage for some of the circuit elements of the electronic stethoscope 1000. The voltage generated by the voltage regulator 1716 will be referred to as a voltage V2. The voltage regulator 1716 may generate an operating voltage for the light emitter 202 and the photodetector 204, and for example, the voltage V2 is 5.8 V. In the example in FIG. 17A, the voltage V2 is applied to both the light emitter 202 and the photodetector 204. Power is supplied to the light emitter 202 and the photodetector 204 from the voltage regulator 1716 of the power supply unit 1710. The voltage regulator 1716 outputs the voltage V2 when a voltage higher than the voltage V2 is applied to the input terminal thereof. Accordingly, the voltage regulator 1716 outputs the voltage V2 when the load switch 1715 is on. The voltage regulator 1716 does not output the voltage V2 when the load switch 1715 is off. The potential at the output terminal of the voltage regulator 1716 when the voltage V2 is not output is a ground potential.

The diaphragm displacement signal processing unit 1730 generates a sound signal representing a sound transmitted from the surface of the target body to the diaphragm 206 by processing the diaphragm displacement signal, and outputs the sound signal to the microcontroller 1700. Specifically, the diaphragm displacement signal processing unit 1730 extracts a component of a specific frequency band included in the diaphragm displacement signal and generates a sound signal. As will be described later, the component of the specific frequency band to be extracted include a component of a frequency band in the range of 10 Hz to 1 kHz. The "diaphragm displacement signal" is a signal generated and output by the photodetector 204 in accordance with the amount of light that has reached the photodetector 204. The diaphragm displacement signal may simply be called a "displacement signal". The amount of light that reaches the photodetector 204 changes in accordance with the displacement of the diaphragm 206. If the light emitter 202 is a laser diode that emits a laser beam as described above, the diaphragm displacement signal may be a signal generated and output by the photodetector 204 in accordance with the position of the light that has reached the photodetector 204. Even when a laser diode is used, the diaphragm displacement signal still represents the displacement of the diaphragm 206. The sound signal generated by the diaphragm displacement signal processing unit 1730 on the basis of the diaphragm displacement signal is output when in the heartbeat mode, and will therefore be referred to as a "cardiac sound signal" in the following descriptions. The cardiac sound signal also represents displacement of the diaphragm 206 (specifically, the component of the specific frequency band), and is therefore a type of diaphragm displacement signal.

The diaphragm displacement signal processing unit 1730 includes a buffer circuit 1731, a high-pass filter (HPF) 1732, and amplifier circuits 1733 and 1734 including low-pass filters (LPFs), in the signal path between the photodetector 204 and the microcontroller 1700. These circuit elements are connected in series. The diaphragm displacement signal processing unit 1730 receives the diaphragm displacement signal from the photodetector 204 and outputs the cardiac sound signal to the microcontroller 1700.

The buffer circuit 1731 receives the diaphragm displacement signal from the photodetector 204 and outputs the diaphragm displacement signal to the HPF 1732. The buffer circuit 1731 performs impedance conversion for the signal path between the photodetector 204 and the HPF 1732. For example, the output impedance of the buffer circuit 1731 is lower than the output impedance of the photodetector 204. The operating power of the buffer circuit 1731 is supplied from the voltage regulator 1716.

The HPF 1732 outputs, to the amplifier circuit 1733, a signal obtained by attenuating a low-frequency component of the diaphragm displacement signal received from the buffer circuit 1731 (i.e., a frequency component lower than a specific cutoff frequency) and passing a high-frequency component of the diaphragm displacement signal (i.e., the frequency component higher than the cutoff frequency). The HPF 1732 in the present embodiment attenuates at least a component less than 10 Hz from the diaphragm displacement signal received from the buffer circuit 1731, and the cutoff frequency of the HPF 1732 is therefore 10 Hz, for example. However, the cutoff frequency may be a value greater than 10 Hz, e.g., 15 Hz or 20 Hz. Alternatively, the cutoff frequency may be at least 10 Hz and less than 20 Hz. The HPF 1732 therefore removes or attenuates at least a component less than 10 Hz from the diaphragm displacement signal received from the buffer circuit 1731.

The HPF 1732 is disposed in the signal path between the photodetector 204 and the microcontroller 1700, and removes or attenuates low-frequency noise present in the diaphragm displacement signal. The low-frequency noise present in the diaphragm displacement signal is a component that does not originate from the vibration transmitted from the surface of the target body to the diaphragm 206. For example, the low-frequency noise may include a component produced by shake in the hand of the user of the electronic stethoscope 1000. The low-frequency noise may also include a change in a DC component produced by the diaphragm 206 being pressed against the surface of the target body. Such low-frequency noise has an extremely large amplitude compared to the component originating from the vibration transmitted from the surface of the target body to the diaphragm 206 (called a "physiological component" hereinafter). Accordingly, amplifying a diaphragm displacement signal in which low-frequency noise is suppressed makes it possible to appropriately obtain the physiological component within the dynamic range of the amplifier circuit. Note that a band pass filter that removes at least the component less than 10 Hz may be used instead of the HPF 1732.

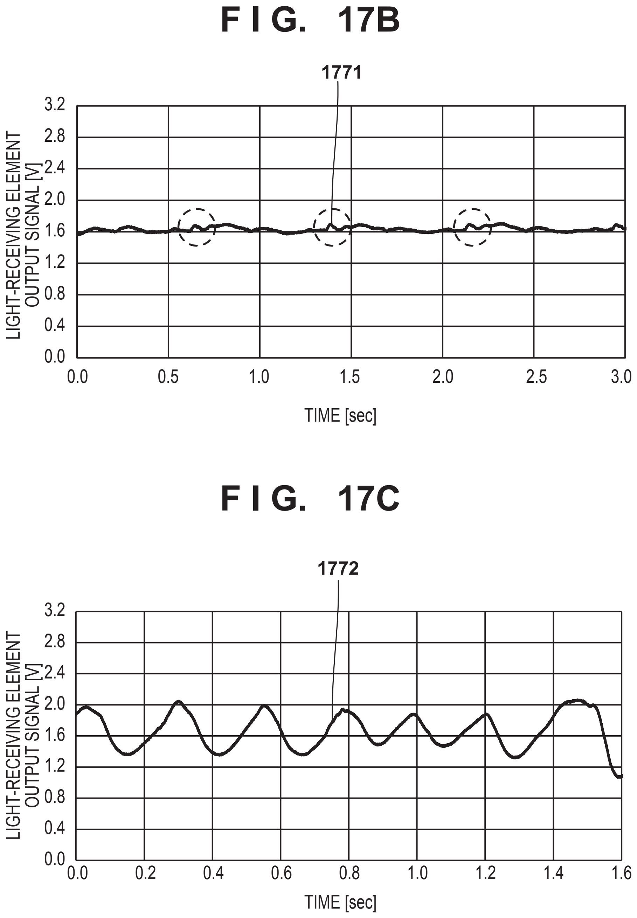

The effect of attenuating the component that is less than 10 Hz from the diaphragm displacement signal (the signal generated and output by the photodetector 204 in accordance with the amount of light that has reached the photodetector 204) will be described with reference to FIGS. 17B to 17H. A graph 1771 in FIG. 17C represents the temporal change in the diaphragm displacement signal when only a cardiac sound is generated, without any hand shake occurring. The diaphragm displacement signal indicated in the graph 1771 is a diaphragm displacement signal output from a node 1735 located in the signal path between the buffer circuit 1731 and the HPF 1732 and obtained and visualized by the microcontroller 1700. As illustrated in FIG. 17B, the amplitude of the biosignal of the cardiac sound is low, at several mV, and is extremely small relative to the dynamic range of the output voltage of the photodetector (about 5 V). The parts indicated by the dotted line circles in FIG. 17B are the I sound of the cardiac sound, but it is difficult to accurately extract the cardiac sound with the signal as-is, and it is necessary to amplify the signal using the amplifier circuit. A graph 1772 in FIG. 17B represents the temporal change in the diaphragm displacement signal when only hand shake is occurring, without the cardiac sound occurring. The diaphragm displacement signal in the graph 1772 is a diaphragm displacement signal output from a node 1735 located in the signal path between the buffer circuit 1731 and the HPF 1732 and obtained and visualized by the microcontroller 1700. For example, when a user holds the electronic stethoscope in their hand, as indicated in FIG. 17C, the amplitude of the signal of the hand shake component may in some cases be several hundred mV, which is much greater than the amplitude of the signal of the component of the cardiac sound.

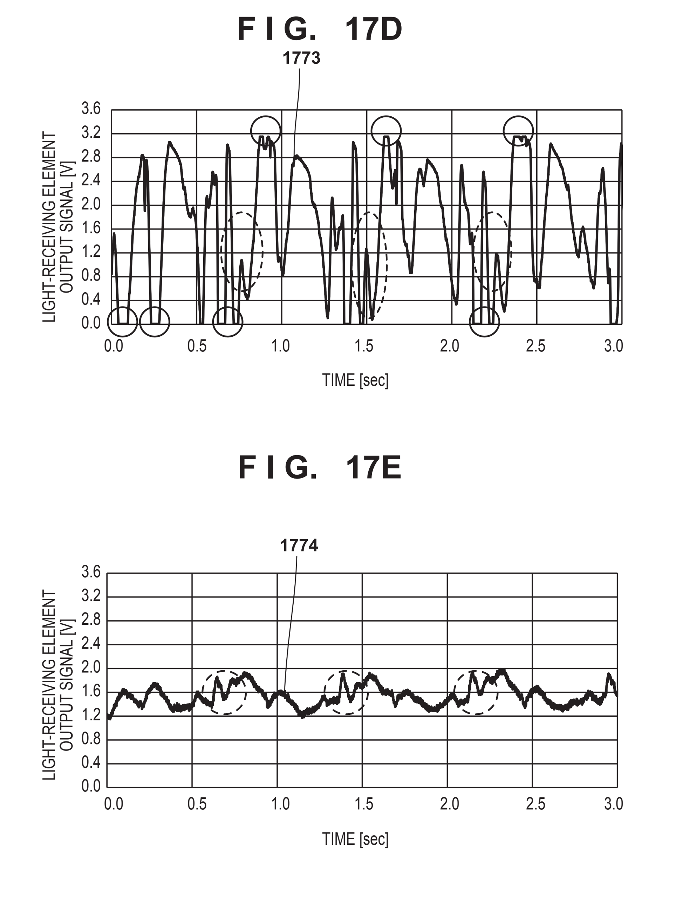

To measure target body vibrations having a low amplitude, it is necessary to amplify the diaphragm displacement signal. A graph 1773 in FIG. 17D represents a signal waveform in which the diaphragm displacement signal obtained when hand shake and the cardiac sound occur is amplified 100 times by the amplifier circuit. The graph 1773 in FIG. 17D indicates the output signal from the photodetector 204 having been amplified 100 times by the amplifier circuit 1733 without passing through the HPF 1732, and obtained and visualized by the microcontroller 1700. When the signal is amplified by using the amplifier circuit to apply a large gain without removing the signal of the hand shake component, there are parts in the graph 1773 in which the signal value partially exceeds the dynamic range and the peaks are cut, as indicated by the parts marked with solid line circles. In addition, as indicated by the dotted line circles in the graph 1773, in the signal waveform in the graph 1773, the cardiac sound is obscured by the signal of the hand shake component, and it is difficult to identify which component is the cardiac sound. On the other hand, a graph 1774 in FIG. 17E indicates an example in which the amplification rate of the amplifier circuit is set low, such that the dynamic range is not exceeded. The graph 1774 in FIG. 17E represents a waveform after the diaphragm displacement signal in which hand shake and the cardiac sound occur is amplified 25 times by the amplifier circuit. The graph 1773 in FIG. 17E indicates the signal output from the photodetector 204 having been amplified 25 times by the amplifier circuit 1733 without passing through the HPF 1732, and obtained and visualized by the microcontroller 1700. In this example, the signal value does not exceed the dynamic range, but the cardiac sound cannot be sufficiently amplified. In addition, the parts indicated by dotted line circles correspond to the I sound of the cardiac sound, but the cardiac sound is obscured by the hand shake component, and the cardiac sound cannot be accurately extracted. Accordingly, in the present embodiment, components less than 10 Hz are attenuated by the HPF 1732, after which the signal is amplified by the amplifier circuit.

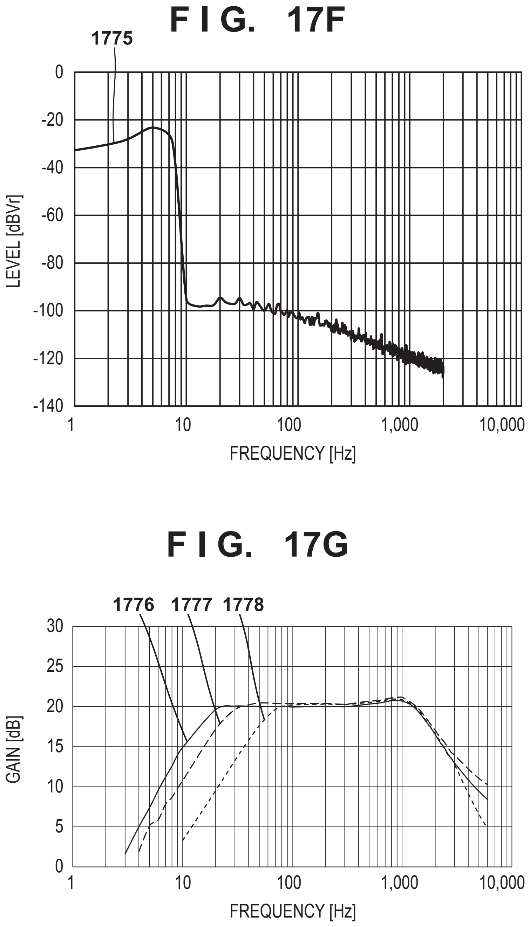

A graph 1775 in FIG. 17F represents a frequency spectrum of the diaphragm displacement signal in which hand shake and the cardiac sound occur. The graph 1775 in FIG. 17F indicates a signal level based on the frequency, after frequency analysis is performed on the diaphragm displacement signal in which the hand shake indicated in FIG. 17C occurs. As indicated by the graph 1775, the signal level of the hand shake component peaks at a frequency of 5 kHz, and decreases sharply with movement from 5 kHz to 10 kHz. The signal level then gradually converges after exceeding 10 KHz. The part of the diaphragm displacement signal output from the photodetector 204 at a frequency of less than 10 Hz can therefore be said to be caused by hand shake. Accordingly, the large amplitude caused by hand shake can be removed by using the HPF 1732 to attenuate the component less than 10 Hz. If the component under 10 Hz of the diaphragm displacement signal output from the photodetector 204 can be effectively attenuated by the HPF 1732, the dynamic range will not be exceeded even if the signal is then amplified by the amplifier circuit at a large gain.

Each graph in FIG. 17G represents the frequency characteristics of the HPF 1732 when the capacitance value of the capacitor is varied. A graph 1776 represents the frequency characteristics when the capacitance value of the capacitor of the HPF 1732 is 0.47 $\mu$F and the cutoff frequency is 10 Hz. A graph 1777 represents the frequency characteristics when the capacitance value of the capacitor of the HPF 1732 is 0.22 $\mu$F and the cutoff frequency is 20 Hz. A graph 1778 represents the frequency characteristics when the capacitance value of the capacitor of the HPF 1732 is 0.1 $\mu$F and the cutoff frequency is 30 Hz. As a result of experimentation, the component less than 10 Hz could be effectively attenuated when the cutoff frequency was set in the range of 10 to 20 Hz. On the other hand, as a result of experimentation, when the cutoff frequency was set to less than 10 Hz, the signal of the hand shake could not be effectively attenuated by the HPF, and hand shake noise was output as a result. On the other hand, when the cutoff frequency was set to greater than 20 Hz, e.g., when the cutoff frequency was set to 30 Hz as in the graph 1778, the cardiac sound component was greatly attenuated, and the cardiac sound could not be accurately extracted.

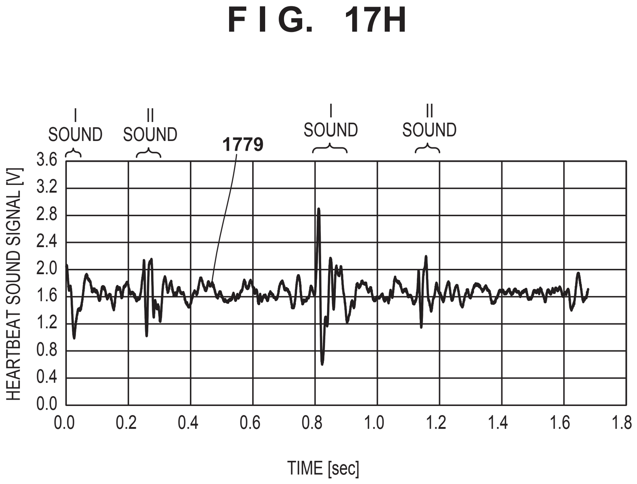

A graph 1779 in FIG. 17H represents the signal after the processing by the diaphragm displacement signal processing unit 1730, i.e., the waveform of the cardiac sound signal. Specifically, the graph 1779 in FIG. 17H indicates the signal output from the photodetector 204 having been processed by the HPF 1732, amplified 100 times by the amplifier circuits 1733 and 1734, and obtained and visualized by the microcontroller 1700. In the present embodiment, the hand shake component having a large amplitude is effectively attenuated by the HPF 1732. Accordingly, as indicated in FIG. 17H, a situation where the signal value amplified by the amplifier circuit exceeds the dynamic range and the peaks are cut can be suppressed. Additionally, in the present embodiment, of the diaphragm displacement signal, the cardiac sound component is attenuated as little as possible, while the hand shake component is attenuated, by the HPF 1732. Furthermore, in the present embodiment, after the hand shake component is attenuated by the HPF 1732, the cardiac sound component having a low amplitude is amplified 100 times by the amplifier circuits 1733 and 1734. Therefore, as indicated in FIG. 17H, the I and II sounds included in the cardiac sound can be accurately extracted.

In the example in FIG. 17A, the buffer circuit 1731 is disposed in the signal path between the photodetector 204 and the HPF 1732. Lowering the output impedance of the circuit elements in the stage previous to the HPF 1732 in this manner ensures that sufficient power is supplied to the capacitor of the HPF 1732, which improves the signal output characteristics of the HPF 1732. This improves the quality of the cardiac sound signal.

The amplifier circuit 1733 amplifies the signal received from the HPF 1732, attenuates the high-frequency component signal (i.e., the frequency component higher than a specific cutoff frequency), and passes the low-frequency component signal (i.e., the frequency component lower than the specific cutoff frequency). The amplifier circuit 1733 attenuates high-frequency noise included in the diaphragm displacement signal and outputs the diaphragm displacement signal to the amplifier circuit 1734. In the present embodiment, the cutoff frequency of the amplifier circuit 1733 is 1 kHz.

However, the cutoff frequency of the amplifier circuit 1733 may be less than 1 kHz, e.g., 950 Hz or 900 Hz. Alternatively, the cutoff frequency of the amplifier circuit 1733 may be at least 1 kHz and less than 2 kHz. Note that the operating power of the amplifier circuit 1733 is supplied from the voltage regulator 1716.

The amplifier circuit 1734 amplifies the signal received from the amplifier circuit 1734, and outputs a signal obtained by attenuating the high-frequency component signal and passing the low-frequency component to the microcontroller 1700. The cutoff frequency of the amplifier circuit 1734 may be the same as the cutoff frequency of the amplifier circuit 1733, or may be different. The operating power of the amplifier circuit 1734 is supplied from the voltage regulator 1716.

Both the amplifier circuits 1733 and 1734 may be inverting amplifier circuits. In this case, connecting the two amplifier circuits 1733 and 1734 in series ensures that the polarity of the diaphragm displacement signal and the polarity of the cardiac sound signal match. Instead, however, the diaphragm displacement signal processing unit 1730 may include only one amplifier circuit having a LPF, and that amplifier circuit may be a non-inverting amplifier circuit. The polarity of the diaphragm displacement signal and the polarity of the cardiac sound signal may also differ from each other.

Although the present embodiment describes a configuration in which the amplifier circuits 1733 and 1734 include LPFs as an example, the configuration may be such that the amplifier circuits 1733 and 1734 do not include LPFs. In this case, a separate LPF may be disposed in the signal path between the HPF 1732 and the microcontroller 1700. A band pass filter may also be disposed in this signal path instead of a LPF.

The diaphragm displacement signal processing unit 1730 has an amplifier circuit in the signal path between the HPF 1732 and the microcontroller 1700, as well as in the signal path between the photodetector 204 and the microcontroller 1700, and the amplifier circuits amplify the cardiac sound signal. Although a configuration in which the diaphragm displacement signal processing unit 1730 includes amplifier circuits is described as an example in the present embodiment, the configuration may be such that the microcontroller 1700 includes an amplifier circuit.

As illustrated in FIG. 17A, the diaphragm displacement signal processing unit 1730 further outputs the diaphragm displacement signal before being processed by the HPF 1732 to the microcontroller 1700. In the example in FIG. 17A, the node 1735 located in the signal path between the buffer circuit 1731 and the HPF 1732 is connected to the microcontroller 1700. The diaphragm displacement signal is output from this node 1735 to the microcontroller 1700. As will be described later, the microcontroller 1700 detects a pressing state (also referred to as a "contact state") of the diaphragm 206 on the basis of this diaphragm displacement signal, and performs volume setting operations (described later). The diaphragm displacement signal to the microcontroller 1700 may be output from another node in the signal path between the photodetector 204 and the HPF 1732. For example, the diaphragm displacement signal to the microcontroller 1700 may be output from a node in the signal path between the photodetector 204 and the buffer circuit 1731.

The microphone signal processing unit 1740 generates a sound signal representing a sound transmitted from the surface of the target body to the diaphragm 206 by processing the microphone signal, and outputs the sound signal to the microcontroller 1700. Specifically, the microphone signal processing unit 1740 generates the sound signal by extracting a component of a specific frequency band included in the microphone signal. As described above, the microphone signal is a signal generated by the condenser microphone 1101 on the basis of air vibrations produced in the internal space 213 facing the inner surface 206b of the diaphragm 206 (see FIG. 2A and the like; this may be the sealed space). The microphone signal is also called a vibration signal. The sound signal generated by the microphone signal processing unit 1740 on the basis of the microphone signal is output in the respiratory sound mode, and will therefore be referred to as a "respiratory sound signal" in the following descriptions. The microphone signal also represents a sound transmitted from the surface of the target body to the diaphragm 206, and the microphone signal is therefore a type of respiratory sound signal.

The microphone signal processing unit 1740 includes an amplifier circuit 1741 having a LPF in the signal path between the condenser microphone 1101 and the microcontroller 1700. The microphone signal processing unit 1740 receives the microphone signal from the condenser microphone 1101 and outputs the respiratory sound signal to the microcontroller 1700.

The amplifier circuit 1741 outputs a signal obtained by amplifying the signal received from the condenser microphone 1101 and attenuating the high-frequency component to the microcontroller 1700. Through this, the amplifier circuit 1741 appropriately removes high-frequency noise present in the microphone signal. The cutoff frequency of the amplifier circuit 1741 may be 1 kHz or lower, e.g., 1 kHz, 950 Hz, or 900 Hz. The operating power of the amplifier circuit 1741 may be supplied from the voltage regulator 1716.

The low-frequency noise present in the microphone signal is smaller than the low-frequency noise present in the diaphragm displacement signal. Accordingly, in the present embodiment, the microphone signal processing unit 1740 does not include an HPF in the signal path between the condenser microphone 1101 and the microcontroller 1700. In this case, the signal path between the condenser microphone 1101 and the microcontroller 1700 passes the component lower than the cutoff frequency of the amplifier circuit 1741. However, an HPF may be disposed in the signal path between the condenser microphone 1101 and the amplifier circuit 1741. In this case, the cutoff frequency of the HPF disposed in the signal path between the condenser microphone 1101 and the amplifier circuit 1741 is set to be lower than the cutoff frequency of the HPF 1732 (e.g., 5 Hz, 3 Hz, or the like). Additionally, the amplifier circuit 1741 may be omitted, and the microphone signal may be output directly from the condenser microphone 1101 to the microcontroller 1700 as the respiratory sound signal. The microcontroller 1700 may amplify this respiratory sound signal.

In the example in FIG. 17A, other circuit elements may be disposed in the signal path between the buffer circuit 1731 and the photodetector 204.

The diaphragm displacement signal, the cardiac sound signal, the respiratory sound signal, and the acceleration signal are supplied to respective input terminals of the microcontroller 1700. The microcontroller 1700 includes the A/D converter 611, which converts these analog signals into digital signals, and the processing by the microcontroller 1700 is performed using the digital signals.

Function Block Configuration of Electronic Auscultation Apparatus of Second Embodiment

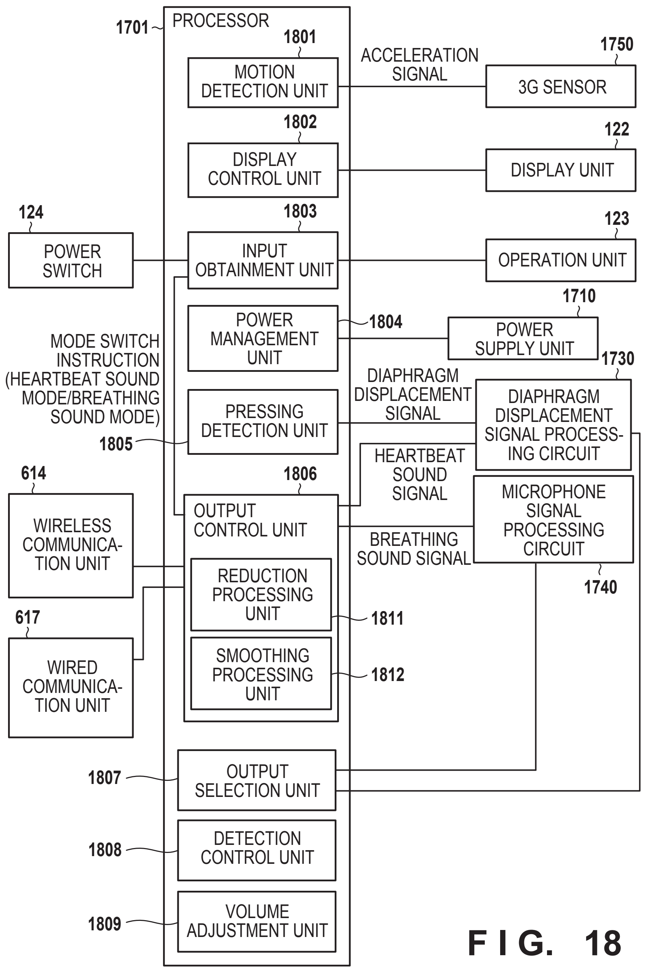

Function blocks implemented by the processor 1701 of the microcontroller 1700 will be described with reference to FIG. 18. Each function block in FIG. 18 is realized by loading programs stored in the non-volatile memory 1702 into the RAM 1704 and executing the programs using the processor 1701. However, some or all of the functional blocks in FIG. 18 may be implemented by dedicated integrated circuits such as application-specific integrated circuits (ASICs).

A motion detection unit 1801 detects motion of the electronic stethoscope 1000 on the basis of the acceleration signal obtained from the accelerometer 1750. For example, the motion detection unit 1801 determines that the electronic stethoscope 1000 is moving when acceleration in at least one of three axial directions, namely the x-axis, the y-axis, and the z-axis, is non-zero or exceeds a threshold. Conversely, the motion detection unit 1801 determines that the electronic stethoscope 1000 is at rest when acceleration in all axial directions is zero or less than the threshold.

A display control unit 1802 controls displays on the display device 122. An input obtainment unit 1803 obtains user inputs using the operation unit 123 and the power switch 124. A power management unit 1804 controls operations for generating a specific voltage, for example, as operations by the power supply unit 1710. Specifically, the power management unit 1804 switches the level of the control signal supplied to the load switch 1715, and switches the load switch 1715 on and off. As described above, when the load switch 1715 is off, the voltage V0 is no longer supplied to the voltage regulator 1716, and thus the power supply from the voltage regulator 1716 is stopped, and the mode transitions to a low-power mode 2601 (described later).

A pressing detection unit 1805 detects that the diaphragm 206 has pressed or made contact on the basis of the diaphragm displacement signal obtained from the diaphragm displacement signal processing unit 1730. Hereinafter, the pressing state of the diaphragm 206 will be referred to simply as a "pressing state". For example, the pressing detection unit 1805 can specify which of a plurality of states the pressing state is. Specifically, the pressing detection unit 1805 specifies whether the pressing state is an in-use state (a first state) or an unused state (a second state). The unused state (the second state) is a pressing state in which the user does not have the diaphragm 206 in close contact with the surface of a target body. The in-use state (the first state) is a pressing state in which the user has the diaphragm 206 in close contact with the surface of a target body. The displacement amount of the diaphragm 206 in the unused state is less than the displacement amount of the diaphragm 206 in the in-use state. Accordingly, the pressing detection unit 1805 determines that the pressing state is the unused state when the displacement amount of the diaphragm 206 specified from the diaphragm displacement signal is less than a threshold. On the other hand, the pressing detection unit 1805 determines that the pressing state is the in-use state when the displacement amount of the diaphragm 206 exceeds the threshold.

Furthermore, the in-use state is subdivided into two states, namely a correct state and an overpressure state. In this case, the pressing detection unit 1805 specifies which of three states the pressing state is, namely the correct state (the first state), the unused state (the second state), or the overpressure state (a third state). The overpressure state (the third state) is a pressing state in which the compressive force at which the diaphragm 206 is pressed against the surface of the target body is too strong, and sound is not properly transmitted from the surface of the target body to the diaphragm 206. The correct state is a pressing state in which sound is properly transmitted from the surface of the target body to the diaphragm 206. The displacement amount of the diaphragm 206 in the overpressure state is greater than the displacement amount of the diaphragm 206 in the correct state. The pressing detection unit 1805 determines that the pressing state is the unused state when the displacement amount of the diaphragm 206 is less than a threshold. On the other hand, the pressing detection unit 1805 determines that the pressing state is the correct state when the displacement amount of the diaphragm 206 exceeds the threshold but is less than another threshold greater than the stated threshold. The pressing detection unit 1805 determines that the pressing state is the overpressure state when the displacement amount of the diaphragm 206 exceeds this other threshold.

An output control unit 1806 transmits the cardiac sound signal obtained from the diaphragm displacement signal processing unit 1730 and the respiratory sound signal obtained from the microphone signal processing unit 1740 to an external device such as the computer 630 or the audio output device 620 through the wireless communication unit 614 or the wired communication unit 617. An output selection unit 1807 selects a sound signal (the cardiac sound signal, the respiratory sound signal, or both) output by the output control unit 1806. The output selection unit 1807 selects the sound signal to be output on the basis of a user input obtained by the input obtainment unit 1803.

The output control unit 1806 executes signal processing on the sound signal before outputting the sound signal. Specifically, the output control unit 1806 includes an amplitude limiting unit 1811 and a smoothing unit 1812. The amplitude limiting unit 1811 executes amplitude limiting for reducing amplitudes exceeding a threshold amplitude by comparing the amplitude of the sound signal with the threshold amplitude. The smoothing unit 1812 performs smoothing for removing components at at least the cutoff frequency (i.e., high-frequency components) from the sound signal after the amplitude limiting has been performed. The amplitude limiting and the smoothing will be described in detail later.

A detection control unit 1808 controls the operations of the displacement detection unit 1011, which includes the light emitter 202 and the photodetector 204, and the vibration detection unit 1012, which includes the condenser microphone 1101. For example, when the displacement detection unit 1011 and the vibration detection unit 1012 have adjustable parameters, the detection control unit 1808 adjusts those parameters.

A volume adjustment unit 1809 adjusts the volume of the sound signal (the cardiac sound signal or the respiratory sound signal) output to the exterior. The volume of the sound signal output to the exterior may be simply referred to as the "volume" hereinafter. For example, the volume adjustment unit 1809 adjusts the volume on the basis of a user input obtained by the input obtainment unit 1803. For example, the volume adjustment unit 1809 increases the volume when the user operates the volume up button 123a included in the operation unit 123 in FIG. 1A to make an instruction to increase the volume. The volume adjustment unit 1809 reduces the volume when the user operates the volume down button 123b included in the operation unit 123 in FIG. 1A to make an instruction to reduce the volume.

The volume adjustment unit 1809 also adjusts the volume on the basis of the pressing state. For example, the volume adjustment unit 1809 can also set the volume to a normal level when the diaphragm 206 is being pressed by the subject by at least a certain amount (i.e., when the pressing state is determined to be the in-use state). Operations for setting the volume on the basis of the pressing state will be described later. The volume of a "normal level" is a volume suitable for listening to the sound signal reproduced by the audio output device 620. The volume adjustment unit 1809 adjusts the value of the normal level on the basis of a user input obtained by the input obtainment unit 1803.

The volume adjustment unit 1809 sets the volume to a mute level when the diaphragm 206 is not being pressed by the subject (when the pressing state is determined to be the unused state). The volume at the "mute level" means that the volume is zero or less than the volume at the normal level. For example, the volume of the mute level may be such that the volume is too low to be suitable for listening to the sound signal reproduced by the audio output device 620. The mute level can also be set to a constant multiple of the normal level (e.g., 10%). In this manner, when the mute level is configured to depend on the normal level, e.g., when the normal level changes in response to a user input made through the volume adjustment buttons included in the operation unit 123, the mute level also changes depending on the normal level. On the other hand, the mute level can also be set independently of the normal level. When the mute level is independent from the normal level, the mute level does not change even if the normal level changes in response to a user input, for example.

The volume adjustment unit 1809 sets the volume to a normal level when the diaphragm 206 is being pressed by the subject by at least a certain amount (i.e., when the pressing state is determined to be the in-use state). The normal level is set in accordance with a user input made through the volume adjustment buttons included in the operation unit 123. On the other hand, the volume adjustment unit 1809 sets the volume to the mute level when the diaphragm 206 is being pressed by the subject by more than the necessary compressive force (i.e., when the pressing state is determined to be the overpressure state). However, the volume need not necessarily be set to the mute level when the pressing state is the overpressure state, and the volume adjustment unit 1809 may set the volume to the normal level in the same manner as when the pressing state is the correct state.

The volume adjustment unit 1809 adjusts the gain of the at least one of the amplifier circuit 1733 and the amplifier circuit 1734 to adjust the volume level of the cardiac sound signal. Alternatively, the volume adjustment unit 1809 may adjust the digital value of the sound signal output to the exterior by the output control unit 1806 to adjust the volume level of the cardiac sound signal. The volume adjustment unit 1809 adjusts the volume level of the respiratory sound signal by adjusting the gain of the amplifier circuit 1741. Alternatively, the volume adjustment unit 1809 may adjust the digital value of the sound signal output to the exterior by the output control unit 1806 to adjust the volume level of the respiratory sound signal.

Setting Operation 1 for Volume of Electronic Auscultation Apparatus of Second Embodiment

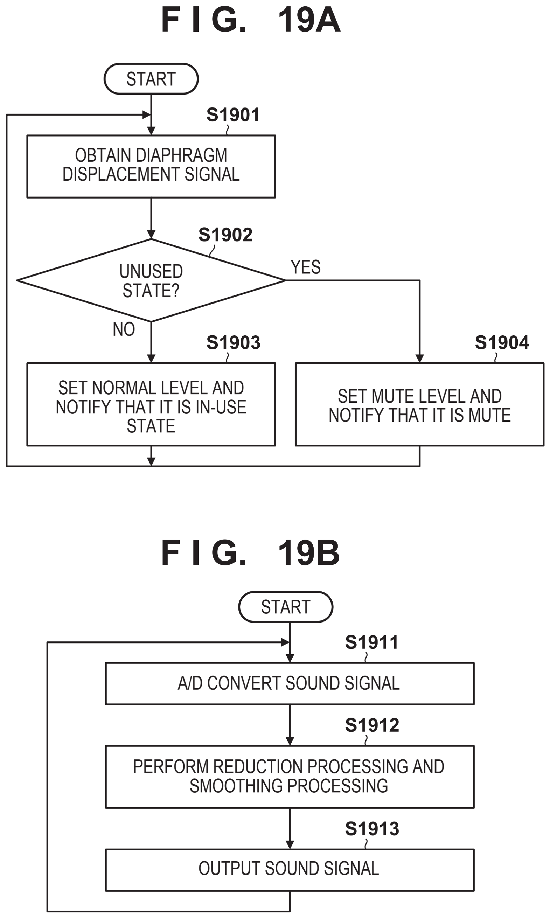

A volume setting operation performed by the electronic stethoscope 1000 will be described with reference to FIG. 19A. FIG. 19A is a flowchart illustrating processing for specifying whether the pressing state is the unused state or the in-use state. Each step of the method in FIG. 19A is realized by the processor 1701 executing a program stored in the non-volatile memory 1702. However, some or all of the steps of the method in FIG. 19A may be implemented by a dedicated integrated circuit. The processor 1701 starts the method in FIG. 19A in response to the power of the electronic stethoscope 1000 being turned on, and ends the method in FIG. 19A in response to the power of the electronic stethoscope 1000 being turned off. In the present embodiment, when the power of the electronic stethoscope 1000 is turned off, the processor 1701 stores a volume setting value at the normal level in the non-volatile memory 1702. When the power of the electronic stethoscope 10000 is turned on, the processor 1701 reads out the normal level volume setting value stored in the non-volatile memory 1702 and sets that value as an initial value of the normal level. In the present embodiment, the volume is set to the mute level when the flowchart in FIG. 19A begins, but the volume may instead be set to the initial value of the normal level.

In S1901, the processor 1701 (e.g., the pressing detection unit 1805) obtains the diaphragm displacement signal from the diaphragm displacement signal processing unit 1730. Because the diaphragm displacement signal is output from the node 1735 of the diaphragm displacement signal processing unit 1730 (see FIG. 17A) while the power of the electronic stethoscope 1000 is turned on, the processor 1701 obtains the diaphragm displacement signal.

In S1902, the processor 1701 (e.g., the pressing detection unit 1805) determines whether the pressing state is the unused state. The processor 1701 moves the sequence to S1904 if the pressing state is determined to be the unused state ("YES" in S1902), and to S1903 if not ("NO" in S1902). In the method in FIG. 19A, determining that the pressing state is not the unused state means that the pressing state is determined to be the in-use state.

In S1903, the processor 1701 (the volume adjustment unit 1809) sets the volume to the normal level. Specifically, if the current volume is at the normal level, the processor 1701 maintains the current volume, whereas if the current volume is at the mute level, the processor 1701 switches the volume to the normal level. The normal level volume setting value is stored in the non-volatile memory 1702 when the power of the electronic stethoscope 1000 is turned off, and that setting value is read out as the initial value of the normal level when the power is turned on. Accordingly, if a volume adjustment button included in the operation unit 123 has not been operated by the user, the initial value is set to the normal level volume. On the other hand, if the volume adjustment button has been operated by the user, the normal level volume is updated, and the updated volume is stored in the non-volatile memory 1702 as the normal level volume. In S1903, the processor 1701 also notifies the user that the pressing state is the in-use state and the volume is the normal level. In the present embodiment, the output control unit 1806 makes this notification as audio through the wireless communication unit 614 or the wired communication unit 617, and the display control unit 1802 makes this notification by lighting the LED included in the display device 122. However, the notification method is not limited thereto, and may be either audio or lighting the LED; or the notification may be made by causing the electronic stethoscope 1000 to vibrate, for example.

In S1904, the processor 1701 (e.g., the volume adjustment unit 1809) sets the volume to the mute level. In other words, if the current volume is at the mute level, the processor 1701 maintains the current volume, whereas if the current volume is at the normal level, the processor 1701 switches the volume to the mute level. In addition, the processor 1701 notifies the user that the pressing state is the unused state and the volume is the mute level. For example, the output control unit 1806 may make this notification through the wireless communication unit 614 or the wired communication unit 617, or the display control unit 1802 may make this notification by lighting the LED included in the display device 122.

The processor 1701 repeats S1901 to S1904. In the present embodiment, the processor 1701 repeats S1901 to S1904 at a predetermined cycle (e.g., 1 ms to 100 ms). According to the method in FIG. 19A, if the pressing state is the in-use state, the volume level is set to the normal level, whereas if the pressing state is the unused state, the volume level is set to the mute level. According to the foregoing embodiment, the electronic stethoscope automatically sets the volume in accordance with the pressing state of the diaphragm. Specifically, whether a target body has contacted the diaphragm (pressed the diaphragm by at least a predetermined amount) is determined according to whether the displacement amount of the diaphragm specified from the diaphragm displacement signal is at least a threshold or less than the threshold, and the volume is automatically adjusted according to the result of the determination. This makes it possible for the user to bring the electronic stethoscope into contact with the target body and output audio only when auscultation is to be performed. This also makes it possible to suppress rubbing sounds, contact sounds, and the like when making contact with the target body, which enables noiseless auscultation.

Sound Signal Output Operations by Electronic Auscultation Apparatus of Second Embodiment

A sound signal output operation performed by the electronic stethoscope 1000 will be described with reference to FIG. 19B. Each step of the method of FIG. 19B is executed by the processor 1701, for example. However, some or all of the steps of the method in FIG. 19B may be implemented by a dedicated integrated circuit. The processor 1701 starts the method illustrated in FIG. 19B in response to the power of the electronic stethoscope 1000 being turned on, or in response to returning from the low-power mode 2601. The processor 1701 ends the method illustrated in FIG. 19B in response to the power of the electronic stethoscope 1000 being turned off, or in response to transitioning to the low-power mode 2601.

In S1911, the processor 1701 (e.g., the output control unit 1806) A/D converts the cardiac sound signal or the respiratory sound signal supplied to the input terminal. Whether to A/D convert the cardiac sound signal or the respiratory sound signal is determined in accordance with the operating mode of the electronic stethoscope 1000. Specifically, when the electronic stethoscope 1000 is in the cardiac sound mode, which is an example of an auscultation mode, the processor 1701 A/D converts the cardiac sound signal. When the electronic stethoscope 1000 is in the respiratory sound mode, which is an example of an auscultation mode, the processor 1701 A/D converts the respiratory sound signal. The A/D-converted sound signal is subject to output from the electronic stethoscope 1000. In the following descriptions of FIG. 19B, the sound signal to be output will be referred to simply as a "sound signal". In S1911, the processor 1701 may A/D convert both the cardiac sound signal and the respiratory sound signal, and may execute the subsequent processing on only the sound signal to be output.

The processor 1701 repeats the subsequent S1911 to S1913 at a predetermined sampling rate (e.g., 2 ms). The numerical value corresponding to the time at which S1911 is started in each of these repeated attempts will be referred to as a "sampling time t". The A/D-converted sound signal (i.e., the sound signal in digital format) is represented by X. The signal value of the sound signal X at the sampling time t is represented by X(t). The processor 1701 quantizes the voltage supplied to the input terminal (i.e., the sound signal in analog format) to a signal value of 12 bits, for example. The processor 1701 stores the sound signal X in the RAM 1704 for use in the subsequent processing.

In S1912, the processor 1701 (e.g., the output control unit 1806) performs signal processing on the sound signal X. Specifically, first, the processor 1701 (e.g., the amplitude limiting unit 1811) generates a sound signal Y by performing the amplitude limiting on the sound signal X. The sound signal Y is a signal in which amplitudes of the sound signal X exceeding an amplitude threshold are reduced. The signal value of the sound signal Y at the sampling time t is represented by Y(t). The processor 1701 stores the sound signal Y in the RAM 1704 for use in the subsequent processing.

The processor 1701 performs the amplitude limiting according to the following Formula 9.

Formula 9

$$Y(t) = \begin{cases} B + C + k(|X(t) - B| - C) & (if \ X(t) > B + C) \\ X(t) & (if \ B - C \leq X(t) \leq B + C) \\ B - C - k(|X(t) - B| - C) & (if \ X(t) < B - C) \end{cases}$$

In Formula 9, a baseline value B is a value representing a baseline of the sound signal in analog format. The amplitude of the sound signal X is given by the difference between the signal value X(t) of the sound signal X and the baseline value B, i.e., |X(t) - B|. The baseline value B is set in advance and stored in the non-volatile memory 1702. When the sound signal is quantized at 12 bits, B = 2047, for example.

In Formula 9, an amplitude threshold C represents the amplitude threshold used in the amplitude limiting. The processor 1701 compares the amplitude of the sound signal X with the amplitude threshold C, and determines Y(t) on the basis of the result of the comparison. When the amplitude of the sound signal X is not greater than the amplitude threshold C, the processor 1701 sets the signal value X(t) of the sound signal X to the signal value Y(t) of the sound signal Y as-is. The amplitude threshold C is set in advance and stored in the non-volatile memory 1702. When the sound signal is quantized at 12 bits, C = 1024, for example.

When the amplitude of the sound signal X is greater than the amplitude threshold C, the processor 1701 sets the amplitude of the sound signal Y to a value obtained by reducing the amplitude of the sound signal X. Specifically, the processor 1701 sets the sum of (i) the difference between the amplitude of the sound signal X and the amplitude threshold C, i.e., a value obtained by multiplying |X(t) - B| - C by a reduction magnification factor k, and (ii) the amplitude threshold C as the amplitude of the sound signal Y. The reduction magnification factor k is a real number of at least 0 and less than 1, and is set in advance and stored in the non-volatile memory 1702. The closer the reduction magnification factor k is to 0, the more the amplitude of the sound signal X is reduced. When k = 0, the amplitude of the sound signal X matches the amplitude threshold C. In other words, the sound signal X is clipped at the amplitude threshold C.

In general, the sound signal X can include a noise component having a larger amplitude than the body sound. By performing the amplitude limiting described above on the sound signal X, the amplitude of the noise component having a large amplitude can be reduced while maintaining the amplitude of the body sound.

Next, the processor 1701 (e.g., the smoothing unit 1812) generates a sound signal Z by performing smoothing on the sound signal Y after the amplitude limiting has been performed. The sound signal Z is a signal smoothed by removing high-frequency components at at least the cutoff frequency from the sound signal Y. The signal value of the sound signal Z at the sampling time t is represented by Z(t). The processor 1701 stores the sound signal Z in the RAM 1704 for use in the subsequent processing.

The processor 1701 performs the smoothing according to the following Formula 10.

Formula 10

$$Z(t) = \sum_{i=0}^{N-1} Y(t - i \times r)$$

In Formula 10, a sampling rate r is a sampling rate of the A/D conversion. A term number N is the number of signal values of the sound signal Y used in the moving average. The term number N is set in advance and stored in the non-volatile memory 1702. N = 32, for example. Taking the moving average in this way removes high-frequency components at at least the cutoff frequency determined by the sampling rate and the term number N from the sound signal Y. As a result, the amount of change in the slope of the sound signal Z before and after the amplitude threshold C is reduced compared to the sound signal Y, which reduces noise uncomfortable to the user.

In S1913, the processor 1701 (e.g., the output control unit 1806) outputs the sound signal Z. The sound signal Z is output in accordance with the volume determined in the operations illustrated in FIG. 19A.

In the processing described above, the processor 1701 generates the sound signal Z by performing both the amplitude limiting and the smoothing on the sound signal X, and outputs the sound signal Z. Instead, however, the processor 1701 may perform the amplitude limiting on the sound signal X, without performing the smoothing. In this case, the processor 1701 may output the sound signal Y. If the value of the reduction magnification factor k is high, the amount of change in the slope of the sound signal Y before and after the amplitude threshold C will be small, and thus even if the sound signal Y is output, there will be little noise that is uncomfortable to the user.

In the example described above, the processor 1701 continually performs amplitude limiting and smoothing while outputting the sound signal. In other words, the processor 1701 performs the amplitude limiting and the smoothing not only while the pressing state is determined to be the in-use state, but also in other periods as well. Instead, however, the processor 1701 may perform the amplitude limiting and the smoothing only some of the time during which the sound signal is being output. When the amplitude limiting and the smoothing are not performed, the processor 1701 outputs the sound signal X for which the signal processing has not been performed. In the electronic stethoscope 1000, noise having a large amplitude is likely to occur when the pressing state changes from the unused state to the in-use state, when the pressing state changes from the in-use state to the unused state, and the like. In particular, immediately after the pressing state is determined to have changed from the in-use state to the unused state, the volume is in the process of transitioning from the normal level to the mute level, and noise having a high volume may therefore reach the user. Accordingly, after determining that the pressing state has changed from the in-use state to the unused state, the processor 1701 may perform the amplitude limiting and the smoothing until a predetermined condition is met, and may skip the amplitude limiting and the smoothing in other periods. This makes it possible to suppress situations where loud noise reaches the user.

The predetermined condition for ending the amplitude limiting and the smoothing may be that the volume has transitioned to the mute level, that a predetermined length of time has passed after the pressing state is determined to have changed from the in-use state to the unused state, that the pressing state is determined to have changed again from the unused state to the in-use state, or a combination thereof.

The processor 1701 may perform the smoothing continually while the sound signal is being output, and perform the amplitude limiting only during some period while the sound signal is being output. For example, after determining that the pressing state has changed from the in-use state to the unused state, the processor 1701 may perform the amplitude limiting until a predetermined condition is met, and may skip the amplitude limiting in other periods. This predetermined condition is the same as described above.

The processor 1701 may switch whether to perform the amplitude limiting and the smoothing on a per-mode basis. For example, the processor 1701 performs both the amplitude limiting and the smoothing in the cardiac sound mode. The period during which the amplitude limiting and the smoothing are performed in the cardiac sound mode may be the entire period in which the sound signal is being output, or only part of that period, as described above. In the respiratory sound mode, the processor 1701 performs the smoothing but does not perform the amplitude limiting. The period during which the smoothing is performed in the respiratory sound mode may be the entire period in which the sound signal is being output, or only part of that period, as described above.

The processor 1701 may switch the cutoff frequency of the smoothing on a per-mode basis. For example, the processor 1701 sets the term number N to 32 in the cardiac sound mode, and sets the term number N to 16 in the respiratory sound mode. As a result, the cutoff frequency of the smoothing in the respiratory sound mode is higher than the cutoff frequency of the smoothing in the cardiac sound mode. The frequency of the body sound to be observed is higher in the respiratory sound mode than in the cardiac sound mode. Accordingly, increasing the cutoff frequency of the smoothing in the respiratory sound mode makes it possible to reduce the risk of even the body sound being filtered.

In the operations described above, a fixed value is used as the amplitude threshold C. Instead, however, the processor 1701 may determine the amplitude threshold C while the sound signal is being output, and execute the amplitude limiting using the determined amplitude threshold C. For example, the processor 1701 may determine the amplitude threshold C on the basis of the amplitude of the sound signal X for which the amplitude limiting is not performed while the pressing state is determined to be the in-use state. For example, the processor 1701 may determine the amplitude threshold C on the basis of the maximum value of the amplitude of the sound signal X for which the amplitude limiting is not performed while the pressing state is determined to be the in-use state. Specifically, when the maximum value of this amplitude is greater than the default value of the amplitude threshold C, the processor 1701 may use that maximum value as the amplitude threshold C. When a value obtained by addition or multiplication of the predetermined value with the maximum value of this amplitude is greater than the default value, the processor 1701 may use this value as the amplitude threshold C. The processor 1701 may use another representative value, e.g., the average value of the peak amplitude, instead of the maximum value of the amplitude of the sound signal X. The likelihood that noise having a large amplitude will occur in the sound signal X while the pressing state is determined to be the in-use state is low. Accordingly, determining the amplitude threshold C on the basis of the amplitude of the sound signal X in this period makes it possible to reduce the amplitude of only the noise.

After determining that the pressing state has changed from the in-use state to the unused state, the processor 1701 may reset the amplitude threshold C determined as described above to the default value on the basis of a predetermined condition being satisfied. The predetermined condition for resetting the amplitude threshold C may be that the volume has transitioned to the mute level, that a predetermined length of time has passed after the pressing state is determined to have changed from the in-use state to the unused state, that the pressing state is determined to have changed again from the unused state to the in-use state, or a combination thereof.

Specific Example 1 of Change in Volume and Amplitude in Electronic Auscultation Apparatus of Second Embodiment

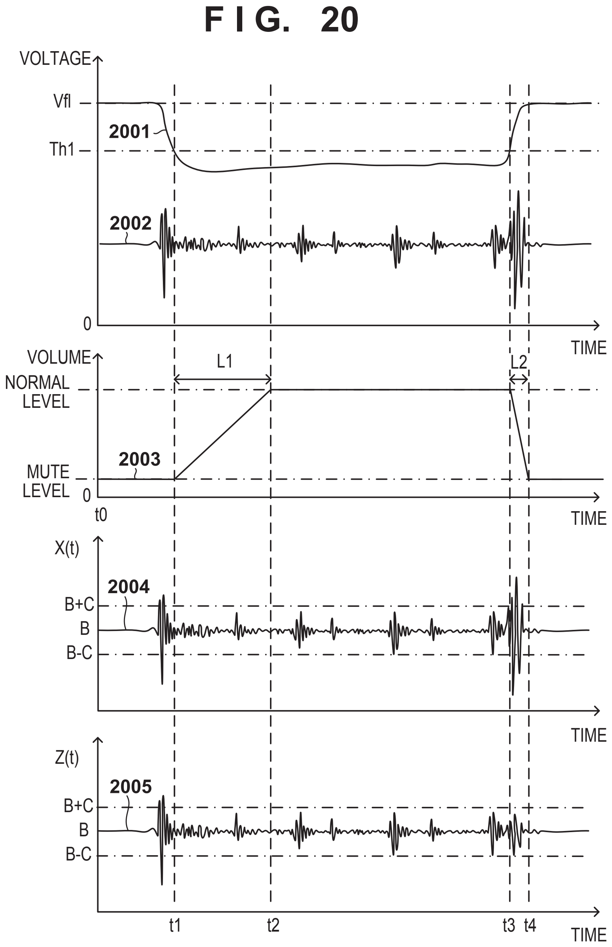

A specific example of changes in the volume and the amplitude made through the method illustrated in FIGS. 19A and 19B will be described with reference to FIG. 20. A graph 2001 represents the temporal change in the diaphragm displacement signal supplied to the microcontroller 1700. A graph 2002 represents the temporal change in the cardiac sound signal supplied to the microcontroller 1700. A graph 2003 represents a temporal change in the volume. A graph 2004 represents the temporal change in the sound signal X. A graph 2005 represents the temporal change in the sound signal Z.

A reference voltage Vfl represents the value of the diaphragm displacement signal in a state where the diaphragm 206 is not in contact with the surface of the target body, i.e., is flat. The reference voltage Vfl is determined when the electronic stethoscope 1000 is manufactured, and is stored in the non-volatile memory 1702. A threshold voltage Th1 represents the value of the diaphragm displacement signal at the boundary between the unused state and the in-use state. Like the reference voltage Vfl, the threshold voltage Th1 is determined when the electronic stethoscope 1000 is manufactured, and is stored in the non-volatile memory 1702.

The pressing detection unit 1805 determines that the pressing state is the unused state when the value of the diaphragm displacement signal is greater than the threshold voltage Th1. On the other hand, the pressing detection unit 1805 determines that the pressing state is the in-use state when the value of the diaphragm displacement signal is lower than the threshold voltage Th1. The pressing detection unit 1805 may determine that the pressing state is either state when the value of the diaphragm displacement signal is equal to the threshold voltage Th1. Instead of comparing the value of the diaphragm displacement signal with the threshold voltage Th1, the pressing detection unit 1805 may compare a difference between the reference voltage Vfl and the value of the diaphragm displacement signal with a threshold. For example, the pressing detection unit 1805 may determine that the pressing state is the unused state when the difference between the voltage Vfl and the value of the diaphragm displacement signal is less than the threshold. The pressing detection unit 1805 may determine that the pressing state is the in-use state when the difference between the voltage Vfl and the value of the diaphragm displacement signal is greater than the threshold. The pressing detection unit 1805 may also count the number of times the value of the diaphragm displacement signal has fallen below the threshold voltage Th1, and determine that the pressing state is the in-use state when that number of times exceeds a predetermined number within a set period of time.

It is assumed that at time t0, the diaphragm 206 is not pressed, that is, a flat state. The value of the diaphragm displacement signal is therefore equal to the reference voltage Vfl. As such, the pressing detection unit 1805 determines that the pressing state is the unused state. As a result, the volume adjustment unit 1809 sets the volume to the mute level. From time t0 to t1, the value of the diaphragm displacement signal is greater than the threshold voltage Th1. As such, the volume adjustment unit 1809 keeps the volume at the mute level.

At time t1, the value of the diaphragm displacement signal falls below the threshold voltage Th1. In response, the pressing detection unit 1805 determines that the pressing state has changed from the unused state to the in-use state. As such, the volume adjustment unit 1809 switches the volume from the mute level to the normal level. However, abruptly switching the volume from the mute level to the normal level may cause discomfort to the user. Accordingly, in the present embodiment, the volume adjustment unit 1809 changes the volume from the mute level to the normal level over a length of time L1. In other words, the length of time L1 is the time required to reduce the volume from the mute level to the normal level. The length of time L1 is preferably 100 ms to 500 ms, for example. The change in volume may be linear or non-linear.

From time t1 to t3, the value of the diaphragm displacement signal is lower than the threshold voltage Th1. As such, after finishing setting the volume to the normal level at time t2, the volume adjustment unit 1809 keeps the volume at the normal level.

At time t3, the value of the diaphragm displacement signal exceeds the threshold voltage Th1. In response, the pressing detection unit 1805 determines that the pressing state has changed from the in-use state to the unused state. In the present embodiment, the pressing state is determined to have changed from the in-use state to the unused state when the value of the diaphragm displacement signal exceeds the threshold Th1 even once. However, taking into account the effects of instantaneous noise, the pressing state may be determined to have changed from the in-use state to the unused state when the value of the diaphragm displacement signal exceeds the threshold Th1 a predetermined number of times within a set period of time. If the pressing detection unit 1805 determines that the pressing state has changed from the in-use state to the unused state, the volume adjustment unit 1809 switches the volume from the normal level to the mute level. Unlike when switching the volume from the mute level to the normal level, when switching the volume from the normal level to the mute level, it is preferable to switch to the mute level in a short period of time. This is because vibrations in the diaphragm 206 caused by the sound of the patient's clothes rubbing or the like may be extracted as a cardiac sound signal while the pressing state of the diaphragm 206 is changing to the unused state, and if that sound is output as audio, a sound uncomfortable to the user may be output. As such, the volume adjustment unit 1809 changes the volume from the mute level to the normal level at a length of time L2 shorter than the length of time L1. In other words, the length of time L2 is the time required to increase the volume from the normal level to the mute level. It is preferable that the length of time L2 be 10 ms to 100 ms, for example. The change in volume may be linear or non-linear.

From time t4 on, the value of the diaphragm displacement signal is greater than the threshold voltage Th1. As such, after the volume has finished switching to the mute level, the volume adjustment unit 1809 keeps the volume at the mute level.

When the pressing state is the unused state, the sound signal does not include the physiological component, and is therefore all noise. As such, the user of the electronic stethoscope 1000 does not need such a sound signal. In particular, immediately before time t1, the user moves the electronic stethoscope 1000 to bring the diaphragm 206 into close contact with the surface of the target body. As indicated by the graph 2002, the cardiac sound signal can contain a large amount of noise due to such actions by the user. Accordingly, the electronic stethoscope 1000 sets the volume to the mute level when the pressing state is the unused state, which suppresses situations where the user hears noise.

From time t1 to t2, the volume adjustment unit 1809 gradually increases the volume from the mute level to the normal level over the relatively long length of time L1. This suppresses situations where the user is confused by sudden increases in the volume. On the other hand, from time t3 to t4, the volume adjustment unit 1809 reduces the volume from the normal level to the mute level over the relatively short length of time L2. This makes it possible to quickly transition to the mute state so that the user is not subjected to noise.

The processor 1701 outputs the sound signal Z. The processor 1701 does not perform the amplitude limiting or the smoothing in periods other than time t3 to t4. Accordingly, the signal value of the sound signal Z is equal to the signal value of the sound signal X. The processor 1701 performs the amplitude limiting and the smoothing in the period from time t3 to t4. Accordingly, the amplitude of the sound signal Z is a value obtained by reducing the amplitude of the sound signal X.

Setting Operation 2 for Volume of Electronic Auscultation Apparatus of Second Embodiment

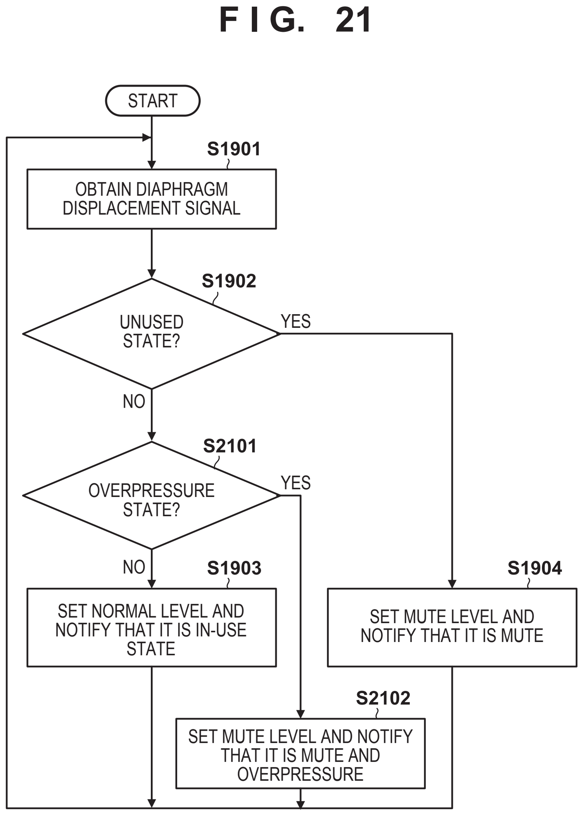

Another volume setting operation performed by the electronic stethoscope 1000 will be described with reference to FIG. 21. The following will describe the differences from the method in FIG. 19A. In the method in FIG. 21, the pressing detection unit 1805 specifies which of three states the pressing state is, namely the unused state, the correct state, or the overpressure state.

In S1902, if the pressing state is determined not to be the unused state ("NO" in S1902), the processor 1701 (e.g., the pressing detection unit 1805) moves the sequence to S2101. In S2101, the processor 1701 (e.g., the pressing detection unit 1805) determines whether the pressing state is the overpressure state. The processor 1701 moves the sequence to S2102 if the pressing state is determined to be the overpressure state ("YES" in S2101), and to S1903 if not ("NO" in S2101). In the method in FIG. 21, determining that the pressing state is neither the unused state nor the overpressure state means that the pressing state is determined to be the correct state.

In S2102, the processor 1701 (e.g., the volume adjustment unit 1809) sets the volume to the mute level. In other words, if the current volume is at the mute level, the processor 1701 maintains the current volume, whereas if the current volume is at the normal level, the processor 1701 switches the volume to the mute level. In addition, the processor 1701 notifies the user of information pertaining to the pressing state of the diaphragm 206, and that the volume is at the mute level. The information pertaining to the pressing state of the diaphragm 206 includes that the pressing state is the overpressure state or the pressing state is the unused state. For example, the output control unit 1806 may make this notification through the wireless communication unit 614 or the wired communication unit 617, or the display control unit 1802 may make this notification by lighting the LED included in the display device 122. Additionally, in S1903 and S1904 in the method of FIG. 21, the processor 1701 (e.g., the output control unit 1806) also notifies the user of information pertaining to the pressing state of the diaphragm 206 (e.g., that the pressing state is not the overpressure state).

Although the volume is set to the mute level by the processor 1701 (e.g., the volume adjustment unit 1809) in S2102 in the present embodiment, the volume may be set to the normal level, for example.

According to the method in FIG. 21, if the pressing state is the correct state, the volume level is set to the normal level. If the pressing state is the unused state or the overpressure state, the volume level is set to the mute level. The method for setting the volume when the pressing state is the correct state or the unused state is the same as that in FIG. 19A.

Specific Example 2 of Change in Volume in Electronic Auscultation Apparatus of Second Embodiment

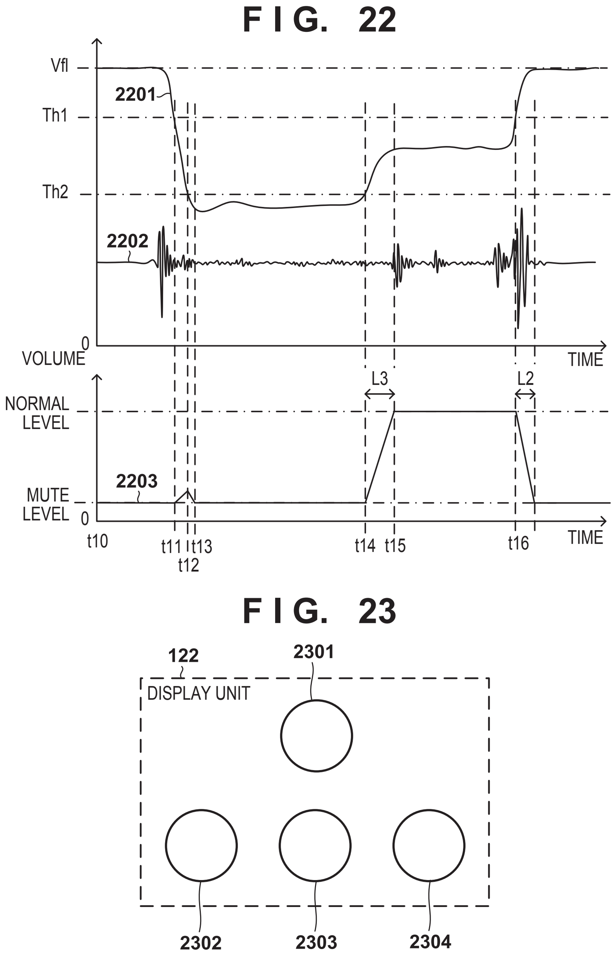

A specific example of the change in the volume made through the method illustrated in FIG. 21 will be described with reference to FIG. 22. A graph 2201 represents the temporal change in the diaphragm displacement signal supplied to the microcontroller 1700. A graph 2202 represents the temporal change in the cardiac sound signal supplied to the microcontroller 1700. A graph 2203 represents a temporal change in the volume.

The reference voltage Vfl represents the value of the diaphragm displacement signal in a state where the diaphragm 206 is not pressed (a flat state). The reference voltage Vfl is determined through testing when the electronic stethoscope 1000 is manufactured, and may be stored in the non-volatile memory 1702. The threshold voltage Th1 represents the value of the diaphragm displacement signal at the boundary between the unused state and the correct state. The threshold voltage Th1 is determined when the electronic stethoscope 1000 is manufactured, and may be stored in the non-volatile memory 1702. A threshold voltage Th2 represents the value of the diaphragm displacement signal at the boundary between the correct state and the overpressure state. The threshold voltage Th2 is determined when the electronic stethoscope 1000 is manufactured, and may be stored in the non-volatile memory 1702.

The pressing detection unit 1805 may determine that the pressing state is the unused state when the value of the diaphragm displacement signal is greater than the threshold voltage Th1. The pressing detection unit 1805 may determine that the pressing state is the correct state when the value of the diaphragm displacement signal is lower than the threshold voltage Th1 and greater than the threshold voltage Th2. The pressing detection unit 1805 may determine that the pressing state is the overpressure state when the value of the diaphragm displacement signal is lower than the threshold voltage Th2. When the value of the diaphragm displacement signal is on the boundary, the pressing detection unit 1805 may determine that the value is on either side of the boundary. Similar to the descriptions of FIG. 20, instead of comparing the value of the diaphragm displacement signal with the threshold voltages Th1 and Th2, the pressing detection unit 1805 may compare a difference between the reference voltage Vfl and the value of the diaphragm displacement signal with a threshold.

It is assumed that at time t10, the diaphragm 206 is in a flat state. As such, the value of the diaphragm displacement signal is equal to the reference voltage Vfl, and thus the pressing state is determined to be the unused state. As a result, the volume adjustment unit 1809 sets the volume to the mute level. From time t0 to t1, the value of the diaphragm displacement signal is greater than the threshold voltage Th1. As such, the volume adjustment unit 1809 keeps the volume at the mute level.

At time t11, the value of the diaphragm displacement signal falls below the threshold voltage Th1. In response, the pressing detection unit 1805 determines that the pressing state has changed from the unused state to the correct state. As such, the volume adjustment unit 1809 switches the volume from the mute level to the normal level. However, due to the strong compressive force of the diaphragm 206 by the user, at time t12, the value of the diaphragm displacement signal falls below the threshold voltage Th2. In response, the pressing detection unit 1805 determines that the pressing state has changed from the correct state to the overpressure state. As such, the volume adjustment unit 1809 switches the volume from the normal level to the mute level.

From time t12 to t14, the value of the diaphragm displacement signal is lower than the threshold voltage Th2. As such, after the volume has finished switching to the mute level at time t13, the volume adjustment unit 1809 keeps the volume at the mute level.

At time t14, in response to the user reducing the compressive force of the diaphragm 206, the value of the diaphragm displacement signal exceeds the threshold voltage Th2. In response, the pressing detection unit 1805 determines that the pressing state has changed from the overpressure state to the correct state. As such, the volume adjustment unit 1809 switches the volume from the mute level to the normal level. The volume adjustment unit 1809 may change the volume from the mute level to the normal level over a length of time L3. The length of time L3 may be 100 ms to 500 ms, for example. The change in volume may be linear or non-linear.

The length of time L3 may be the same as the length of time L1, or may be shorter than the length of time L1. The user performing an operation to reduce the compressive force of the diaphragm 206 can be considered to be the user being prepared to listen to the sound signal. As such, setting the length of time L3 to be shorter than the length of time L1 makes it possible to deliver the sound signal to the user quickly. The length of time L3 may be the same as the length of time L2, or may be longer than the length of time L2.

From time t14 to t16, the value of the diaphragm displacement signal is lower than the threshold voltage Th1 and greater than the threshold voltage Th2. As such, after finishing switching the volume to the normal level at time t15, the volume adjustment unit 1809 keeps the volume at the normal level. The operations after time t16 are the same as the operations after time t3 in FIG. 20, and thus redundant descriptions thereof will be omitted.

A specific example of the display device 122 will be described with reference to FIG. 23. The display device 122 includes four light-emitting units 2301 to 2304. Each of the light-emitting units 2301 to 2304 is constituted by an LED, for example.

The light-emitting unit 2301 lights up when the power of the electronic stethoscope 1000 is on, and turns off when the power of the electronic stethoscope 1000 is off. Having the light-emitting unit 2301 turned on or off by the display control unit 1802 enables the user to easily understand the power state of the electronic stethoscope 1000. Note that the display control unit 1802 may turn off the light-emitting units 2302 to 2304 in addition to the light-emitting unit 2301 when the power of the electronic stethoscope 1000 is turned off. The display control unit 1802 may also cause the light-emitting unit 2301 to flash for several seconds after the power of the electronic stethoscope 1000 is turned on to notify the user that preparations for startup processing are underway.

The light-emitting unit 2302 notifies the user that the volume level is the mute level. For example, the display control unit 1802 lights the light-emitting unit 2302 when the volume level is the mute level. The display control unit 1802 turns off the light-emitting unit 2302 when the volume level is the normal level. This enables the user to easily understand that the volume level is the mute level.

The light-emitting unit 2303 may notify the user that the pressing state is the in-use state. Specifically, the display control unit 1802 lights the light-emitting unit 2303 when the pressing state is the in-use state. The display control unit 1802 turns off the light-emitting unit 2303 when the pressing state is not the in-use state (i.e., the unused state). This enables the user to easily understand that the pressing state is the in-use state.

The light-emitting unit 2304 notifies the user that the pressing state is the overpressure state. For example, the display control unit 1802 lights the light-emitting unit 2304 when the pressing state is the overpressure state. The display control unit 1802 turns off the light-emitting unit 2304 when the pressing state is not the overpressure state (i.e., the correct state or the unused state). This enables the user to easily understand that the pressing state is the overpressure state.

Although the present embodiment describes a configuration in which the display control unit 1802 makes notifications regarding the state of the electronic stethoscope 1000 using the light-emitting units 2301 to 2304, the present invention is not limited thereto. For example, if the display device 122 includes a liquid crystal display, a message may be displayed on the liquid crystal display. Alternatively, an audio message indicating the state of the electronic stethoscope 1000 may be transmitted to the audio output device 620.

Variations on Power Supply Unit of Electronic Auscultation Apparatus of Second Embodiment

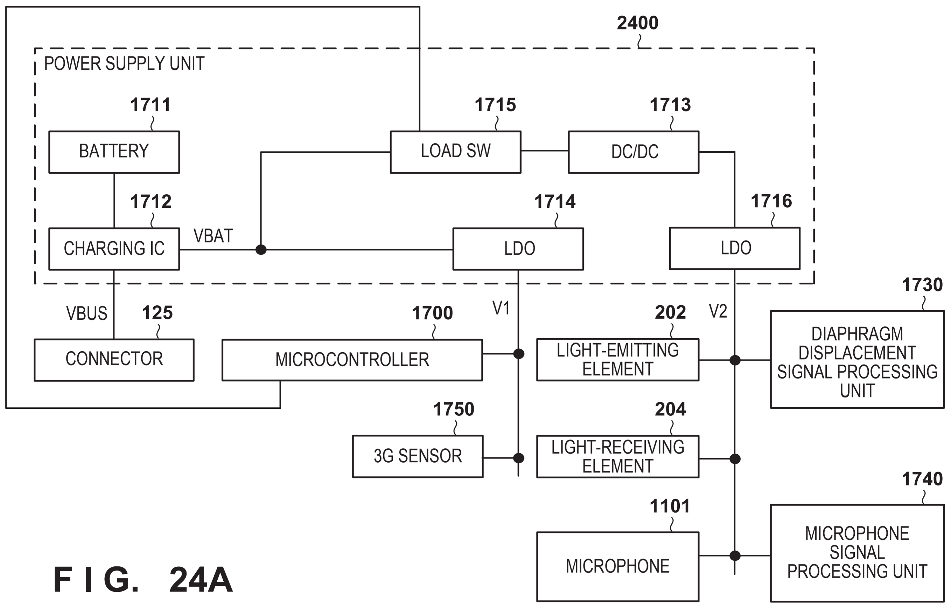

Variations on the power supply unit 1710 illustrated in FIG. 17A will be described with reference to FIGS. 24A to 24C. A power supply unit 2400 according to the variation illustrated in FIG. 24A differs from the power supply unit 1710 in terms of the location of the boost converter 1713. In the power supply unit 2400, the boost converter 1713 is disposed in the path between the load switch 1715 and the voltage regulator 1716. The voltage VBAT from the charging integrated circuit 1712 is supplied to each of the voltage regulator 1714 and the load switch 1715. The voltage VBAT (e.g., 3.7 V) is higher than the output voltage of the voltage regulator 1714 (e.g., 3.3 V). Accordingly, the voltage regulator 1714 outputs the voltage V1 while the voltage VBAT is being supplied.

The voltage VBAT from the charging integrated circuit 1712 is supplied to the boost converter 1713 when the load switch 1715 is on. The boost converter 1713 boosts the voltage VBAT to a voltage (e.g., 6.8 V) that is higher than the output voltage of the voltage regulator 1716 (e.g., 5.8 V), and supplies this voltage to the voltage regulator 1716. The voltage regulator 1716 outputs the voltage V2 while the voltage is being supplied from the boost converter 1713.

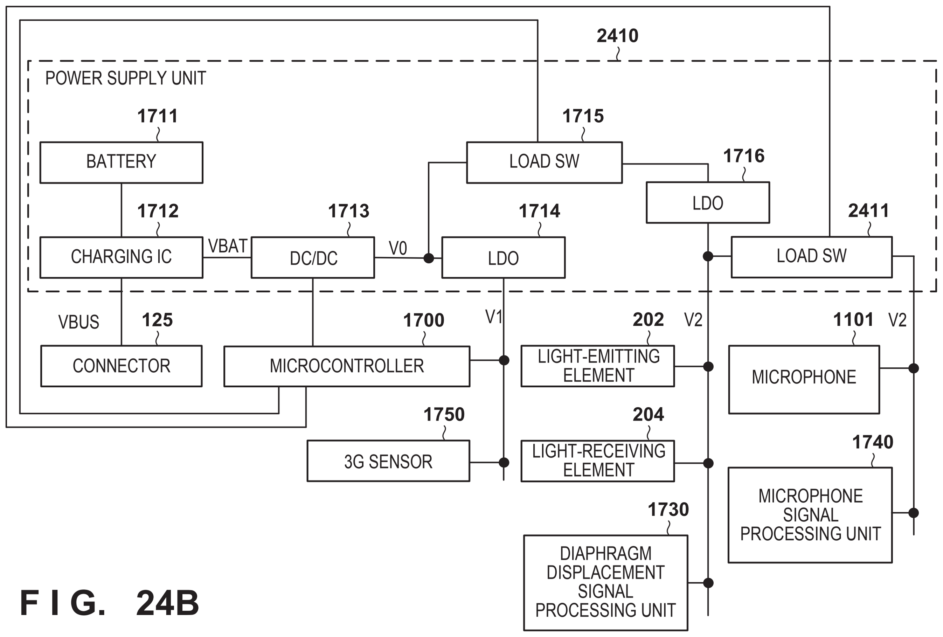

A power supply unit 2410 according to the variation illustrated in FIG. 24B differs from the power supply unit 1710 in that a load switch 2411 is further provided. The boost converter 1713 may be disposed in the position illustrated in FIG. 24A instead of the position illustrated in FIG. 24B.

The load switch 2411 is a switch that switches on (a conductive state) and off (a non-conductive state) in response to a control signal from the microcontroller 1700. The voltage V2 is supplied to the load switch 2411 from the voltage regulator 1716. This voltage V2 is output from the output terminal of the load switch 2411 when the load switch 2411 is on. This voltage V2 is not output from the output terminal of the load switch 2411 when the load switch 2411 is off. The voltage at the output terminal of the load switch 2411 may be a ground voltage when the load switch 2411 is off.

When the power supply unit 2410 is used in the electronic stethoscope 1000, the operating power of the condenser microphone 1101 may be provided from the voltage regulator 1716 through the load switch 2411. The operating power of the circuit elements included in the microphone signal processing unit 1740 may also be provided from the voltage regulator 1716 through the load switch 2411. On the other hand, even if the power supply unit 2410 is used in the electronic stethoscope 1000, the operating power of the light emitter 202 and the photodetector 204 may be provided directly from the voltage regulator 1716 (i.e., without going through the load switch 2411). The operating power of the circuit elements included in the diaphragm displacement signal processing unit 1730 may also be provided from the voltage regulator 1716 through the load switch 2411.

According to the power supply unit 2410, the microcontroller 1700 (e.g., the power management unit 1804) can stop supplying power to the vibration detection unit 1012 (e.g., including the condenser microphone 1101) while power is being supplied to the displacement detection unit 1011 (e.g., including the light emitter 202 and the photodetector 204). With the power supply unit 2410, the voltage generated by the same voltage regulator 1716 is supplied to both the displacement detection unit 1011 and the vibration detection unit 1012. Instead, however, the power supply unit 2410 may include a voltage regulator, separate from the voltage regulator 1716, for generating the voltage supplied to the vibration detection unit 1012.

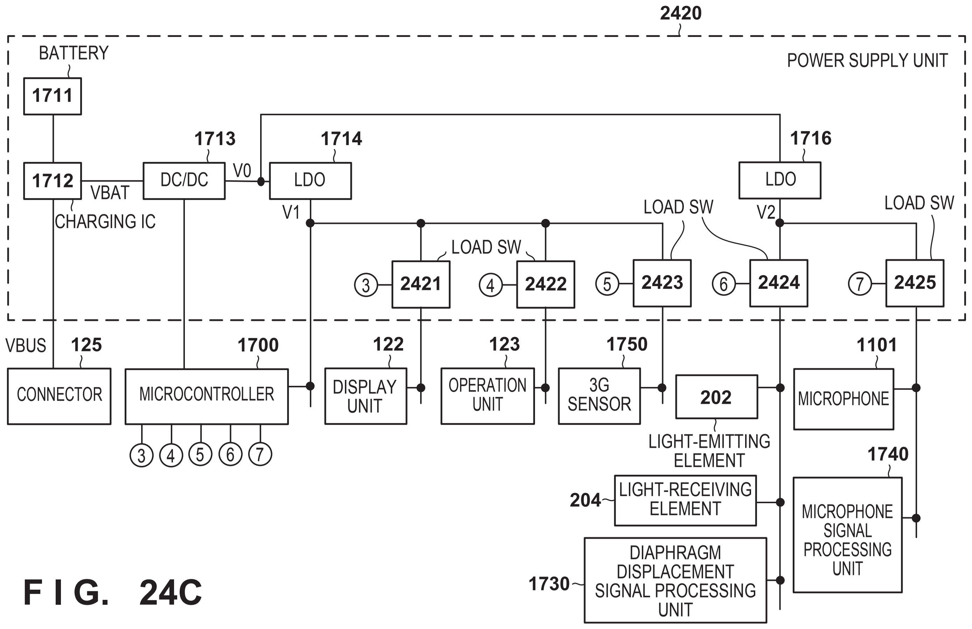

A power supply unit 2420 according to the variation illustrated in FIG. 24C differs from the power supply unit 1710 in that load switches 2421 to 2425 are provided instead of the load switch 1715. In the power supply unit 2420, the voltage V0 is supplied to the voltage regulator 1716 directly from the boost converter 1713. The load switches 2421 to 2425 are switches that switch on and off in response to control signals from the microcontroller 1700. A voltage V1 is supplied to the load switches 2421 to 2423 from the voltage regulator 1714. This voltage V1 is output from the output terminal of the load switch 2421 when the load switch 2421 is on. This voltage V1 is not output from the output terminal of the load switch 2421 when the load switch 2421 is off. The voltage at the output terminal of the load switch 2421 may be a ground voltage when the load switch 2421 is off. The same applies to the voltages output from the load switches 2422 to 2423. The voltage V2 is supplied to the load switches 2424 and 2425 from the voltage regulator 1716. This voltage V2 is output from the output terminal of the load switch 2424 when the load switch 2424 is on. This voltage V2 is not output from the output terminal of the load switch 2424 when the load switch 2424 is off. The voltage at the output terminal of the load switch 2424 may be a ground voltage when the load switch 2424 is off. The same applies to the voltage output from the load switch 2425.

The output terminal of the load switch 2421 is connected to the display device 122. The voltage V1 is supplied from the power supply unit 2420 to the display device 122 while the load switch 2421 is on. The display device 122 operates using the voltage V1. The voltage V1 is not supplied to the display device 122 while the load switch 2421 is off. The output terminal of the load switch 2422 is connected to the operation unit 123. The voltage V1 is supplied from the power supply unit 2420 to the operation unit 123 while the load switch 2422 is on. The operation unit 123 operates using the voltage V1. The voltage V1 is not supplied to the operation unit 123 while the load switch 2422 is off. The output terminal of the load switch 2423 is connected to the accelerometer 1750. The voltage V1 is supplied from the power supply unit 2420 to the accelerometer 1750 while the load switch 2423 is on. The accelerometer 1750 operates using the voltage V1. The voltage V1 is not supplied to the accelerometer 1750 while the load switch 2423 is off.

The output terminal of the load switch 2424 is connected to the light emitter 202, the photodetector 204, and the diaphragm displacement signal processing unit 1730. The voltage V2 is supplied from the power supply unit 2420 to the light emitter 202, the photodetector 204, and the diaphragm displacement signal processing unit 1730 while the load switch 2424 is on. The light emitter 202, the photodetector 204, and the diaphragm displacement signal processing unit 1730 operate using the voltage V2. The voltage V2 is not supplied to the light emitter 202, the photodetector 204, and the diaphragm displacement signal processing unit 1730 while the load switch 2424 is off. The output terminal of the load switch 2425 is connected to the condenser microphone 1101 and the microphone signal processing unit 1740. The voltage V2 is supplied from the power supply unit 2420 to the condenser microphone 1101 and the microphone signal processing unit 1740 while the load switch 2425 is on. The condenser microphone 1101 and the microphone signal processing unit 1740 operate using the voltage V2. The voltage V2 is not supplied to the condenser microphone 1101 and the microphone signal processing unit 1740 while the load switch 2425 is off.

The microcontroller 1700 can control the load switches 2421 to 2425 to turn on and off individually. Accordingly, the microcontroller 1700 can set whether to supply operating power to each of the plurality of constituent elements connected to the output terminals of the load switches 2421 to 2425 on an individual basis. In the power supply unit 2420, any one or more load switches among the load switches 2421 to 2425 may be omitted, and the voltage V1 or V2 may be directly supplied to the corresponding constituent element.

Variations on Wired Communication Unit of Electronic Auscultation Apparatus of Second Embodiment

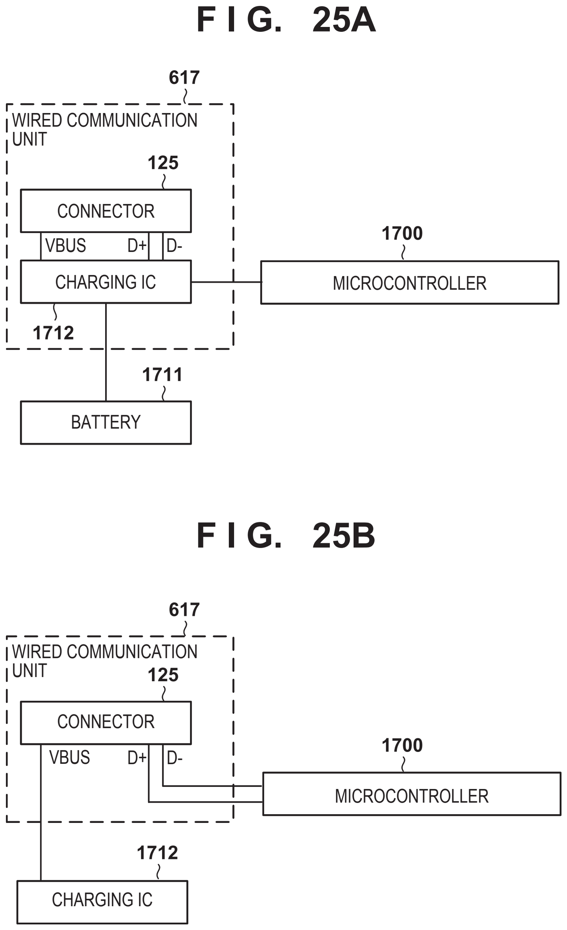

Variations on the wired communication unit 617 illustrated in FIG. 17A will be described with reference to FIGS. 25A and 25B. In the variation illustrated in FIG. 25A, the wired communication unit 617 is constituted by the connector 125 and the charging integrated circuit 1712. The microcontroller 1700 may be capable of communicating with the charging integrated circuit 1712, for example, through I2C. The charging integrated circuit 1712 may also be capable of communicating with an external device connected to the connector 125. In such a configuration, the charging integrated circuit 1712 may support charging by USB Power Delivery (USB PD). In the variation illustrated in FIG. 25B, the wired communication unit 617 is constituted by the connector 125. The microcontroller 1700 may be capable of communicating directly with an external device connected to the connector 125.

Operating Modes of Electronic Auscultation Apparatus of Second Embodiment

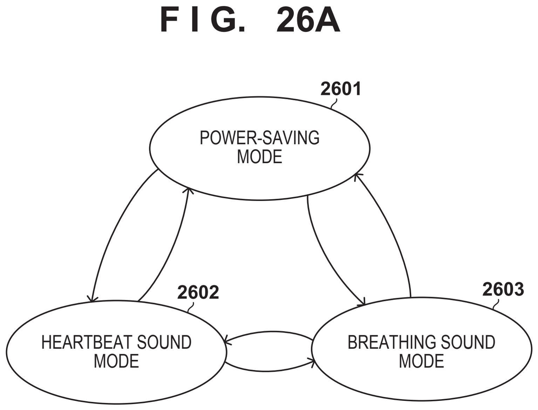

An example of transitions among operating modes of the electronic stethoscope 1000 will be described with reference to FIG. 26A. The electronic stethoscope 1000 of the second embodiment is capable of operating in a plurality of operating modes, including the low-power mode 2601, a cardiac sound mode 2602, and a respiratory sound mode 2603. The low-power mode 2601 is an operating mode that consumes less power than the cardiac sound mode 2602 and the respiratory sound mode 2603. To reduce power consumption, in the present embodiment, the electronic stethoscope 1000 is configured to transition to the low-power mode 2601 under set conditions, such as when not in use. The cardiac sound mode 2602 is an operating mode used for listening to cardiac sounds, and is one auscultation mode. In the cardiac sound mode 2602 in the present embodiment, the voltage V1 is supplied to the microcontroller 1700, and the voltage V2 is supplied to the photosensor including the light emitter 202 and the photodetector 204. In the cardiac sound mode 2602, the cardiac sound signal can be transmitted to the exterior (e.g., the computer 630 or the audio output device 620) using the light emitter 202 and the photodetector 204. The respiratory sound mode 2603 is an operating mode used for listening to respiratory sounds, and is one auscultation mode. In the respiratory sound mode 2603 in the present embodiment, the voltage V1 is supplied to the microcontroller 1700, and the voltage V2 is supplied to the condenser microphone 1101. In the respiratory sound mode 2603, the respiratory sound signal can be output to the external device using the condenser microphone 1101. The electronic stethoscope 1000 may further be capable of operating in an operating mode in which both the cardiac sound signal and the respiratory sound signal are transmitted to the exterior (e.g., the computer 630). However, if the electronic stethoscope does not include the condenser microphone 1101, as in the first embodiment, the respiratory sound mode 2603 is not included.

In the low-power mode 2601, the cardiac sound mode 2602, and the respiratory sound mode 2603, the power supply unit 2400 supplies operating power to the microcontroller 1700. Specifically, the voltage regulator 1714 generates the voltage V1 and supplies the voltage V1 to the microcontroller 1700. As described above, the voltage V1 is also supplied to the accelerometer 1750.

In the low-power mode 2601, the microcontroller 1700 turns the load switch 1715 off at least intermittently. For example, in the low-power mode 2601, the microcontroller 1700 turns the load switch 1715 off intermittently (i.e., on intermittently). In this case, the voltage regulator 1716 generates the voltage V2 intermittently. Instead, however, in the low-power mode 2601, the microcontroller 1700 may keep the load switch 1715 off at all times. In this case, the voltage regulator 1716 does not generate the voltage V2. When the voltage V2 is not generated, the supply of operating power to the light emitter 202, the photodetector 204, and the condenser microphone 1101 stops, and these circuit elements do not operate.

In the cardiac sound mode 2602 and the respiratory sound mode 2603, the microcontroller 1700 may keep the load switch 1715 on at all times. Instead, however, in the cardiac sound mode 2602 and the respiratory sound mode 2603, the microcontroller 1700 may turn the load switch 1715 on more frequently than in the low-power mode 2601. When the load switch 1715 is on, the voltage regulator 1716 generates the voltage V2. When the voltage V2 is generated, the light emitter 202 and the photodetector 204 are supplied with an operating voltage, and these circuit elements therefore operate. In the foregoing example, the voltage V2 is higher than the voltage V1. When the operating voltage of the light emitter 202 and the photodetector 204 is lower than the operating voltage of the microcontroller 1700, the voltage V2 may be lower than the voltage V1. When the electronic stethoscope 1000 includes the power supply unit 1710 or the power supply unit 2400, the load switch 1715 is turned on to supply operating power to the condenser microphone 1101 as well.

When the electronic stethoscope 1000 includes the power supply unit 2410, in the cardiac sound mode 2602, the microcontroller 1700 may keep the load switch 2411 off at all times. When the electronic stethoscope 1000 includes the power supply unit 2410, in the respiratory sound mode 2603, the microcontroller 1700 may keep the load switch 2411 on at all times.

As described above, the power supplied to the light emitter 202 and the photodetector 204 in the low-power mode 2601 is lower than the power supplied to the light emitter 202 and the photodetector 204 in the cardiac sound mode 2602 and the respiratory sound mode 2603. In addition, the power supplied to the condenser microphone 1101 in the low-power mode 2601 is lower than the power supplied to the condenser microphone 1101 in the respiratory sound mode 2603. The power supplied to the condenser microphone 1101 in the cardiac sound mode 2602 may be lower than the power supplied to the condenser microphone 1101 in the cardiac sound mode 2602. Such a configuration saves energy in the electronic stethoscope 1000.

The electronic stethoscope 1000 may have a plurality of low-power modes. The plurality of low-power modes are implemented by the electronic stethoscope 1000 having the power supply unit 2420 (FIG. 24C). The power supplied to the constituent elements in each mode will be described with reference to Table 4 below.

Table 4

|  | Display unit | Operation unit | Accelerometer | Displacement detection unit | Vibration detection unit |
|---|---|---|---|---|---|
| Heartbeat sound mode | ON | ON | ON | ON | OFF |
| Breathing sound mode | ON | ON | ON | OFF | ON |
| Power-saving mode A | OFF | ON | OFF | OFF | OFF |
| Power-saving mode B | OFF | OFF | ON | OFF | OFF |

(continued)

|  | Display unit | Operation unit | Accelerometer | Displacement detection unit | Vibration detection unit |
|---|---|---|---|---|---|
| Power-saving mode C | OFF | OFF | OFF | ON | OFF |
| Power-saving mode D | OFF | OFF | OFF | OFF | ON |

In the example in Table 4, the electronic stethoscope 1000 has four low-power modes A to D. In Table 4, "ON" indicates that power is supplied to the corresponding constituent element, and "OFF" indicates that power is not supplied to the corresponding constituent element. The displacement detection unit 1011 includes the light emitter 202, the photodetector 204, and the diaphragm displacement signal processing unit 1730. The vibration detection unit 1012 includes the condenser microphone 1101 and the microphone signal processing unit 1740.

While the electronic stethoscope 1000 is operating in the cardiac sound mode, the microcontroller 1700 turns the load switches 2421 to 2424 on and the load switch 2425 off. As a result, operating power is supplied to the display device 122, the operation unit 123, the accelerometer 1750, and the displacement detection unit 1011, and operating power is not supplied to the vibration detection unit 1012. While the electronic stethoscope 1000 is operating in the respiratory sound mode, the microcontroller 1700 turns the load switches 2421 to 2423 and 2425 on and the load switch 2424 off. As a result, operating power is supplied to the display device 122, the operation unit 123, the accelerometer 1750, and the vibration detection unit 1012, and operating power is not supplied to the displacement detection unit 1011.

While the electronic stethoscope 1000 is operating in the low-power mode A, the microcontroller 1700 turns the load switch 2422 on and the load switches 2421 and 2423 to 2425 off. As a result, operating power is supplied to the operation unit 123, and operating power is not supplied to the display device 122, the accelerometer 1750, the displacement detection unit 1011, and the vibration detection unit 1012. The microcontroller 1700 returns from the low-power mode A in response to the operation unit 123 being operated by the user. Specifically, the microcontroller 1700 returns from the low-power mode A in response to the any of the plurality of buttons in the operation unit 123 being operated by the user. In this case, each button in the operation unit 123 has a function for accepting an instruction pertaining to transitioning the mode of the electronic stethoscope 1000 from the user. The operating power of the operation unit 123 is lower than the operating power of any of the display device 122, the accelerometer 1750, the displacement detection unit 1011, and the vibration detection unit 1012. Accordingly, the low-power mode A consumes less power than any of the low-power modes B to D.

While the electronic stethoscope 1000 is operating in the low-power mode B, the microcontroller 1700 turns the load switch 2423 on and the load switches 2421, 2422, 2424 and 2425 off. As a result, operating power is supplied to the accelerometer 1750, and operating power is not supplied to the display device 122, the operation unit 123, the displacement detection unit 1011, and the vibration detection unit 1012. The microcontroller 1700 returns from the low-power mode B in response to the accelerometer 1750 detecting a predetermined operation. For example, the microcontroller 1700 returns from the low-power mode B when upward acceleration (the direction opposite from the direction of gravity) in the electronic stethoscope 1000 is greater than a threshold. Such acceleration can occur when the electronic stethoscope 1000 is lifted by the user. Accordingly, the microcontroller 1700 returns from the low-power mode B in accordance with a natural action performed by the user to use the electronic stethoscope 1000.

In the foregoing example, an accelerometer 1750 is used for returning from the low-power mode B, but an electrostatic sensor may be used instead of the accelerometer 1750. The electrostatic sensor is provided on the surface of the grip 120, and is used to detect that the user has gripped the grip 120. While the electronic stethoscope 1000 is operating in the low-power mode B, the microcontroller 1700 supplies operating power to the electrostatic sensor. The microcontroller 1700 returns from the low-power mode B in response to detecting the user making contact with the electrostatic sensor. In this case, too, the microcontroller 1700 returns from the low-power mode B in accordance with a natural action performed by the user to use the electronic stethoscope 1000.

While the electronic stethoscope 1000 is operating in the low-power mode C, the microcontroller 1700 turns the load switch 2424 on and the load switches 2421 to 2423 and 2425 off. As a result, operating power is supplied to the displacement detection unit 1011, and operating power is not supplied to the display device 122, the operation unit 123, the accelerometer 1750, and the vibration detection unit 1012. The microcontroller 1700 returns from the low-power mode C in response to the diaphragm 206 being pressed. For example, the microcontroller 1700 returns from the low-power mode C when the output from the photodetector 204 based on the displacement amount of the diaphragm 206 (i.e., the diaphragm displacement signal) exceeds a threshold. In this manner, the microcontroller 1700 returns from the low-power mode C in accordance with a natural action performed by the user to use the electronic stethoscope 1000.

While the electronic stethoscope 1000 is operating in the low-power mode D, the microcontroller 1700 turns the load switch 2425 on and the load switches 2421 to 2424 off. As a result, operating power is supplied to the vibration detection unit 1012, and operating power is not supplied to the display device 122, the operation unit 123, the accelerometer 1750, and the displacement detection unit 1011. The microcontroller 1700 returns from the low-power mode D in response to the condenser microphone 1101 detecting a predetermined sound. For example, the microcontroller 1700 returns from the low-power mode D in response to the condenser microphone 1101 detecting a sound at a wavelength corresponding to the respiratory sound. In this manner, the microcontroller 1700 returns from the low-power mode D in accordance with a natural action performed by the user to use the electronic stethoscope 1000.

In the low-power modes B to D, the apparatus returns from the low-power mode without the user operating the operation unit 123. Which of the low-power modes A to D is used may be set when the electronic stethoscope 1000 is shipped. Furthermore, which of the low-power modes A to D is used may be settable by the user. Furthermore, the electronic stethoscope 1000 need not include one or more of the low-power modes A to D. The electronic stethoscope 1000 may also have a low-power mode other than the low-power modes A to D in Table 4. For example, the electronic stethoscope 1000 may have a low-power mode in which only two or more specific load switches among the load switches 2422 to 2425 are turned on.

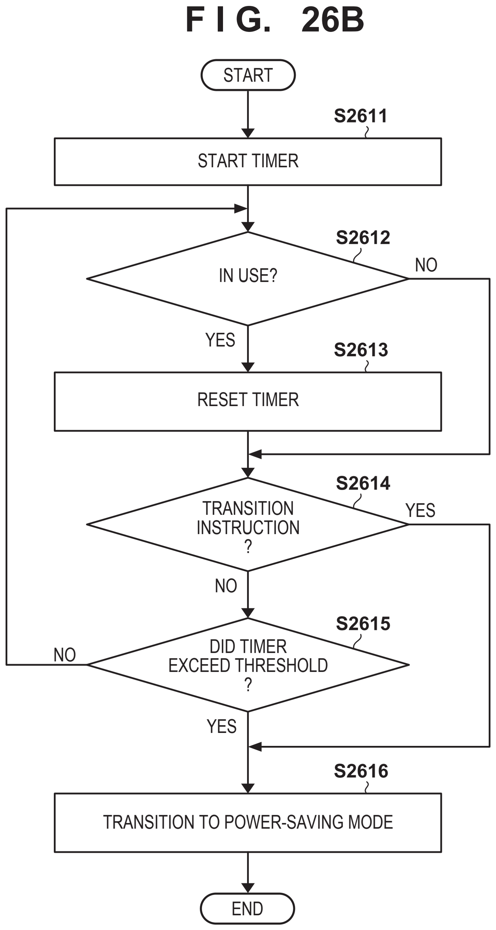

Operations for transitioning from the cardiac sound mode 2602 to the low-power mode 2601 will be described with reference to FIG. 26B. The low-power mode 2601 is a low-power mode, among the low-power modes A to D, that is set in advance. Each step of the method in FIG. 26B is realized by the processor 1701 executing a program stored in the non-volatile memory 1702. However, some or all of the steps of the method in FIG. 26B may be implemented by a dedicated integrated circuit. The processor 1701 starts the method in FIG. 26B in response to the electronic stethoscope 1000 entering the cardiac sound mode 2602. Operations for transitioning from the respiratory sound mode 2603 to the low-power mode 2601 are the same as in FIG. 26B.

In S2611, the processor 1701 starts a timer. In S2612, the processor 1701 determines whether the user is using the electronic stethoscope 1000. If the user is determined to be using the electronic stethoscope 1000 ("YES" in S2612), the processor 1701 resets the timer in S2613 and then moves the sequence to S2614. If the user is determined not to be using the electronic stethoscope 1000 ("NO" in S2612), the processor 1701 moves the sequence to S2614 without resetting the timer. If the timer is not reset, the value of the timer continues to increase.

The processor 1701 may determine whether the user is using the electronic stethoscope 1000 on the basis of at least one of the plurality of conditions. For example, the processor 1701 determines that the user is using the electronic stethoscope 1000 when at least one of the following conditions is satisfied: the pressing state of the diaphragm 206 is the in-use state; the condenser microphone 1101 is detecting a sound; the value measured by the accelerometer 1750 exceeds a threshold; and Bluetooth communication is established. Instead, however, the processor 1701 may determine that the user is using the electronic stethoscope 1000 when all of these conditions are satisfied.

In S2614, the processor 1701 determines whether an instruction to transition to a low-power mode has been obtained from the user. The processor 1701 moves the sequence to S2616 if an instruction to transition to the low-power mode is determined to have been obtained from the user ("YES" in S2614), and to S2615 if not ("NO" in S2614). In S2615, the processor 1701 determines whether the value of the timer has exceeded a threshold. The processor 1701 moves the sequence to S2616 if the value of the timer is determined to have exceeded the threshold ("YES" in S2615), and to S2612 if not ("NO" in S2615).

In this manner, the processor 1701 moves the sequence to S2616 when an instruction to transition to a low-power mode is obtained from the user or the value of the timer exceeds the threshold, and transitions to the low-power mode in S2616. In other cases, the processor 1701 repeats S2612 to S2615. When S2612 to S2615 are repeated, the timer indicates the length of time that has passed since the user was determined to have stopped using the electronic stethoscope 1000. Accordingly, when a predetermined length of time (the threshold in S2615) has passed after the user is determined to have stopped using the electronic stethoscope 1000, the processor 1701 transitions to the low-power mode even without an explicit instruction from the user to transition. For example, when the output from the photodetector 204 (i.e., the diaphragm displacement signal) does not change for a set period of time, the processor 1701 determines that a predetermined length of time (the threshold in S2615) has passed after the user is determined to have stopped using the electronic stethoscope 1000, and transitions the mode of the electronic stethoscope 1000 to the low-power mode.

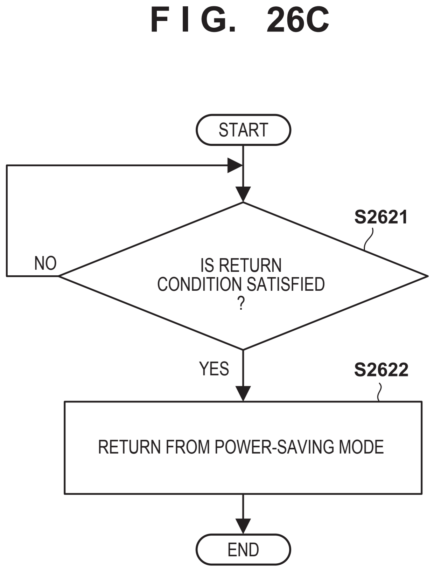

Operations for returning from the low-power mode 2601 will be described with reference to FIG. 26C. The low-power mode 2601 is a low-power mode, among the low-power modes A to D, that is set in advance. Each step of the method of FIG. 26C is executed by the processor 1701, for example. However, some or all of the steps of the method in FIG. 26C may be implemented by a dedicated integrated circuit. The processor 1701 starts the method in FIG. 26C in response to the electronic stethoscope 1000 entering the low-power mode 2601.

In S2621, the processor 1701 determines whether a condition for returning from the low-power mode 2601 ("return condition" hereinafter) is satisfied. The processor 1701 moves the sequence to S2622 if the return condition is determined to be satisfied ("YES" in S2621), and repeats S2621 if not ("NO" in S2621). In S2622, the processor 1701 returns from the low-power mode 2601.

The return condition differs depending on the low-power mode 2601, as described above with reference to Table 4. For example, as described above, in the low-power mode A, the return condition is that the operation unit 123 is operated by the user. The return condition may be that during operation of the electronic stethoscope 1000 in a low-power mode in which only two or more specific load switches among the load switches 2422 to 2425 are turned on, at least one condition for the constituent element to which operating power is supplied through the load switches that are turned on is satisfied.

Transitions of Operating Modes of Electronic Auscultation Apparatus of Second Embodiment

Transitions among the operating modes will be described next. In the low-power mode 2601, the microcontroller 1700 identifies a state of the electronic stethoscope 1000. The microcontroller 1700 may transition from the low-power mode 2601 to the cardiac sound mode 2602 or the respiratory sound mode 2603 on the basis of the identified state. The microcontroller 1700 transitions to the cardiac sound mode 2602 when the current setting of the output selection unit 1807 is the cardiac sound signal, and transitions to the respiratory sound mode 2603 when the setting is the respiratory sound signal. As described above, the setting in the output selection unit 1807 may be changeable by a user input made through the operation unit 123. The following descriptions assume that the cardiac sound signal is set by the output selection unit 1807.

For example, in the low-power mode 2601, the microcontroller 1700 identifies a state of the diaphragm 206 on the basis of the diaphragm displacement signal. Specifically, the identified state of the electronic stethoscope 1000 is the state of the diaphragm 206. In this case, the microcontroller 1700 transitions from the low-power mode 2601 to the cardiac sound mode 2602 on the basis of the state of the diaphragm 206. In the low-power mode 2601, the microcontroller 1700 turns the load switch 1715 on intermittently (e.g., at a period of 10 ms to 100 ms) to obtain the diaphragm displacement signal, and turns the load switch 1715 off once the diaphragm displacement signal is obtained.

The microcontroller 1700 transitions from the low-power mode 2601 to the cardiac sound mode 2602 on the basis of the pressing state of the diaphragm 206 changing from the unused state to the in-use state described above. For example, the microcontroller 1700 transitions from the low-power mode 2601 to the cardiac sound mode 2602 in response to the pressing state of the diaphragm 206 changing from the unused state to the in-use state.

Additionally, in the low-power mode 2601, the microcontroller 1700 identifies motion of the electronic stethoscope 1000 on the basis of an acceleration signal output from the accelerometer 1750. In other words, the identified state of the electronic stethoscope 1000 is movement of the electronic stethoscope 1000. In this case, the microcontroller 1700 transitions from the low-power mode 2601 to the cardiac sound mode 2602 on the basis of the motion of the electronic stethoscope 1000 detected by the accelerometer 1750. Since the diaphragm displacement signal need not be used when using the acceleration signal to identify the state of the electronic stethoscope 1000, the microcontroller 1700 may also keep the load switch 1715 off at all times in the low-power mode 2601.

On the other hand, the microcontroller 1700 transitions from the low-power mode 2601 to the cardiac sound mode 2602 on the basis of acceleration of the electronic stethoscope 1000 exceeding a threshold acceleration. For example, the microcontroller 1700 may transition from the low-power mode 2601 to the cardiac sound mode 2602 immediately in response to the acceleration of the electronic stethoscope 1000 exceeding the threshold acceleration. The threshold acceleration is set to a value at which the electronic stethoscope 1000 can be detected as having been moved by the user.

The above-described transitions based on the diaphragm displacement signal and the above-described transitions based on the acceleration signal may be combined. For example, the microcontroller 1700 may transition from the low-power mode 2601 to the cardiac sound mode 2602 when one of the condition pertaining to the diaphragm displacement signal and the condition pertaining to the acceleration signal is satisfied. Instead, however, the microcontroller 1700 may transition from the low-power mode 2601 to the cardiac sound mode 2602 when both the condition pertaining to the diaphragm displacement signal and the condition pertaining to the acceleration signal are satisfied.

Furthermore, in the present embodiment, the microcontroller 1700 can perform this transition on the basis of obtaining an instruction from the user to transition from the low-power mode 2601 to the cardiac sound mode 2602. For example, when the power switch 124 is operated by the user, an instruction to transition from the low-power mode 2601 to the cardiac sound mode 2602 is obtained. Alternatively, the operation unit 123 may include a button for obtaining such an instruction from the user. Additionally, the microcontroller 1700 may monitor pairing requests from external devices to the Bluetooth circuit 1703 in the low-power mode 2601. The microcontroller 1700 may then transition from the low-power mode 2601 to the cardiac sound mode 2602 on the basis of receiving a pairing request from an external device.

The transition from the cardiac sound mode 2602 to the low-power mode 2601 will be described in detail next. The transition from the respiratory sound mode 2603 to the low-power mode 2601 is similar to the transition from the cardiac sound mode 2602 to the low-power mode 2601, and thus redundant descriptions thereof will be omitted. In the cardiac sound mode 2602 too, the microcontroller 1700 identifies a state of the electronic stethoscope 1000. The microcontroller 1700 may transition from the cardiac sound mode 2602 to the low-power mode 2601 on the basis of the identified state.

For example, in the cardiac sound mode 2602, the microcontroller 1700 identifies a state of the diaphragm 206 on the basis of the diaphragm displacement signal. The microcontroller 1700 transitions from the cardiac sound mode 2602 to the low-power mode 2601 on the basis of the pressing state of the diaphragm 206 being the unused state described above. For example, the microcontroller 1700 transitions from the cardiac sound mode 2602 to the low-power mode 2601 in response to the pressing state of the diaphragm 206 changing from the in-use state to the unused state. Instead, however, the microcontroller 1700 may transition from the cardiac sound mode 2602 to the low-power mode 2601 in response to the unused state continuing for at least a predetermined threshold time (e.g., 5 seconds).

For example, in the cardiac sound mode 2602, the microcontroller 1700 identifies motion of the electronic stethoscope 1000 on the basis of an acceleration signal output from the accelerometer 1750. The microcontroller 1700 may transition from the cardiac sound mode 2602 to the low-power mode 2601 on the basis of the acceleration of the electronic stethoscope 1000 being less than a threshold acceleration. For example, the microcontroller 1700 may transition from the cardiac sound mode 2602 to the low-power mode 2601 in response to a state where the acceleration of the electronic stethoscope 1000 is lower than the threshold acceleration continuing for at least a predetermined threshold time (e.g., 5 seconds).

The above-described transitions based on the diaphragm displacement signal and the above-described transitions based on the acceleration signal may be combined. For example, the microcontroller 1700 may transition from the cardiac sound mode 2602 to the low-power mode 2601 when one of the condition pertaining to the diaphragm displacement signal and the condition pertaining to the acceleration signal is satisfied. Instead, however, the microcontroller 1700 may transition from the cardiac sound mode 2602 to the low-power mode 2601 when both the condition pertaining to the diaphragm displacement signal and the condition pertaining to the acceleration signal are satisfied.

Furthermore, the microcontroller 1700 can perform the stated transition on the basis of obtaining an instruction from the user to transition from the cardiac sound mode 2602 to the low-power mode 2601. For example, when the power switch 124 is operated by the user, an instruction to transition from the low-power mode 2601 to the cardiac sound mode 2602 may be obtained, and the transition may be made from the cardiac sound mode 2602 to the low-power mode 2601; or the transition from the cardiac sound mode 2602 to the low-power mode 2601 may be made on the basis of an instruction made using a button included in the operation unit 123.

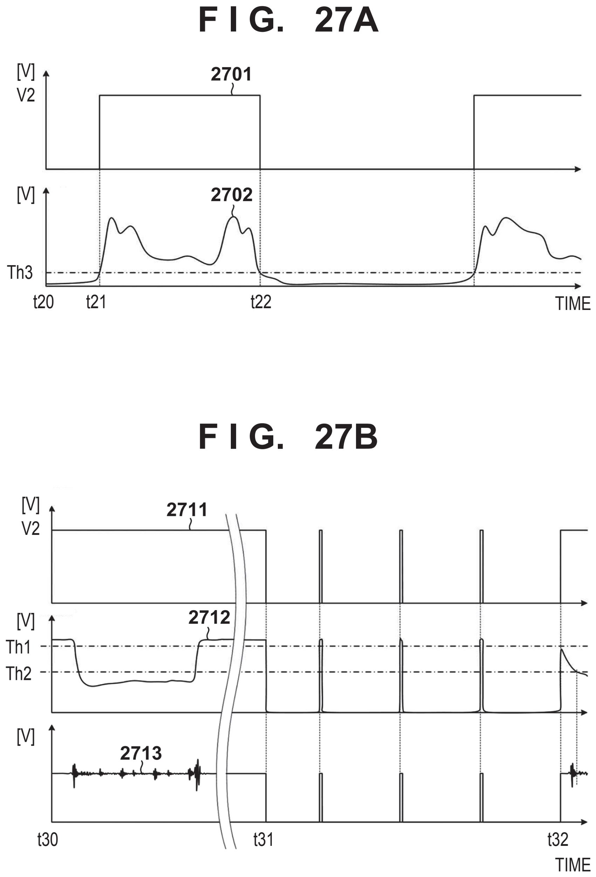

A specific example of an operating mode transition will be described with reference to FIGS. 27A and 27B. FIG. 27A illustrates a transition based on the acceleration signal. A graph 2701 represents the voltage at the output terminal of the voltage regulator 1716. A graph 2702 represents the acceleration signal. At time t20, the electronic stethoscope 1000 is operating in the low-power mode 2601. Accordingly, the voltage at the output terminal of the voltage regulator 1716 is a ground voltage. At time t21, the acceleration signal exceeds a threshold acceleration Th3. In response, the microcontroller 1700 transitions the electronic stethoscope 1000 from the low-power mode 2601 to the cardiac sound mode 2602. The voltage at the output terminal of the voltage regulator 1716 becomes the voltage V2 as a result.

At time t22, the acceleration signal falls below the threshold acceleration Th3. In response, the microcontroller 1700 transitions the electronic stethoscope 1000 from the cardiac sound mode 2602 to the low-power mode 2601. The voltage at the output terminal of the voltage regulator 1716 becomes the ground voltage as a result.

FIG. 27B illustrates a transition based on the diaphragm displacement signal. A graph 2711 illustrates the voltage at the output terminal of the voltage regulator 1716. A graph 2712 represents the diaphragm displacement signal. A graph 2713 represents the cardiac sound signal. Time t30 is a point in time when the electronic stethoscope 1000 is activated and is operating in the cardiac sound mode 2602. At time t30, the voltage at the output terminal of the voltage regulator 1716 is the voltage V2. When the diaphragm displacement signal exceeds the threshold voltage Th1, the microcontroller 1700 sets the timer and measures time. Then, when the time for which the diaphragm displacement signal continuously exceeds the threshold voltage Th1 has passed a predetermined length of time (e.g., 5 seconds), the microcontroller 1700 executes the processing for transitioning to the low-power mode 2601.

On the other hand, if the diaphragm displacement signal is detected to have fallen below the threshold voltage Th1 during the period when the timer is set and the time is being measured, the microcontroller 1700 resets the timer. In the example in FIG. 27B, it is assumed that at time t31, the duration of the state in which the diaphragm displacement signal is greater than the threshold voltage Th1 has reached the predetermined time (e.g., 5 seconds). In response, the microcontroller 1700 transitions the electronic stethoscope 1000 from the cardiac sound mode 2602 to the low-power mode 2601. The voltage at the output terminal of the voltage regulator 1716 becomes the ground voltage as a result.

The present embodiment describes an example in which the operating mode transitions to the low-power mode 2601 when a predetermined length of time passes after the timing at which the diaphragm displacement signal exceeds the threshold voltage Th1. However, as another example, the method may be such that the operating mode transitions to the low-power mode 2601 when the number of times the diaphragm displacement signal exceeds the threshold voltage Th1 exceeds a predetermined number of times. In addition, the condition for transitioning to the low-power mode 2601 in the present embodiment is set to 5 seconds from the timing at which the diaphragm displacement signal exceeds the threshold voltage Th1. However, the time for transitioning to the low-power mode may be set to be changeable by the user according to the usage environment of the electronic stethoscope 1000.

The microcontroller 1700 then turns the load switch 1715 on intermittently. As a result, the voltage at the output terminal of the voltage regulator 1716 becomes V2 intermittently. At time t32, the diaphragm displacement signal falls below the threshold voltage Th1. In response, the microcontroller 1700 transitions the electronic stethoscope 1000 from the low-power mode 2601 to the cardiac sound mode 2602. The voltage at the output terminal of the voltage regulator 1716 stays at the voltage V2 as a result.

As described above, the electronic stethoscope 1000 according to the present embodiment has a plurality of operating modes, including the cardiac sound mode 2602, the respiratory sound mode 2603, and the low-power mode 2601. The microcontroller 1700 then executes processing for transitioning to one of the plurality of operating modes on the basis of (1) the diaphragm displacement signal output from the photodetector 204, (2) the acceleration signal output from the accelerometer 1750, and/or (3) a user input made through the operation unit 123. According to the present embodiment, the electronic stethoscope 1000 transitions to the low-power mode 2601 when in the unused state, which makes it possible to reduce power consumption and reduce battery consumption. As a result, the operating time of the electronic stethoscope 1000 can be extended. In addition, the electronic stethoscope 1000 returns from the low-power mode 2601 when the electronic stethoscope 1000 may be used, which improves the usability for the user.

Note that the conditions for transitioning to the low-power mode 2601 and the conditions for returning from the low-power mode 2601 are not limited to the foregoing (1) to (3), and transitions to and returns from the low-power mode 2601 may be made under other conditions. An example will be described. For example, the microcontroller 1700 may monitor pairing requests from external devices to the Bluetooth circuit 1703 in the low-power mode 2601, and control operating mode transitions on the basis of the result of the monitoring. The microcontroller 1700 transitions from the low-power mode 2601 to the cardiac sound mode 2602 or the respiratory sound mode 2603 upon receiving a pairing request from an external device.

The operating mode can also transition to the low-power mode 2601, return from the low-power mode 2601, or the like based only on a user input made through the operation unit 123. In this case, the load switch 1715 does not need to be turned on intermittently while in the low-power mode 2601, and thus a higher power consumption effect can be achieved. The operating mode may also transition to the low-power mode 2601, return from the low-power mode 2601, or the like in response to the power switch 124 being pressed for a long time.

Note that the user may be notified of the transition of the operating mode when transitioning from the cardiac sound mode 2603 or the respiratory sound mode 2603 to the low-power mode 2601, when returning from the low-power mode 2601 to the cardiac sound mode 2603 or the respiratory sound mode 2603, and the like. Specifically, the display control unit 1802 controls the light-emitting unit 2301 to cause the light-emitting unit 2301 to flash for a set period of time (e.g., for several seconds). This flashing processing notifies the user of the transition of the operating mode.

Third Embodiment

Example Configuration of Electronic Auscultation Apparatus of Third Embodiment

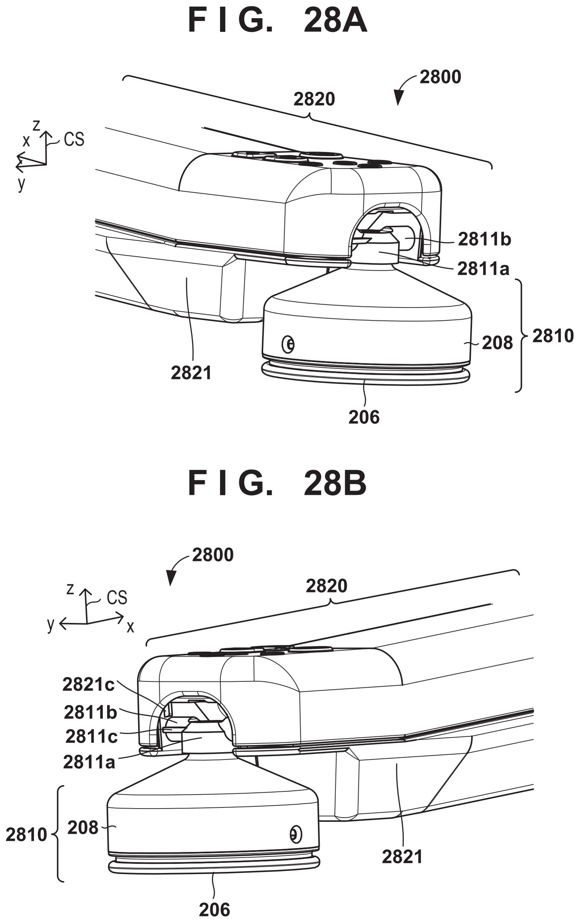

An example configuration of an electronic stethoscope 2800 according to a third embodiment will be described with reference to FIGS. 28A to 30. The electronic stethoscope described above includes a chest piece, which is a part applied to the skin of a patient, and a grip having relatively heavy components such as a battery. If the grip held by the user is fixed to the chest piece, it may be difficult to apply the chest piece evenly to the skin or the like of the patient depending on the posture of the patient (laying down, sitting, standing, or the like). In addition, depending on the angle of the chest piece and the grip, the electronic stethoscope may take up space or be difficult to store when not in use. Accordingly, the electronic stethoscope 2800 according to the third embodiment has one characteristic in that a chest piece 2810 is coupled to a grip 2820 so as to be capable of pivoting relative to the grip 2820. The following will primarily describe the differences from the electronic stethoscope 1000 according to the second embodiment. The differences between the second embodiment and the third embodiment may be applied to the first embodiment. The external appearance of the electronic stethoscope 2800 is similar to that of the electronic stethoscope 100 described with reference to FIGS. 1A and 1B. The variations described in the first embodiment or the second embodiment may also be applied to the third embodiment. Specifically, the displacement detection unit 1011 and the vibration detection unit 1012 according to the second embodiment may be provided within the chest piece 2810 of the electronic stethoscope 2800 according to the third embodiment. In such a case, the relay board 1103 and the hole 208a may be disposed within the chest piece 2810 and outside the sealed space 1100. This makes it possible to pivot the chest piece 2810 without compromising the sound detection sensitivity of the microphone.

The electronic stethoscope 2800 includes the chest piece 2810 and the grip 2820. Aside from the differences described below, the chest piece 2810 is the same as the chest piece 1010 of the electronic stethoscope 1000, and the grip 2820 is the same as the grip 120 of the electronic stethoscope 1000.

External Appearance of Electronic Auscultation Apparatus of Third Embodiment

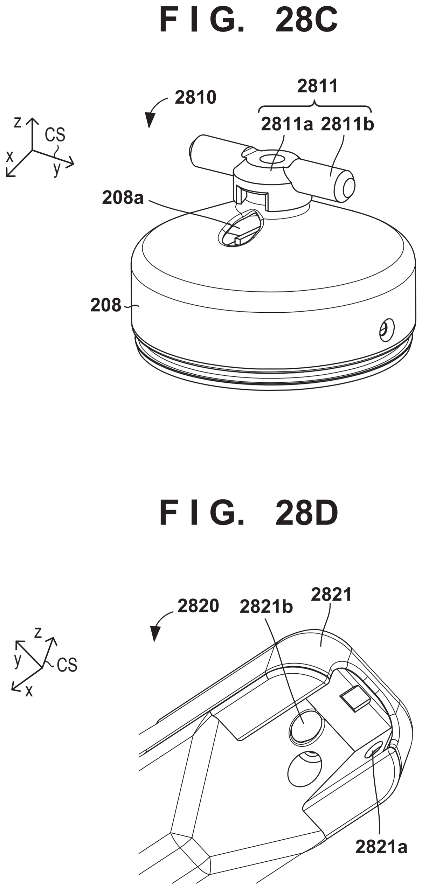

FIGS. 28A and 28B are perspective views of the part of the electronic stethoscope 2800 including the chest piece 2810 seen from different angles. FIG. 28C is a perspective view of the chest piece 2810. FIG. 28D is a perspective view of the grip 2820.

The chest piece 2810 is coupled to the grip 2820 so as to be capable of pivoting relative to the grip 2820. An example of a specific configuration for enabling such coupling will be described hereinafter. The chest piece 2810 may be capable of pivoting relative to the grip 2820 through a configuration different from that described below. Making the chest piece 2810 capable of pivoting relative to the grip 2820 makes it easier for the contact surface 206a of the diaphragm 206 to come into close contact with the surface of the target body when the electronic stethoscope 2800 is used, the electronic stethoscope 2800 can accurately detect displacement in the biological surface.

The chest piece 2810 includes a coupling member 2811 attached substantially in the center of the upper surface of the housing 208. The coupling member 2811 is provided on a projection formed substantially in the center of the upper surface of the housing 208. The coupling member 2811 may be formed from a metal that is the same material as the housing 208, or may be formed from a different material (e.g., a resin). The coupling member 2811 may also be part of the housing 208, and the coupling member 2811 and the housing 208 may be formed as an integral entity.

The coupling member 2811 includes a central part 2811a and a rotational shaft 2811b. The rotational shaft 2811b extends outward from the central part 2811a. The rotational shaft 2811b has two end parts located on opposite sides, and the central part 2811a is located between the two end parts. The rotational shaft 2811b extends substantially parallel to the contact surface 206a of the diaphragm 206. The rotational shaft 2811b has a cylindrical surface. Specifically, the cross-section of the rotational shaft 2811b along the xz plane is circular. In the present specification, a straight line and a plane being "substantially parallel" means that the angle formed by the straight line and the plane is at least 0° and at most 10°. Additionally, two straight lines being "substantially parallel" means that the angle formed by the two straight lines is at least 0° and at most 10°. A straight line and a plane being "substantially orthogonal" means that the angle formed by the straight line and the plane is at least 80° and at most 90°. Additionally, two straight lines being "substantially orthogonal" means that the angle formed by the two straight lines is at least 80° and at most 90°. Here, the "angle formed" refers to an angle between the two that falls within a range of at least 0° and at most 90°.

The grip 2820 has a housing 2821, formed from a resin, that houses a battery 2911 and a main circuit board 2910 (described later). The grip 2820 has a rod shape, and the chest piece 2810 is coupled to one end thereof. The direction in which the grip 2820 extends will be referred to as the "longitudinal direction" of the grip 2820. For example, the longitudinal direction of the grip 2820 is the direction in which the longest line segment in the outer surface of the housing 2821 of the grip 2820 extends. The longitudinal direction of the grip 2820 is also the direction in which the long side of a circuit board (described later) housed in the housing 2821 extends. The housing 2821 is of a size that can be gripped by the user. Specifically, the length of the housing 2821 (e.g., the size of the grip 2820 in the longitudinal direction) is within a range of 50 mm to 150 mm, for example. The length of the periphery of the housing 2821 about the longitudinal direction is within a range of 50 mm to 200 mm, for example.

As illustrated in FIG. 28D, the housing 2821 includes two receiving parts 2821a. One receiving part 2821a engages with one end part of the rotational shaft 2811b, and the other receiving part 2821a engages with the other end part of the rotational shaft 2811b. Through this, the chest piece 2810 is coupled to the grip 2820 so as to be capable of pivoting relative to the grip 2820. Specifically, in such a configuration, the chest piece 2810 rotates relative to the grip 2820 about the rotational shaft 2811b along a plane orthogonal to the direction in which the rotational shaft 2811b extends (the xz plane). As used herein, "the chest piece 2810 being capable of pivoting relative to the grip 2820" means that the angle of the contact surface 206a of the diaphragm 206 relative to the longitudinal direction of the grip 2820 can be changed. The chest piece 2810 may be capable of pivoting relative to the grip 2820 in a different manner instead. For example, the chest piece 2810 may be capable of pivoting relative to the grip 2820 without having a specific pivoting axis, e.g., by sliding on a rail including a curved part.

Each receiving part 2821a has a recess or hole into which the rotational shaft 2811b is inserted, and supports the rotational shaft 2811b so as to be capable of rotating. The two receiving parts 2821a are provided at opposing positions with a space therebetween, and the central part 2811a of the coupling member 2811 is disposed in this space. The chest piece 2810 is coupled to the grip 2820 such that the rotational shaft 2811b overlaps with the diaphragm 206 when the contact surface 206a of the diaphragm 206 is viewed in plan view. As a result, the user can bring the contact surface 206a of the diaphragm 206 into close contact with the surface of the target body while gripping the grip 2820 naturally.

Cross-Sectional Configuration of Electronic Auscultation Apparatus of Third Embodiment

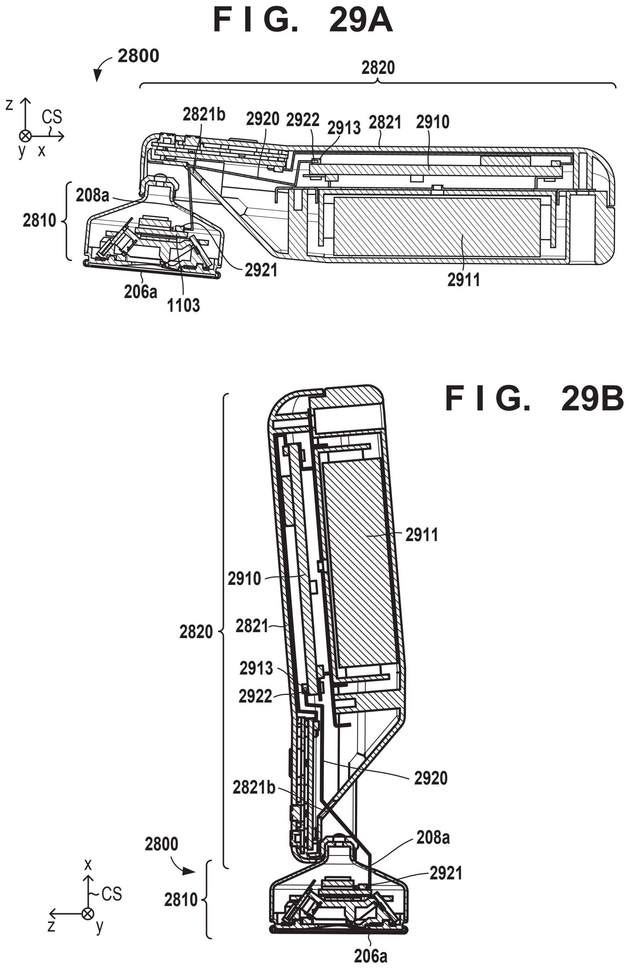

A cross-sectional view of the electronic stethoscope 2800 in a plane parallel to the xz plane and passing through the central part 2811a of the coupling member 2811 will be described with reference to FIGS. 29A to 29D. FIG. 29A illustrates a case where the chest piece 2810 is located at one end of the range the chest piece 2810 is capable of pivoting relative to the grip 2820, and FIG. 29B illustrates a case where the chest piece 2810 is located at the other end of the range the chest piece 2810 is capable of pivoting relative to the grip 2820. In other words, the chest piece 2810 can pivot (and specifically, can rotate) relative to the grip 2820 between the position illustrated in FIG. 29A and the position illustrated in FIG. 29B.

In the following descriptions, the position illustrated in FIG. 29A will be referred to as a "home position" of the chest piece 2810, and the position illustrated in FIG. 29B will be referred to as an "inverted position" of the chest piece 2810. The home position may be a position at which the chest piece 2810 stops when the chest piece 2810 is pivoted about the rotational shaft 2811b in what is the counterclockwise direction in FIGS. 29A to 29D. The inverted position is a position at which the chest piece 2810 stops when the chest piece 2810 is pivoted about the rotational shaft 2811b in what is the clockwise direction in FIGS. 29A to 29D. For example, it is desirable that the chest piece 2810 be capable of pivoting at least 60 degrees relative to the grip 2820. For example, an upper limit of the range over which the chest piece 2810 can pivot relative to the grip 2820 may be 80°, 90°, or 100°.

Preferably, when the chest piece 2810 is in the home position (FIG. 29A), the angle formed between the longitudinal direction of the grip 2820 (e.g., the x-axis direction) and the contact surface 206a of the diaphragm 206 is at least 0° at most 45°. Furthermore, this angle may be 30° or less, or 15° or less. When the chest piece 2810 is in the inverted position (FIG. 29B), the angle formed between the longitudinal direction of the grip 2820 (e.g., the x-axis direction) and the contact surface 206a of the diaphragm 206 may be at least 80° at most 90°. Furthermore, this angle may be at least 70°, or at least 60°.

The grip 2820 includes the main circuit board 2910 and the battery 2911. The main circuit board 2910 includes circuit elements for controlling the operations of the electronic stethoscope 2800 as a whole (e.g., integrated circuits, electrode pads, conductive patterns, and the like). Specifically, the main circuit board 2910 controls the operations of the chest piece 2810, the operations of the display device 122, and the operations of the operation unit 123. For example, the main circuit board 2910 constitutes the audio output unit 1020 described above (FIG. 10). The main circuit board 2910 may have a board shape, and may have a mounting surface parallel to the xy plane. As described above, the longitudinal direction of the main circuit board 2910 (e.g., the direction in which the long side of the mounting surface extends) may be taken as the longitudinal direction of the grip 2820.

The battery 2911 stores power used by the electronic stethoscope 2800. The battery 2911 in the present embodiment has a plate or columnar shape, and the longitudinal direction of the battery 2911 matches the longitudinal direction of the grip 2820.

The electronic stethoscope 2800 includes a wire harness 2920 connecting the relay board 1103 of the chest piece 2810 with the main circuit board 2910 of the grip 2820. Specifically, the wire harness 2920 includes a connector 2922 for connecting to a connector 2913 provided on the main circuit board 2910 of the grip 2820, and a connector 2921 for connecting to the relay board 1103 (and specifically, a connector thereof) of the chest piece 2810. The connector 2921 and the connector 2922 are located at opposite end parts of the wire harness 2920.

The hole 208a through the wire harness 2920 is passed is formed in the chest piece 2810 as described above. A hole 2821b through which the wire harness 2920 is passed is also formed in the grip 2820 (and specifically, in the housing 2821 thereof). The hole 208a is formed at a position overlapping the grip 2820 when the contact surface 206a of the diaphragm 206 is viewed in plan view, when the chest piece 2810 is in the home position. Through this, the wire harness 2920 passing through the hole 208a is hidden by the grip 2820, which reduces the risk of the wire harness 2920 being damaged due to factors outside the electronic stethoscope 2800. As illustrated in FIG. 28C, the hole 208a is formed in a different position than the coupling member 2811. However, the hole 208a may be in a position at which the hole 208a penetrates the coupling member 2811. The hole 2821b is formed in a surface that is opposite the chest piece 2810 when the chest piece 2810 is in the home position. This makes it possible to shorten the length of the wire harness 2920. This also reduces the risk of the wire harness 2920 being damaged due to factors outside the electronic stethoscope 2800. The hole 2821b need not be formed in the grip 2820, and in this case, the grip 2820 may have a connector that connects the inside and the outside of the housing 2821. The part of the connector within the housing 2821 may be connected to the main circuit board 2910, and the part of the connector outside the housing 2821 may be connected to the wire harness 2920. The same applies to the chest piece 2810, and a connector may be formed instead of the hole 208a.

Wire Bundle of Electronic Auscultation Apparatus of Third Embodiment

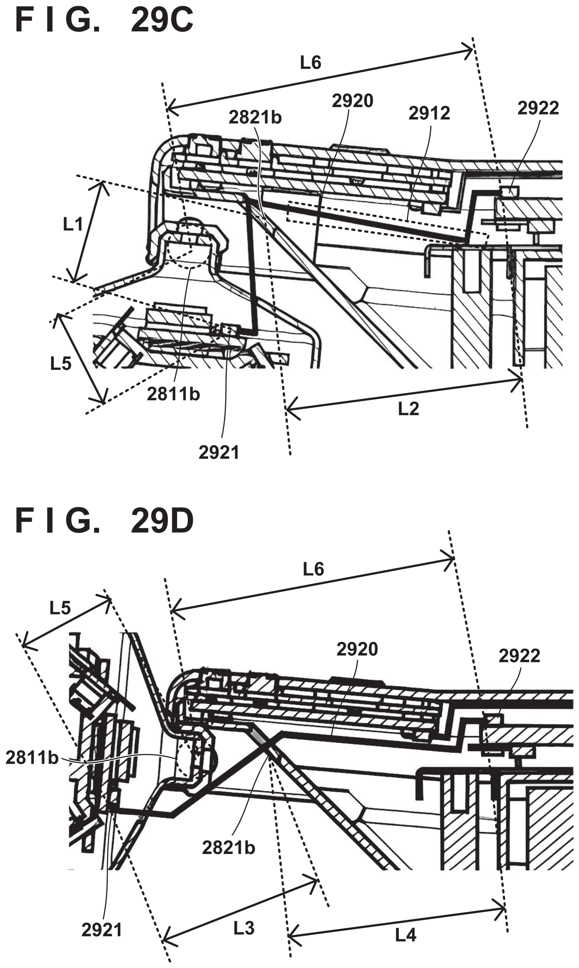

The wire harness 2920 will be described next in more detail with reference to FIGS. 29C and 29D. FIG. 29C is a diagram illustrating the part of FIG. 29A that includes the wire harness 2920. FIG. 29D is a diagram illustrating the part of FIG. 29B that includes the wire harness 2920.

The sum of (i) the distance between the connector 2921 of the chest piece 2810 and the hole 2821b of the grip 2820 and (ii) the distance between the connector 2922 of the grip 2820 and the hole 2821b of the grip 2820 will be described. The distance between the connector and the hole may be a distance between a center of the connector and a center of the hole, a distance between a part of the connector closest to the hole and a part of the hole closest to the connector, or a distance measured in a different manner.

The sum of these distances varies as the chest piece 2810 pivots relative to the grip 2820. Because the wire harness 2920 passes through the hole 2821b of the grip 2820, the length of the wire harness 2920 is greater than the maximum value of the sum of these distances in the range over which the chest piece 2810 can pivot.

Specifically, as illustrated in FIG. 29C, when the chest piece 2810 is in the home position, the distance between the connector 2921 of the chest piece 2810 and the hole 2821b of the grip 2820 is represented by L1, and the distance between the connector 2922 of the grip 2820 and the hole 2821b of the grip 2820 is represented by L2. As illustrated in FIG. 29D, when the chest piece 2810 is in the inverted position, the distance between the connector 2921 of the chest piece 2810 and the hole 2821b of the grip 2820 is represented by L3, and the distance between the connector 2922 of the grip 2820 and the hole 2821b of the grip 2820 is represented by L4. The distance between the connector 2921 of the chest piece 2810 and the center of the rotational shaft 2811b is represented by L5, and the distance between the connector 2922 of the grip 2820 and the center of the rotational shaft 2811b is represented by L6. L5 and L6 are constant regardless of the position of the chest piece 2810 relative to the grip 2820.

The stated sum of the distances increases as the connector 2921 of the chest piece 2810 moves away from the hole 2821b of the grip 2820. Accordingly, the relationship L3 + L4 > L1 + L2 is established. When the chest piece 2810 is in the inverted position, the stated sum of the distances may be at a maximum. Accordingly, the length of the wire harness 2920 is greater than L3 + L4. In the present embodiment, L3 > L1 > L5 is satisfied, and L6 > L2 = L4 is satisfied.

When the chest piece 2810 is pivoted such that the stated sum of the distances decreases (e.g., when the chest piece 2810 is pivoted from the inverted position toward the home position), the distance needed for the wire harness 2920 decreases, and part of the wire harness 2920 bends. The housing 2821 of the grip 2820 is provided with a containment part housing having a space 2912 that contains the bent part of the wire harness 2920. When the chest piece 2810 is in the inverted position, the wire harness 2920 is not contained in this space 2912, and when the chest piece 2810 is in the home position, a part of the wire harness 2920 is contained in this space 2912. By providing the housing 2821 of the grip 2820 with this space 2912, situations where the wire harness 2920 bends outside the housing 2821 are suppressed. This reduces the risk of the wire harness 2920 being pinched and cut by the chest piece 2810 and the grip 2820.

The electronic stethoscope 2800 of the present embodiment includes a holding mechanism for holding the chest piece 2810 in the inverted position. For example, the holding mechanism may be constituted by a projection 2811c (FIG. 28B) provided on the rotational shaft 2811b of the coupling member 2811, and a rotation stopper 2821c (FIG. 28B) provided in the receiving part 2821a of the grip 2820.

Details of Holding Mechanism of Chest Piece of Electronic Auscultation Apparatus of Third Embodiment

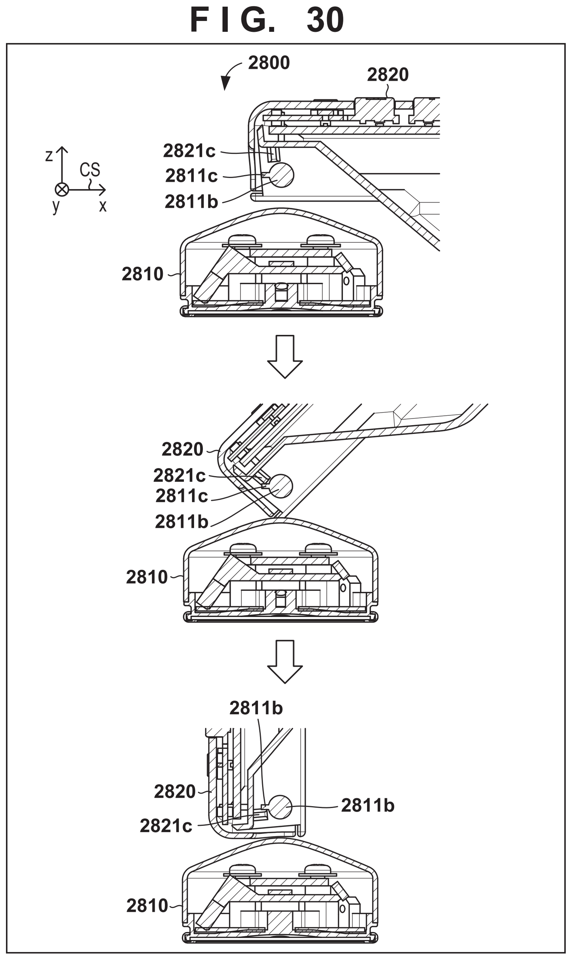

The holding mechanism for holding the chest piece 2810 in the inverted position will be described in further detail with reference to FIG. 30. Each section of FIG. 30 is part of a cross-sectional view of the electronic stethoscope 2800 in a plane parallel to the xz plane.

In the top section of FIG. 30, the chest piece 2810 is in the home position. As the chest piece 2810 pivots toward the inverted position relative to the grip 2820, the projection 2811c approaches the rotation stopper 2821c, as illustrated in the middle section of FIG. 30. The projection 2811c and the rotation stopper 2821c are configured such that the projection 2811c passes over the rotation stopper 2821c when a torque of at least a threshold is applied to the chest piece 2810. For example, this torque threshold may be a value greater than the torque produced by the weight of the chest piece 2810, e.g., about 0.5 [N/m]. When the projection 2811c passes over the rotation stopper 2821c, the chest piece 2810 is in the inverted position.

When the chest piece 2810 is in the inverted position, the chest piece 2810 is held in the inverted position by the projection 2811c and the rotation stopper 2821c unless a torque of at least the threshold acts on the chest piece 2810. When the contact surface 206a of the diaphragm 206 is placed on the horizontal surface (e.g., a table top) with the chest piece 2810 held in the inverted position, the electronic stethoscope 2800 stands on its own. As a result, when the user temporarily stops using the electronic stethoscope 2800, the electronic stethoscope 2800 can be placed on a table with the contact surface 206a of the diaphragm 206 protected (e.g., by the table top). Holding the chest piece 2810 in the inverted position also makes it easier to store the electronic stethoscope 2800 after use.

Variation on Electronic Auscultation Apparatus of Third Embodiment

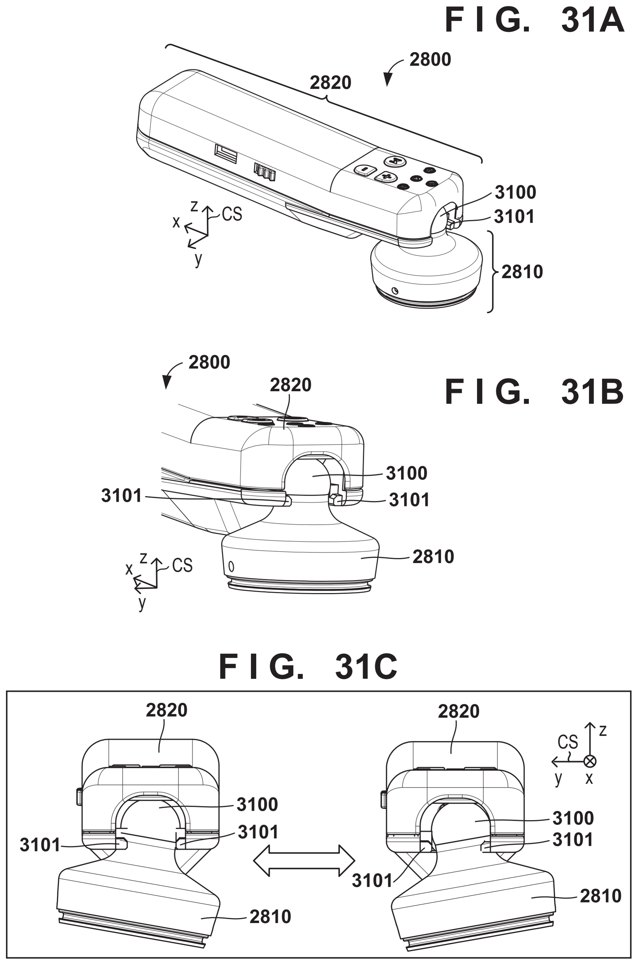

A variation on the electronic stethoscope 2800 will be described with reference to FIGS. 31A to 31C. FIG. 31A is a perspective view of the electronic stethoscope 2800 according to the variation, and FIGS. 31B and 31C are perspective views of parts of the electronic stethoscope 2800 from other angles.

In this variation, the chest piece 2810 has a projection 3100 instead of the rotational shaft 2811b, and the grip 2820 has receiving parts 3101 instead of the receiving part 2821a. The projection 3100 has a spherical surface. Specifically, the housing 2821 of the grip 2820 has two receiving parts 3101. One of the receiving parts 3101 supports a part of the spherical surface of the projection 3100, while the other of the receiving parts 3101 supports another part of the spherical surface of the projection 3100. As a result, the two receiving parts 3101 uniformly contact and engage with the spherical surface of the projection 3100.

The chest piece 2810 can be rotated in multiple axial directions relative to the grip 2820 by the spherical surface of the projection 3100 sliding in the receiving parts 3101. Specifically, the chest piece 2810 can be rotated along the xz plane in the same manner as the electronic stethoscope 2800 illustrated in FIGS. 28A to 28D, and can also be rotated along the yz plane as illustrated in FIG. 31C. Having such a configuration makes it easier for the contact surface 206a of the diaphragm 206 to come into closer contact with the surface of the target body when the electronic stethoscope 2800 is used.

Fourth Embodiment

Example Configuration of Electronic Auscultation Apparatus of Fourth Embodiment

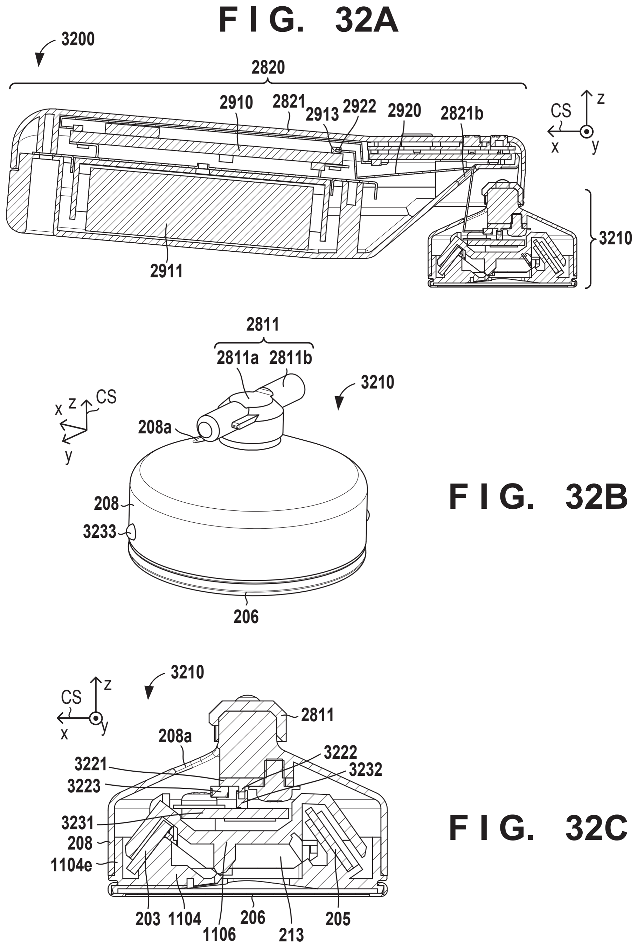

An example of the configuration of an electronic stethoscope 3200 according to a fourth embodiment will be described with reference to FIGS. 32A to 33D. The electronic stethoscope may need to be replaced due to deterioration of the diaphragm over time, wear on the contact surface, and the like. However, if only the diaphragm is the part to be replaced, there is a risk that the user will touch the photosensor during the replacement, the positional relationship between the diaphragm and the photosensor will change, and the like. In such a case, the measurement accuracy is affected by the characteristics of the diaphragm, the precision of the components to which the photosensor is attached, the optical properties, and the like. Accordingly, one of the characteristics of the electronic stethoscope according to the fourth embodiment is that the electronic stethoscope includes a base unit and a replacement unit, as well as an attachment/removal mechanism that enables the replacement unit, which includes the diaphragm, to be attached to and removed from the base unit easily. The following will primarily describe the differences from the electronic stethoscope 2800 according to the third embodiment, and will omit descriptions of points that are the same as those of the electronic stethoscope 2800. The differences between the third embodiment and the fourth embodiment can be applied to the first embodiment or the second embodiment. The external appearance of the electronic stethoscope 3200 is similar to that of the electronic stethoscope 100 described with reference to FIGS. 1A and 1B. The variations described in the first embodiment to the third embodiment can also be applied to the fourth embodiment.

The electronic stethoscope 3200 includes a chest piece 3210 and the grip 2820. Aside from the differences described below, the chest piece 3210 is the same as the chest piece 2810 of the electronic stethoscope 2800, and the grip 2820 is the same as the grip 2820 of the electronic stethoscope 2800.

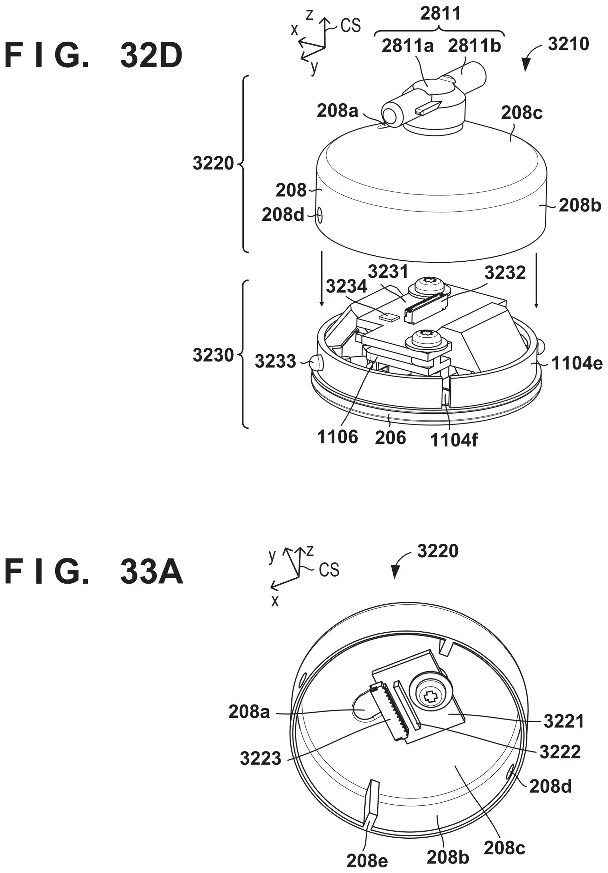

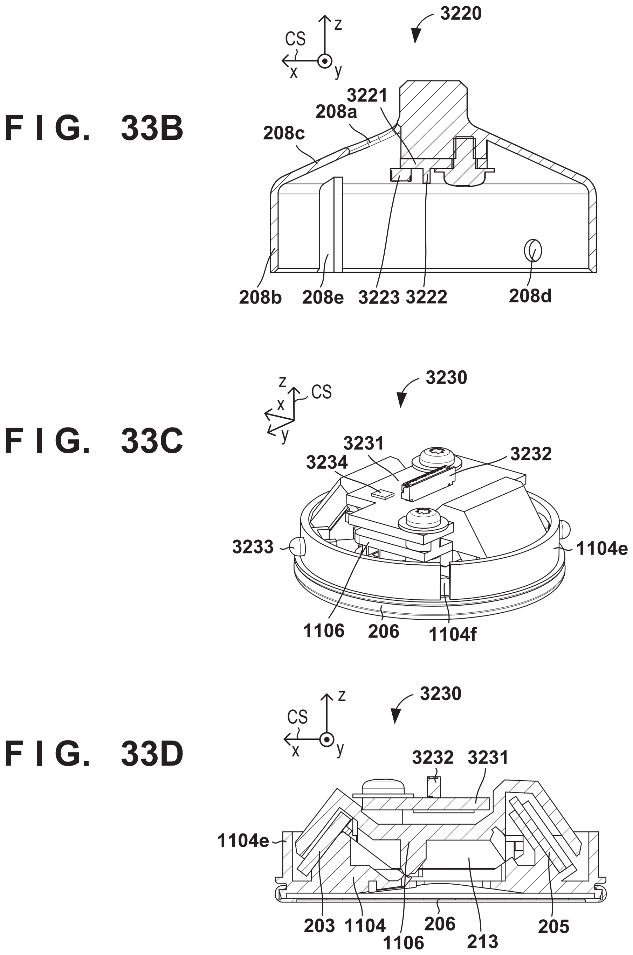

FIG. 32A is a cross-sectional view of the electronic stethoscope 2800 in a plane parallel to the xz plane. FIG. 32B is a perspective view of the chest piece 3210. FIG. 32C is a diagram focusing on the chest piece 3210 in the cross-sectional view in FIG. 32A. FIG. 32D is a schematic diagram illustrating a user operation for removing a replacement unit 3230 from a base unit 3220. FIG. 33A is a perspective view of a part of the base unit 3220 (the part included in the chest piece 3210). FIG. 33B is a diagram focusing on a part of the base unit 3220 (the part included in the chest piece 3210) in the cross-sectional view in FIG. 32A. FIG. 33C is a perspective view of the replacement unit 3230. FIG. 33D is a diagram focusing on the replacement unit 3230 in the cross-sectional view in FIG. 32A.

As illustrated in FIG. 32D, the electronic stethoscope 3200 includes the base unit 3220 and the replacement unit 3230. The base unit 3220 is the part of the electronic stethoscope 3200 that is not expected to be replaced through a user operation during the life of the product. The base unit 3220 includes the grip 2820 and a part of the chest piece 3210. However, the constituent elements of the base unit 3220 are not necessarily expected to be replaced, and some of the constituent elements of the base unit 3220 can be replaced through factory repair or the like. The battery 2911 of the grip 2820 can also be replaced by the user.

The replacement unit 3230 is the part of the electronic stethoscope 3200 that is expected to be replaced through a user operation during the life of the product. In the electronic stethoscope 3200, the replacement unit 3230 is coupled to the base unit 3220 such that the replacement unit 3230 can be removed through a user operation. The electronic stethoscope 3200 has an attachment/removal mechanism that couples the replacement unit 3230 to the base unit 3220 so as to be removable through a user operation. Hereinafter, this attachment/removal mechanism will be described as an "attachment/removal mechanism of the electronic stethoscope 3200", or simply an "attachment/removal mechanism".

The chest piece 3210 differs from the chest piece 2810 of the third embodiment in that the chest piece 3210 includes a relay board 3231, a connector 3232, a lock pin 3233, a relay board 3221, a connector 3222, and a connector 3223.

The relay board 3231 is coupled to the holding member 201 (e.g., the upper holding member 1106 thereof). In the electronic stethoscope 3200, this coupling is performed using a fastener such as a screw.

The relay board 3231 is connected to the light-emitting circuit board 203 by a lead line (not shown). The relay board 3231 transmits a control signal for instructing light to be emitted to the light-emitting circuit board 203, and supplies power, over the lead line. The relay board 3231 is also connected to the light-receiving circuit board 205 by a lead line (not shown). The relay board 3231 receives the displacement signal from the light-receiving circuit board 205, and supplies power to the light-receiving circuit board 205, over the lead line. The relay board 3231 is also connected to the condenser microphone 1101 by a lead line (not shown). The relay board 3231 receives the sound signal from the condenser microphone 1101, and supplies power to the condenser microphone 1101, over the lead line.

The relay board 3221 is coupled to an inner surface of the housing 208. In the electronic stethoscope 3200, this coupling is performed using a screw.

The connector 3232 is mounted to the upper surface of the relay board 3231, and the connector 3222 and the connector 3223 are mounted to the lower surface of the relay board 3221. The connector 3222 and the connector 3223 are electrically connected to each other by a conductive pattern formed in the relay board 3221. The connector 2921 of the wire harness 2920 extending through the hole 208a of the housing 208 is connected to the connector 3223 of the relay board 3221. The connector 3222 of the relay board 3221 is connected to the connector 3232 of the relay board 3231. As a result, signals from the relay board 3231 are transmitted to the main circuit board 2910 through the connector 3232, the connector 3222, the connector 3223, and the wire harness 2920. Additionally, power from the main circuit board 2910 is also supplied to the relay board 3231 through the connector 3232, the connector 3222, the connector 3223, and the wire harness 2920.

Among the constituent elements of the chest piece 3210, the holding member 201, the light emitter 202, the light-emitting circuit board 203, the photodetector 204, the light-receiving circuit board 205, the diaphragm 206, the reflective medium 207, the relay board 3231, and the connector 3232 are included in the replacement unit 3230. Among the constituent elements of the chest piece 3210, the housing 208, the coupling member 2811, the relay board 3221, the connector 3222, and the connector 3223 are included in the base unit 3220. Accordingly, in the electronic stethoscope 3200, it is not only the diaphragm 206 that is replaced, but the constituent elements constituting the displacement detection unit 1011 (i.e., the light emitter 202, the photodetector 204, and the reflective medium 207) are also replaced along with the diaphragm 206.

In the present embodiment, the reflective medium 207 is attached to the diaphragm 206. Accordingly, if the diaphragm 206 is removed from the holding member 201 and replaced with a different diaphragm 206 through a user operation, the positional relationship between the light emitter 202 and the reflective medium 207, or the positional relationship between the photodetector 204 and the reflective medium 207, may change. If these positional relationships change, there is a risk that the displacement of the surface of the target body cannot be accurately detected. Accordingly, in the present embodiment, including the constituent elements constituting the displacement detection unit 1011 and the diaphragm 206 in the replacement unit 3230 makes it possible to replace the diaphragm 206 without reducing the accuracy with which the surface of the target body is detected.

As described above, the holding member 201 holds the light emitter 202, the photodetector 204, and the diaphragm 206 in a predetermined positional relationship. The replacement unit 3230 is removable from the base unit 3220 through a user operation while the holding member 201 holds the light emitter 202, the photodetector 204, and the diaphragm 206 in this positional relationship. This suppresses a drop in the accuracy of at which displacement in the surface of a target body is detected due to a shift in these positional relationships. Furthermore, the replacement unit 3230 can be removed from the base unit 3220 while keeping the internal space 213 facing the inner surface 206b of the diaphragm 206. In other words, the user can be prevented from touching the reflective medium 207, the light emitter 202, and the photodetector when the unit is replaced. This suppresses a drop in the accuracy with which displacement in the surface of the target body is detected due to the reflective medium 207, the light emitter 202, the photodetector 204, and the like being soiled as a result of user operations.

In the embodiments described above, the connector 2913 of the main circuit board 2910 included in the grip 2820 and the connector 3223 of the relay board 3221 coupled to the housing 208 are electrically connected by the wire harness 2920. Additionally, the connector 3222 of the relay board 3221 included in the base unit 3220 and the relay board 3231 included in the replacement unit 3230 are electrically connected by coupling the base unit 3220 with the replacement unit 3230 through the attachment/removal mechanism. Using such a configuration makes it possible to attach and remove the replacement unit 3230 including the displacement detection unit 1011 and the diaphragm 206, while implementing a configuration in which the chest piece 3210 pivots relative to the grip 2820. In other words, the electronic stethoscope 3200 achieves both an improvement in the user's operability and easier replacement of the replacement unit 3230.

If the chest piece 3210 includes the condenser microphone 1101 and the sealing member 1102 as in the third embodiment, these constituent elements are also included in the replacement unit 3230. This enables the replacement unit 3230 to be replaced through a user operation while keeping the internal space 213 in the chest piece 3210 a sealed space.

The shapes of the holding member 201 and the housing 208 will be described in further detail with reference to FIG. 32C. The holding member 201 includes a side wall 1104e that extends along the outer periphery of the diaphragm 206. In the electronic stethoscope 3200 of the present embodiment, the base holding member 1104 has the side wall 1104e.

As illustrated in FIG. 32D, the housing 208 has a cylindrical part 208b and a top plate part 208c. The top plate part 208c is a curved surface-shaped part integrally formed with the cylindrical part 208b, connected to an upper end of the cylindrical part 208b across the entire circumference thereof. A central part of the top plate part 208c protrudes upward, and the coupling member 2811 is coupled to the protruding part. The coupling member 2811 is coupled to the housing 208 in a manner that does not anticipate removal through a user operation. The housing 208 is coupled to the grip 2820 via the coupling member 2811 so as to be capable of pivoting.

When the replacement unit 3230 is coupled to the base unit 3220, the cylindrical part 208b is located on an outer side of the side wall 1104e, and surrounds the periphery of the side wall 1104e. The outer diameter of the side wall 1104e and the inner diameter of the cylindrical part 208b are approximately equal. A lower end of the cylindrical part 208b of the housing 208 contacts the holding member 201. When the housing 208 is coupled to the holding member 201, the housing 208 covers the constituent elements held by the holding member 201, i.e., the light-emitting circuit board 203 on which the light emitter 202 is formed, the light-receiving circuit board 205 on which the photodetector 204 is formed, and the relay board 3231 on which the connector 3232 is mounted.

Attachment/Removal Mechanism of Electronic Auscultation Apparatus of Fourth Embodiment

The attachment/removal mechanism of the electronic stethoscope 3200 will be described next with reference to FIGS. 32A to 32D. The attachment/removal mechanism of the electronic stethoscope 3200 is constituted by the lock pin 3233 and a hole 208d formed in the housing 208. A hole for passing the lock pin 3233 through is formed in the side wall 1104e. The side wall 1104e supports the lock pin 3233 such that the lock pin 3233 can move in the longitudinal direction of the lock pin 3233.

When the replacement unit 3230 is coupled to the base unit 3220, the lock pin 3233 passes through both the hole in the side wall 1104e and the hole 208d in the housing 208, and the tip of the lock pin 3233 protrudes from the housing 208. The lock pin 3233 is biased toward the outer side of the chest piece 3210 by a biasing member such as a spring. As such, unless an external force is applied, the tip of the lock pin 3233 remains protruding from the housing 208. The base side of the lock pin 3233 is thicker than the hole in the side wall 1104e such that the lock pin 3233 does not fall out from the chest piece 3210.

The lock pin 3233 has a spherical part constituting the tip and a cylindrical part extending from the spherical part. When no external force is applied to the lock pin 3233, the cylindrical part of the lock pin 3233 is located within the hole 208d in the side wall 1104e. As a result, unless an external force is applied to the lock pin 3233, the range over which the replacement unit 3230 can move relative to the base unit 3220 is limited to the gap between the lock pin 3233 and the housing 208. In other words, movement of the replacement unit 3230 relative to the base unit 3220 is locked by the lock pin 3233.

On the other hand, when the lock pin 3233 is pushed inward by a user operation, the spherical part of the lock pin 3233 is located within the hole 208d in the side wall 1104e. In this state, when the replacement unit 3230 is pulled upward in the z-axis direction, away from the base unit 3220, the side wall 1104e pushes the lock pin 3233 further inward, which enables the replacement unit 3230 to move for the purpose of removal from the base unit 3220. In other words, the lock pin 3233 unlocks the movement of the replacement unit 3230 relative to the base unit 3220.

As illustrated in FIGS. 33A and 33B, the electronic stethoscope 3200 further includes a regulating mechanism that regulates a movement path of the replacement unit 3230 relative to the base unit 3220. In the electronic stethoscope 3200, the regulating mechanism is constituted by a projection 208e provided on the inner surface of the cylindrical part 208b of the housing 208 and a slit 1104f provided in the side wall 1104e of the holding member 201. The projection 208e and the slit 1104f regulate the movement path of the replacement unit 3230 relative to the base unit 3220 to movement in the normal direction of the contact surface 206a of the diaphragm 206. The connector 3222 and the connector 3232 are coupled at the correct angle as a result. Although the projection 208e is a rectangular cuboid in the example illustrated in FIG. 33A, another shape that can pass through the slit 1104f, such as a cube, may be used instead. Although the slit 1104f penetrates the side wall 1104e in the example illustrated in FIG. 33C, the slit 1104f may be configured as a recess formed in the side wall 1104e.

User operations for attaching and removing the replacement unit 3230 to and from the base unit 3220 will be described with reference to FIG. 32D. To remove the replacement unit 3230 from the base unit 3220, the user moves the replacement unit 3230 in a direction away from the base unit 3220 in the z-axis direction, in a state where the lock pin 3233 is pressed. As described above, when the lock pin 3233 is pushed in, the movement of the replacement unit 3230 relative to the base unit 3220 is unlocked. In this state, the replacement unit 3230 is removed from the base unit 3220 by moving the replacement unit 3230 relative to the base unit 3220 such that the projection 208e moves along the slit 1104f. Through this user operation, the connector 3232 on the replacement unit 3230 side (see FIGS. 32C, 33A, and 33B) is disconnected from the connector 3222 on the base unit 3220 side, and the electrical contacts of the connector 3222 and the electrical contacts of the connector 3232 are separated from each other.

On the other hand, when the replacement unit 3230 is attached to the base unit 3220, the user aligns the projection 208e with the slit 1104f, and then moves the replacement unit 3230 toward the base unit 3220 in the z-axis direction with the lock pin 3233 pushed. When the replacement unit 3230 is correctly coupled to the base unit 3220, the lock pin 3233 protrudes from the hole 208d, and movement of the replacement unit 3230 relative to the base unit 3220 is locked. Through this user operation, the connector 3222 on the base unit 3220 side and the connector 3232 on the replacement unit 3230 side are coupled, and the electrical contacts of the connector 3222 and the electrical contacts of the connector 3232 contact each other. Because the connector 3232 included in the replacement unit 3230 is located outside the internal space 213, the connector 3232 is coupled to the connector 3222 while keeping the internal space 213 sealed.

The electronic stethoscope 3200 includes two of the lock pins 3233. The number of the lock pins 3233 need not be two, and may be one, three or more, or the like, using no more than two lock pins 3233 makes it easy for the user to manipulate the lock pins 3233 with their fingertips. In the electronic stethoscope 3200, the lock pin 3233 is included in the replacement unit 3230, and the hole 208d that engages with the lock pin 3233 is included in the base unit 3220. Instead, however, the lock pin may be included in the base unit 3220, and the hole that engages with the lock pin may be included in the replacement unit 3230. In the electronic stethoscope 3200, the chest piece 3210 is coupled to the grip 2820 so as to be capable of pivoting. Instead, however, the chest piece 3210 may be coupled to the grip 2820 so as to be incapable of pivoting. The electronic stethoscope 3200 need not include the grip 2820, and may be constituted by the chest piece 3210 alone. In this case too, the chest piece 3210 may be divided into the base unit and the replacement unit. The attachment/removal mechanism using the lock pin 3233 enables attachment/detachment without the need for a tool such as a screwdriver. Instead, however, the operation of attaching and removing the replacement unit 3230 to and from the base unit 3220 may include an operation of using a tool, such as using a screwdriver to remove a screw.

After the replacement unit 3230 is removed from the base unit 3220, the same replacement unit 3230 may be attached to the base unit 3220, or another replacement unit 3230 may be attached to the base unit 3220. The positional relationship between the reflective medium 207, the light emitter 202, and the photodetector 204 in the replacement unit 3230 varies from one replacement unit 3230 to another due to manufacturing error or the like. As such, parameters determined by this positional relationship have values unique to each replacement unit 3230. Such parameters can include the amount of light that reaches the photodetector 204 when the diaphragm 206 is not pressed, or the amount of change in the amount of light that reaches the photodetector 204 per unit of displacement amount of the diaphragm 206. Accordingly, the replacement unit 3230 may further include a memory 3234 that stores the parameters determined by the positional relationship between the reflective medium 207, the light emitter 202, and the photodetector 204. The memory 3234 may be mounted on the relay board 3231, for example. After the replacement unit 3230 is attached to the base unit 3220, the audio output unit 1020 mounted by the main circuit board 2910 included in the base unit 3220 may read out the parameters from the memory 3234 and adjust the generation of the signal in accordance with those parameters.

Variations on Attachment/Removal Mechanism of Electronic Auscultation Apparatus of Fourth Embodiment

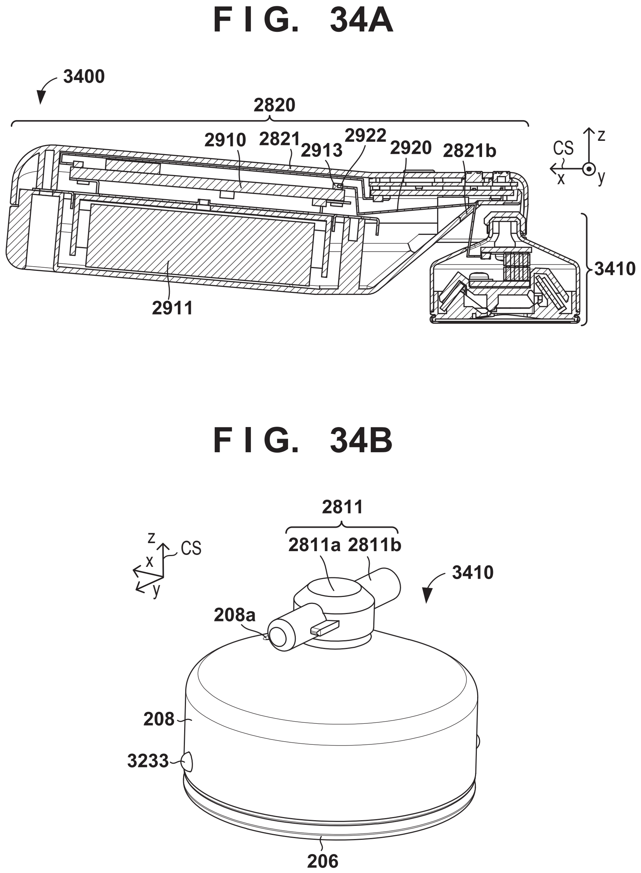

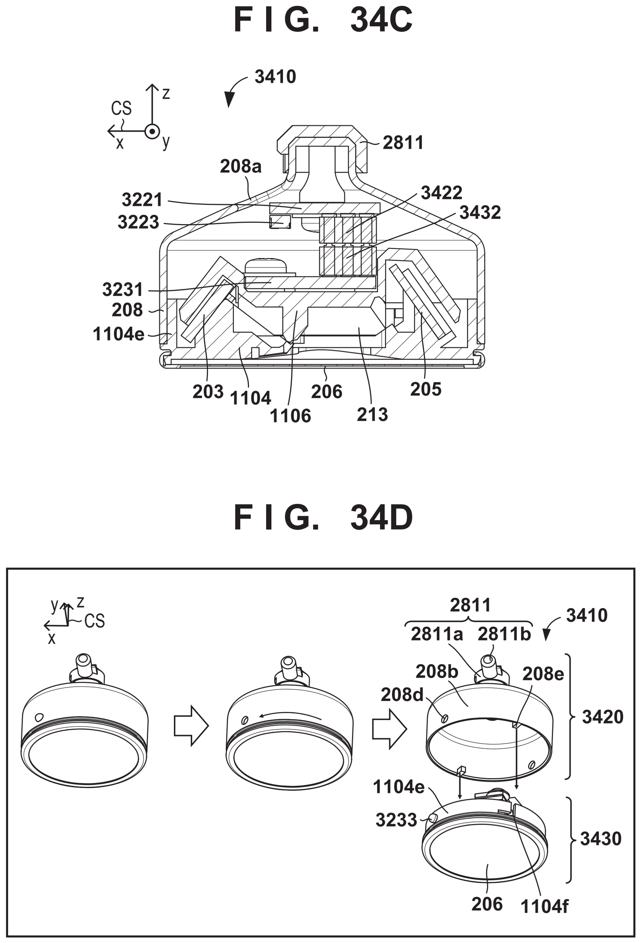

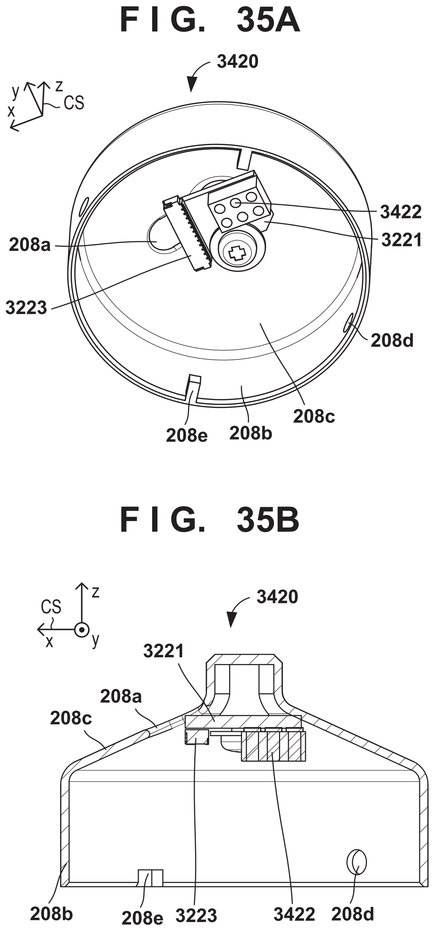

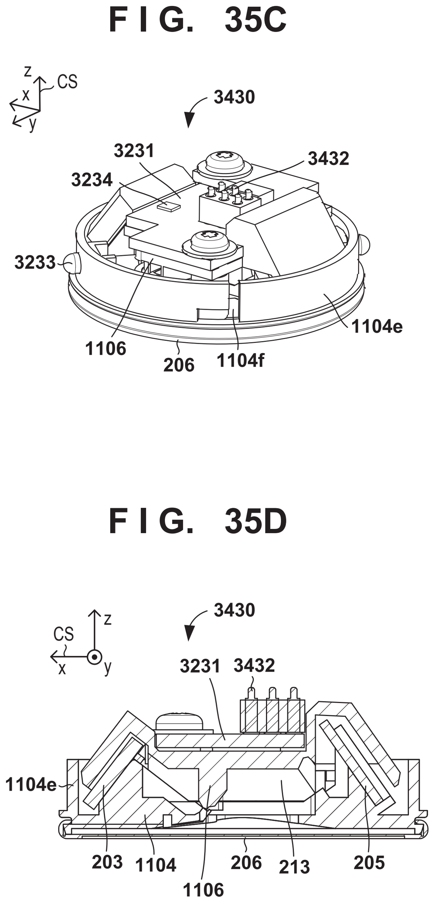

Variations on the attachment/removal mechanism of the electronic stethoscope 3200 according to the fourth embodiment will be described with reference to FIGS. 34A to 35D. FIGS. 34A to 35D correspond to FIGS. 32A to 33D, respectively. An electronic stethoscope 3400 differs from the electronic stethoscope 3200 in that a chest piece 3410 is provided instead of the chest piece 3210. In addition, the electronic stethoscope 3400 differs from the electronic stethoscope 3200 in that a base unit 3420 and a replacement unit 3430 are provided instead of the base unit 3220 and the replacement unit 3230. The other points are the same as those of the electronic stethoscope 3200.

The electronic stethoscope 3400 has a connector 3422 and a connector 3432 instead of the connector 3222 and the connector 3232. The connector 3432 is mounted on the upper surface of the relay board 3231. The connector 3422 is mounted on the lower surface of the relay board 3221. The connector 3422 and the connector 3223 are electrically connected to each other by a conductive pattern formed in the relay board 3221. The connector 3422 includes a plurality of electrical contacts, and a part of each electrical contact is exposed on the lower surface of the connector 3422. The connector 3432 includes a plurality of electrical contacts, and a part of each electrical contact is exposed on the upper surface of the connector 3432.

In the electronic stethoscope 3400, the shape of the projection 208e and the shape of the slit 1104f are different from those in the electronic stethoscope 3200 described above. The slit 1104f has an L shape. Specifically, the slit 1104f includes a vertical part extending in the normal direction of the contact surface 206a of the diaphragm 206 from the upper end of the side wall 1104e, and a horizontal part extending along the outer periphery of the diaphragm 206 from the lower end of the vertical part. The projection 208e may be a cube as illustrated, or may be another shape capable of passing through the slit 1104f.

User operations for attaching and removing the replacement unit 3430 to and from the base unit 3420 will be described with reference to FIG. 34D. A user operation for removing the replacement unit 3430 from the base unit 3420 includes rotating the replacement unit 3430 relative to the base unit 3420 with the lock pin 3233 pressed, and then moving the replacement unit 3430 away from the base unit 3420. As described above, when the lock pin 3233 is pushed in, the movement of the replacement unit 3430 relative to the base unit 3420 is unlocked. In this state, the replacement unit 3430 is removed from the base unit 3420 by moving the replacement unit 3430 relative to the base unit 3420 such that the projection 208e moves along the slit 1104f. Through this user operation, the electrical contacts of the connector 3422 and the electrical contacts of the connector 3432 are separated from each other.

A user operation for attaching the replacement unit 3430 to the base unit 3420 includes aligning the projection 208e with the slit 1104f, moving the replacement unit 3430 toward the base unit 3420 with the lock pin 3233 pressed, and then rotating the replacement unit 3430 relative to the base unit 3420. When the replacement unit 3430 is correctly coupled to the base unit 3420, the lock pin 3233 protrudes from the hole 208d, and movement of the replacement unit 3430 relative to the base unit 3420 is locked. Through this user operation, the electrical contacts of the connector 3422 and the electrical contacts of the connector 3432 contact each other. Because the connector 3432 included in the replacement unit 3430 is located outside an internal space 233, the connector 3432 is coupled to the connector 3422 while maintaining the internal space 233.

The regulating mechanism that regulates the movement path of the replacement unit 3230 relative to the base unit 3220 may be configured in another manner. For example, the regulating mechanism may be constituted by a thread provided on the inner surface of the cylindrical part 208b of the housing 208, and a threaded groove provided on the outer surface of the side wall 1104e. In this case, the user operation for removing the replacement unit 3430 from the base unit 3420 may be to rotate the replacement unit 3430 relative to the base unit 3420 while pressing the lock pin 3233.

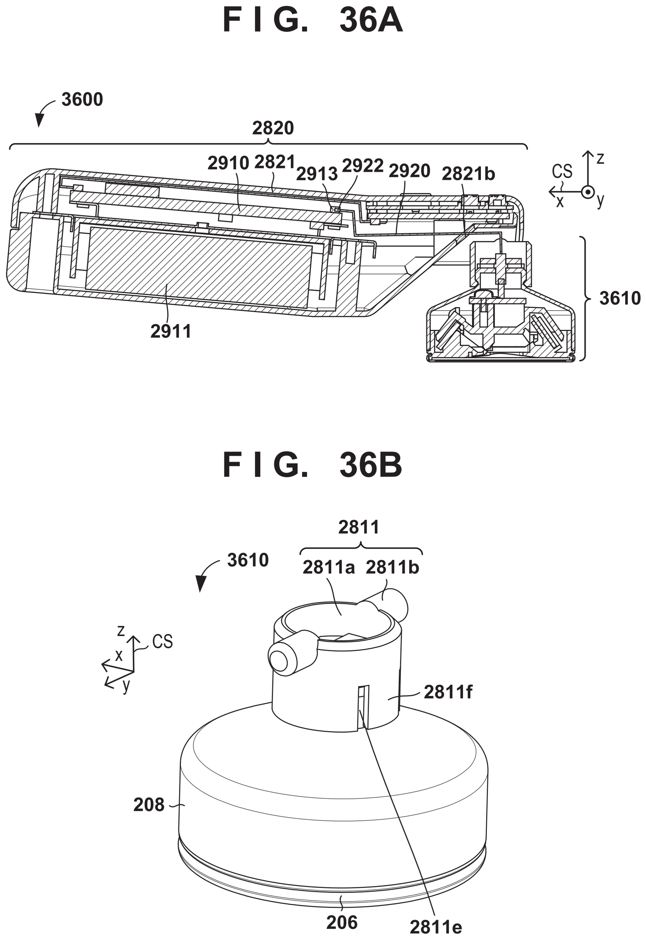

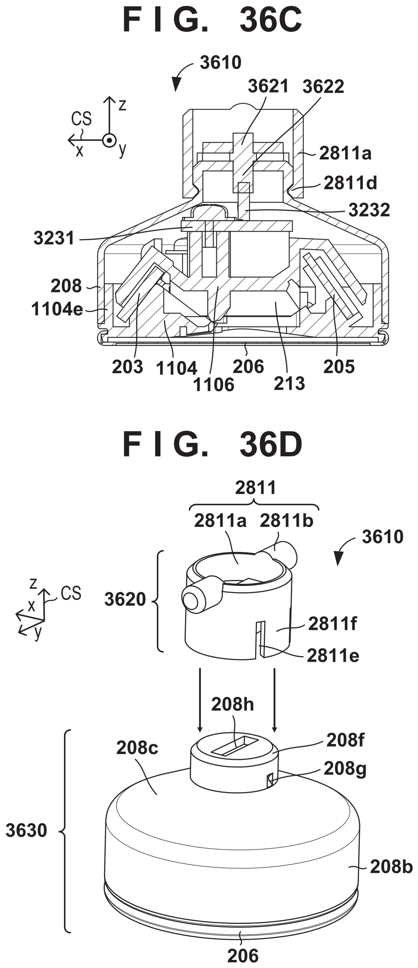

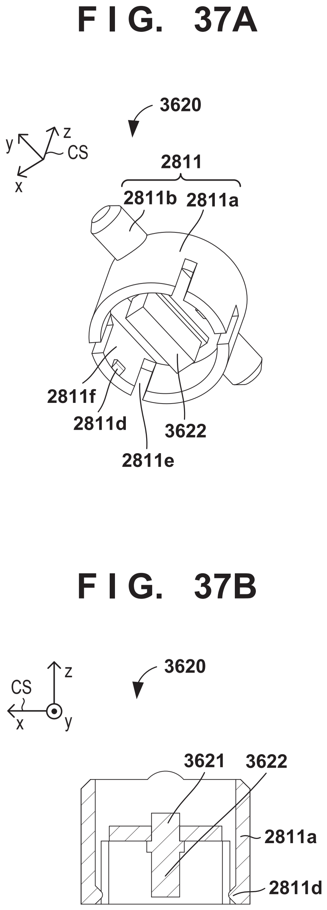

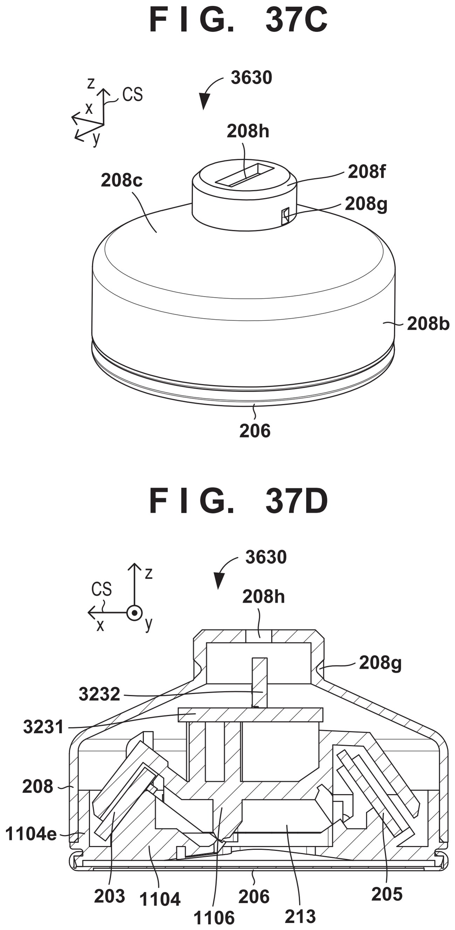

An electronic stethoscope 3600 according to a variation on the electronic stethoscope 3200 according to the fourth embodiment will be described with reference to FIGS. 36A to 37D. FIGS. 36A to 37D correspond to FIGS. 32A to 33D, respectively. The electronic stethoscope 3600 differs from the electronic stethoscope 3200 in that a chest piece 3610 is provided instead of the chest piece 3210. In addition, the electronic stethoscope 3600 differs from the electronic stethoscope 3200 in that a base unit 3620 and a replacement unit 3630 are provided instead of the base unit 3220 and the replacement unit 3230.

As illustrated in FIG. 36D, in the electronic stethoscope 3600 according to the variation on the fourth embodiment, the housing 208 is included in the replacement unit 3630 rather than in the base unit 3620. The housing 208 is coupled to the holding member 201 in a manner that does not anticipate removal through a user operation. Accordingly, the replacement unit 3630 does not include the attachment/removal mechanism described in FIGS. 33A to 33D (the lock pin 3233, the hole 208d, the projection 208e, and the slit 1104f).

On the other hand, the coupling member 2811 includes a connector 3621 and a connector 3622. The connector 3621 and the connector 3622 are electrically connected to each other. The connector 3621 is exposed to the outside of the chest piece 3610, and the connector 2921 of the wire harness 2920 is connected thereto. The connector 3622 is located inside the chest piece 3610 and is connected to the connector 3232. A hole 208h for passing the connector 3622 through is formed in the upper surface of the housing 208.

In the electronic stethoscope 3600, the housing 208 is coupled to the coupling member 2811 through an attachment/removal mechanism so as to be removable through a user operation. The attachment/removal mechanism is constituted by a recess 208g, a projection 2811d, and a flap 2811f. The recess 208g is formed on a side surface of a projection 208f located in the center of the upper surface of the housing 208. The flap 2811f is included in the coupling member 2811. Specifically, the flap 2811f is formed by two slits 2811e extending from the lower end of the central part 2811a of the coupling member 2811. The projection 2811d is formed on the inner surface of the flap 2811f. The projection 2811d may be referred to as a "claw".

When the housing 208 is coupled to the coupling member 2811, the projection 2811d engages with the recess 208g. The projection 2811d remains engaged with the recess 208g unless at least a predetermined force is applied to the housing 208 in a direction away from the coupling member 2811. The surface of the projection 2811d is inclined with respect to the surface normal of the contact surface 206a of the diaphragm 206. Accordingly, when a force is applied to the housing 208 in a direction away from the coupling member 2811, the flap 2811f deforms and expands outward, and the projection 2811d exits the recess 208g.

User operations for attaching and removing the replacement unit 3630 to and from the base unit 3620 will be described with reference to FIG. 36D. A user operation for removing the replacement unit 3630 from the base unit 3620 includes moving the replacement unit 3630 relative to the base unit 3620 such that the flap 2811f deforms. In the example illustrated in FIG. 36D, the user operation includes moving the replacement unit 3630 away from the base unit 3620. Instead, however, by changing the orientation of the projection 2811d, the user operation may include moving the replacement unit 3630 in another direction relative to the base unit 3620. Through this user operation, the connector 3232 is disconnected from the connector 3622, and the electrical contacts of the connector 3622 and the electrical contacts of the connector 3232 are separated from each other.

A user operation for attaching the replacement unit 3630 to the base unit 3620 includes passing the connector 3622 through the hole 208h and then moving the replacement unit 3630 relative to the base unit 3620 such that the flap 2811f deforms. In this user operation, the connector 3622 and the hole 208h function as a regulating mechanism that regulates the movement path of the replacement unit 3630 relative to the base unit 3620. In the example illustrated in FIG. 36D, the user operation includes moving the replacement unit 3630 closer to the base unit 3620. When the replacement unit 3630 is correctly coupled to the base unit 3620, the projection 2811d engages with the recess 208g. Through this user operation, the connector 3622 and the connector 3232 are coupled, and the electrical contacts of the connector 3622 and the electrical contacts of the connector 3232 contact each other. Because the connector 3232 included in the replacement unit 3630 is located outside the internal space 213, the connector 3232 is coupled to the connector 3622 while maintaining the internal space 213.

The electronic stethoscope 3600 includes two flaps 2811f having projections 2811d. Instead, however, the number of flaps 2811f having the projections 2811d may be one, or three or more. In the electronic stethoscope 3600, the flap 2811f has the projection 2811d and the housing 208 has the recess 208g. Instead, however, the flap 2811f may have a recess and the housing 208 may have a projection.

According to the foregoing embodiment, an electronic stethoscope capable of accurately detecting displacement in a surface of a target body can be provided. The electronic stethoscope according to the present embodiment has various advantages over conventional stethoscopes. Several examples will be described below.

First, one advantage is that the volume can be adjusted. The electronic stethoscope of the present embodiment adjusts the gain of the digitized sound signal and transmits the signal to the audio output device. Accordingly, the volume can be easily changed manually or automatically. For example, the volume can be increased as needed according to the doctor's hearing ability and the patient's constitution, which improves the audibility and usability.

Second, one advantage is that low-noise body sound auscultation can be performed. Depending on the auscultation environment, various types of noise may enter the displacement signal, such as ambient noise, the sound of clothing rubbing on the diaphragm 206 when the auscultation apparatus is applied to the patient's skin over the clothing, and the like. Such noise is eliminated or reduced by various signal processing circuits and microcontrollers. As a result, the audibility of the body sound is improved.

Third, one advantage is that low-frequency sounds and ultra-low-frequency sounds can be auscultated. In the auscultation of cardiac sounds, it may be necessary to listen to sounds at frequencies in the tens of Hz, which are difficult to hear by the human ear. In addition, it may be necessary to listen to cardiac sounds below 20 Hz, which are said to be inaudible to the human ear. The electronic stethoscope according to the present embodiment makes it possible to hear low-frequency body sounds at around 10 Hz.

Fourth, one advantage is that the body sound data can be stored in a high-capacity storage device of a computer, the body sound can be listened to again, and the body sound can be linked to electronic medical record describing the details of the patient diagnosis. The doctor can also use the computer to diagnose diseases or the like of the patient in question from a report visualizing the sound signal.

Fifth, one advantage is that the apparatus can be used for online medical care. The electronic stethoscope of the present embodiment is capable of wirelessly outputting the body sound data to earphones and mobile devices in real time. As a result, a doctor can make a diagnosis even in an environment where the doctor and the patient are not together.

The electronic stethoscope described above is mainly used as a diagnostic tool for doctors, nurses, and public health nurses to apply a diaphragm to the surface of a target body, such as a patient, and listen to cardiac sounds and respiratory sounds. However, applications in which the electronic stethoscope is used not as a diagnostic tool, but by a general user other than a medical professional, are of course conceivable. For example, it is conceivable for a general user to use the electronic stethoscope for promoting and managing their health. In such a case, it is also conceivable that the electronic stethoscope can be installed in a health management device having a function for measuring vital data such as pulse and body temperature. Accordingly, the electronic stethoscope according to the present embodiment can also be applied in a health management device capable of simultaneously obtaining biometric information other than body sounds, as well as body sounds.

Furthermore, the electronic stethoscope of the present embodiment may be used in the diagnosis of animals aside from humans. In other words, it goes without saying that the electronic stethoscope can be applied as an electronic stethoscope for pets used by veterinarians.

The electronic stethoscope of the present embodiment detects displacement of the diaphragm, and therefore may be referred to as a "displacement detection apparatus". The detection of anomalous sounds in industrial situations is one non-auscultation application of the displacement detection apparatus. For example, pipelines such as gas and water supply lines may produce low-frequency sounds inaudible to the human ear when anomalies occur. The displacement detection apparatus of the present embodiment can be applied to an apparatus for identifying the source of such low-frequency sounds and listening to the low-frequency sounds. When using the displacement detection apparatus in such an application, the user bring the diaphragm of the displacement detection apparatus into contact with an object of interest. The displacement detection apparatus then detects vibration in the object, converts a displacement signal representing the vibration into a sound signal, and transmits the signal to the audio output device. This enables the user to identify the source of the low-frequency sound. Pipelines such as gas and water supply lines, as well as outdoor air conditioner units, are conceivable as sources of low-frequency sounds. A motor, which is a source of vibration, can also be the object of interest. In addition, various industrial machines can also be objects of interest. In other words, in addition to auscultation, the displacement detection apparatus can be used for various purposes as an apparatus that detects vibrations in an object of interest and outputs sound.

Other Embodiments

The present invention can be implemented by processing of supplying a program for implementing one or more functions of the above-described embodiment to a system or apparatus via a network or storage medium, and causing one or more processors in a computer of the system or apparatus to read out and execute the program. The present invention can also be implemented by a circuit (for example, an ASIC) for implementing one or more functions.

Summary of Embodiments

1. An electronic device characterized by comprising:

a diaphragm that contacts a subject, the diaphragm being provided with a reflective medium on a surface of the diaphragm opposite from a contact surface of the diaphragm that makes contact with the subject;
a light-emitting diode;
an aperture that narrows light emitted from the light-emitting diode;
a photodetector having a light-receiving surface that receives light that passes through the aperture and is specularly reflected by the reflective medium;
output means for outputting a signal that is based on light in an illuminated region formed by specularly-reflected light reaching the light-receiving surface; and
a housing that houses the light-emitting diode, the aperture, and the photodetector within the housing,
wherein the diaphragm is configured to form part of an exterior of the electronic device along with the housing, and to elastically deform when pressed by the subject in contact with the contact surface, and
a boundary line that is between the illuminated region on the light-receiving surface and a region other than the illuminated region, and that is formed by the light narrowed by the aperture and specularly reflected by the reflective medium, moves in accordance with displacement of the contact surface caused by elastic deformation of the diaphragm, thereby changing an area of the illuminated region on the light-receiving surface and changing an output of the output means.

2. The electronic device according to Item 1, characterized in that when a displacement amount of the contact surface increases, the area of the illuminated region on the light-receiving surface decreases.

3. The electronic device according to Item 1, characterized in that when a displacement amount of the contact surface increases, the area of the illuminated region on the light-receiving surface increases.

4. The electronic device according to any one of Items 1 to 3, characterized in that the area of the illuminated region is determined by the boundary line, a first line segment that does not move in accordance with displacement of the contact surface and whose length does not change even when the contact surface displaces, and a second line segment that does not move in accordance with displacement of the contact surface and whose length changes when the contact surface displaces.

5. The electronic device according to Item 4, characterized by comprising, when the aperture is a first aperture, a second aperture that narrows the light specularly reflected by the reflective medium,
wherein the first line segment is a line segment formed by the light narrowed by the second aperture.

6. The electronic device according to Item 4, characterized in that the first line segment is a line segment that forms an edge of a photosensitive area of the photodetector.

7. The electronic device according to any one of Items 1 to 5, characterized in that the electronic device is a device that measures a vibration in the subject.

8. The electronic device according to any one of Items 1 to 7, characterized by comprising transmitting means for transmitting a sound signal that is based on output from the output means to an external audio output device.

9. The electronic device according to Item 6 or 7, characterized by comprising:

a chest piece, including the housing, that measures a vibration in the subject; and
a grip attached to the chest piece and gripped by a user.

10. The electronic device according to Item 9, characterized in that the grip includes a display device that displays a state of the electronic device.

11. The electronic device according to Item 9 or 10, characterized in that the grip includes an operation unit for accepting a setting for the electronic device.

12. The electronic device according to any one of Items 1 to 11, characterized in that the reflective medium is provided in a partial region including a center of a circle of the diaphragm, on the surface opposite from the contact surface.

13. The electronic device according to any one of Items 1 to 12, characterized in that when an incident angle of light incident on a surface of the reflective medium is $\theta$, a relationship of $45° < \theta < 90°$ is satisfied.

14. The electronic device according to any one of Items 1 to 13, characterized in that the reflective medium is a sheet-shaped member affixed to the surface of the diaphragm opposite from the contact surface.

15. The electronic device according to any one of Items 1 to 14, characterized in that the housing is constituted by a metal material, and an areal density of the housing is greater than an areal density of the diaphragm.

16. An electronic device characterized by comprising:

a diaphragm that contacts a subject, the diaphragm being provided with a reflective medium on a surface of the diaphragm opposite from a contact surface of the diaphragm that makes contact with the subject;
a light-emitting diode;
an aperture that narrows light emitted from the light-emitting diode;
a photodetector having a light-receiving surface that receives light that passes through the aperture and is specularly reflected by the reflective medium;
output means for outputting a signal that is based on light reaching the light-receiving surface; and
a housing that houses the light-emitting diode, the aperture, and the photodetector within the housing,
wherein the diaphragm is configured to form part of an exterior of the electronic device along with the housing, and to elastically deform when pressed by the subject in contact with the contact surface.

17. An electronic device characterized by comprising:

a diaphragm that contacts a subject, the diaphragm being provided with a reflective medium on a surface of the diaphragm opposite from a contact surface of the diaphragm that makes contact with the subject;
a light-emitting diode;
an aperture that narrows light emitted from the light-emitting diode;
a photodetector having a light-receiving surface that receives light that passes through the aperture and is specularly reflected by the reflective medium, the photodetector generating a signal that is based on light reaching the light-receiving surface;
extracting means for extracting a signal of a component of a specific frequency band included in the signal generated by the photodetector;
audio output means for outputting a sound that is based on the signal extracted by the extracting means; and
control means for increasing or reducing a volume of the audio output means, based on the signal generated by the photodetector before being extracted by the extracting means.

18. The electronic device according to Item 17, characterized in that the control means determines whether the diaphragm is in a state of being pressed by the subject based on the signal generated by the photodetector before being extracted by the extracting means, increases the volume of the audio output means from a first volume to a second volume greater than the first volume in a case where the diaphragm is determined to be in the state of being pressed by the subject, and reduces the volume from the second volume to the first volume in a case where the diaphragm is determined not to be in the state of being pressed by the subject.

19. The electronic device according to Item 18, characterized in that a time required to reduce the volume from the second volume to the first volume is shorter than a time required to increase the volume from the first volume to the second volume.

20. The electronic device according to any one of Items 17 to 19, characterized by comprising notifying means for notifying a user of information pertaining to a state of pressing of the diaphragm determined based on the signal generated by the photodetector before being extracted by the extracting means.

21. The electronic device according to any one of Items 17 to 20, characterized in that the component of the specific frequency band includes a component of a frequency band in a range of 10 Hz to 1 kHz.

22. An electronic device characterized by comprising:

a diaphragm that contacts a subject, the diaphragm being provided with a reflective medium on a surface of the diaphragm opposite from a contact surface of the diaphragm that makes contact with the subject;
a light-emitting diode;
an aperture that narrows light emitted from the light-emitting diode;
a photodetector having a light-receiving surface that receives light that is emitted from the light-emitting diode and is specularly reflected by the reflective medium, the photodetector generating a signal that is based on light reaching the light-receiving surface;
a high-pass filter that passes a frequency component of the signal generated by the photodetector higher than a cutoff frequency of at least 10 Hz and less than 20 Hz;
an amplifier circuit that amplifies the signal processed by the high-pass filter;
a signal processing unit that processes a signal generated by the photodetector; and
transmitting means for transmitting a sound signal that is based on the signal amplified by the amplifier circuit to an external audio output device.

23. The electronic device according to Item 22, characterized by further comprising a low-pass filter that passes a frequency component of the signal processed by the high-pass filter lower than a cutoff frequency of at least 1 kHz and less than 2 kHz.

24. An electronic device capable of operating in a plurality of modes including a first mode and a second mode having a lower power consumption than the first mode, the electronic device characterized by comprising:

a diaphragm that contacts a subject, the diaphragm being provided with a reflective medium on a surface of the diaphragm opposite from a contact surface of the diaphragm that makes contact with the subject;
a light-emitting diode;
an aperture that narrows light emitted from the light-emitting diode;
a photodetector having a light-receiving surface that receives light that passes through the aperture and is reflected by the reflective medium, the photodetector generating a signal that is based on light reaching the light-receiving surface;
audio output means for outputting sound that is based on the signal generated by the photodetector; and
control means for transitioning the mode of the electronic device to the first mode or the second mode, based on an output from the photodetector.

25. The electronic device according to Item 24, characterized in that in the first mode, the control means transitions the mode of the electronic device to the second mode in a case where the output from the photodetector does not change for a set period of time.

26. The electronic device according to Item 24 or 25, characterized in that in the second mode, the control means transitions the mode of the electronic device to the first mode in a case where the output from the photodetector exceeds a threshold.

27. The electronic device according to any one of Items 24 to 26, characterized by comprising an accelerometer that detects motion of the electronic device,
wherein the control means transitions the mode of the electronic device to the second mode or the first mode, based on the output from the photodetector and an output from the accelerometer.

28. The electronic device according to any one of Items 24 to 27, characterized by comprising accepting means for accepting an instruction regarding a transition of the mode of the electronic device from a user,
wherein the control means transitions the mode of the electronic device from the second mode to the first mode, based on the output from the photodetector and the instruction accepted by the accepting means.

29. The electronic device according to any one of Items 24 to 28, characterized by comprising a display device that displays a state of the electronic device,
wherein in the first mode, power is supplied to the display device, and in the second mode, supply of power to the display device is stopped.

30. An electronic device characterized by comprising:

a diaphragm that contacts a subject, the diaphragm being provided with a reflective medium on a surface of the diaphragm opposite from a contact surface of the diaphragm that makes contact with the subject;
a light-emitting diode;
an aperture that narrows light emitted from the light-emitting diode;
a photodetector having a light-receiving surface that receives light that passes through the aperture and is specularly reflected by the reflective medium; and
output means for outputting a signal that is based on light in an illuminated region formed by specularly-reflected light reaching the light-receiving surface,
wherein the diaphragm is configured to elastically deform when pressed by the subject in contact with the contact surface,
a boundary line that is between the illuminated region on the light-receiving surface and a region other than the illuminated region, and that is formed by the light narrowed by the aperture and specularly reflected by the reflective medium, moves in accordance with displacement of the contact surface caused by elastic deformation of the diaphragm, thereby changing an area of the illuminated region on the light-receiving surface and changing an output of the output means, and
a movement amount of the boundary line is greater than a displacement amount of the contact surface caused by the elastic deformation of the diaphragm.

31. An electronic device characterized by comprising a chest piece for measuring a vibration in a subject and a grip to be operated by a user,

wherein the chest piece includes:

a diaphragm that contacts the subject, the diaphragm being provided with a reflective medium on a surface of the diaphragm opposite from a contact surface of the diaphragm that contacts the subject;
a light-emitting diode;
an aperture that narrows light emitted from the light-emitting diode; and
a photodetector having a light-receiving surface that receives light that passes through the aperture and is specularly reflected by the reflective medium,

the grip includes:
output means for outputting, to the exterior, a signal that is based on light reaching the light-receiving surface, and
the chest piece is coupled to the grip to be capable of pivoting relative to the grip.

32. An electronic device characterized by comprising a base unit and a replacement unit,

wherein the replacement unit includes:

a diaphragm that contacts a subject, the diaphragm being provided with a reflective medium on a surface of the diaphragm opposite from a contact surface of the diaphragm that makes contact with the subject;
a light-emitting diode;
an aperture that narrows light emitted from the light-emitting diode;
a photodetector that receives light that passes through the aperture and is specularly reflected by the reflective medium; and
a holding member that holds the light-emitting diode and the photodetector,

the base unit includes:

a housing that houses the light-emitting diode, the aperture, the photodetector, and the holding member within the housing; and
output means for outputting, to the exterior, a signal that is based on the output of the photodetector, and

the electronic device comprises an attachment and removal mechanism that couples the holding member to the housing so as to be attachable and removable by a user operation.

33. An electronic device characterized by comprising:

a diaphragm that contacts a subject, the diaphragm being provided with a reflective medium on a surface of the diaphragm opposite from a contact surface of the diaphragm that makes contact with the subject;
a light-emitting diode;
an aperture that narrows light emitted from the light-emitting diode;
a photodetector having a light-receiving surface that receives light that passes through the aperture and is specularly reflected by the reflective medium;
vibration detecting means for detecting an air vibration in a space facing the reflective medium the air vibration being produced by displacement of the contact surface of the diaphragm; and
output means for outputting, to the exterior, a signal based on light reaching the light-receiving surface and a signal based on the air vibration detected by the vibration detecting means.

The invention is not limited to the above embodiment and various changes and modifications can be made within the spirit and scope of the invention. Therefore, claims have been appended to apprise the public of the scope of the invention.

This application claims priority from Japanese Patent Application No. 2023-106205, filed June 28, 2023; Japanese Patent Application No. 2023-206277, filed December 6, 2023; Japanese Patent Application No. 2023-213194, filed December 18, 2023; Japanese Patent Application No. 2023-207172, filed December 7, 2023; Japanese Patent Application No. 2023-214037, filed December 19, 2023; Japanese Patent Application No. 2023-213195, filed December 18, 2023; Japanese Patent Application No. 2023-210285, filed December 13, 2023; Japanese Patent Application No. 2023-198526, filed November 22, 2023; Japanese Patent Application No. 2024-057616, filed March 29, 2024; Japanese Patent Application No. 2024-063542, filed April 10, 2024; and Japanese Patent Application No. 2024-065612, filed April 15, 2024. The entire contents thereof are hereby incorporated by reference herein.

**Claims**

1.  An electronic device comprising:

a diaphragm having a contact surface configured to contact a subject and a reflective medium on a surface of the diaphragm opposite the contact surface, the diaphragm being configured to elastically deform when pressed by the subject at the contact surface;
a light-emitting diode;
an aperture configured to narrow light emitted by the light-emitting diode;
a photodetector having a light-receiving surface configured to receive light that passes through the aperture and is specularly reflected by the reflective medium;
an output unit configured to output a signal based on light in an illuminated region formed on the light-receiving surface by the reflected light; and
a housing enclosing the light-emitting diode, the aperture, and the photodetector,
wherein the diaphragm is configured to form part of an exterior of the electronic device together with the housing, and
wherein a boundary of the illuminated region, the boundary being defined by the light narrowed by the aperture and specularly reflected by the reflective medium, moves in accordance with displacement of the contact surface caused by elastic deformation of the diaphragm, thereby changing an area of the illuminated region on the light-receiving surface and changing an output of the output unit.

2.  The electronic device according to claim 1, wherein an area of the illuminated region on the light-receiving surface decreases as a displacement amount of the contact surface increases.

3.  The electronic device according to claim 1, wherein an area of the illuminated region on the light-receiving surface increases as a displacement amount of the contact surface increases.

4.  The electronic device according to any one of claims 1 to 3, wherein the area of the illuminated region is defined by:

the boundary;
a first edge that is stationary with respect to displacement of the contact surface and whose length remains unchanged despite the displacement; and

a second edge that is stationary with respect to the displacement but whose length changes when the contact surface displaces.

5. The electronic device according to claim 4, further comprising a second aperture configured to narrow light specularly reflected by the reflective medium, wherein the first edge is defined by light narrowed by the second aperture.

6. The electronic device according to claim 4, wherein the first edge is an edge of a photosensitive area of the photodetector.

7. The electronic device according to any one of claims 1 to 5, wherein the electronic device is configured to measure vibration of the subject.

8. The electronic device according to any one of claims 1 to 7, further comprising a transmitter configured to transmit an audio signal based on an output of the output unit to an external audio output device.

9. The electronic device according to claim 6 or 7, further comprising:

a chest piece including the housing and configured to measure vibration of the subject; and

a grip attached to the chest piece and gripped by a user.

10. The electronic device according to claim 9, wherein the grip includes a display device configured to display a state of the electronic device.

11. The electronic device according to claim 9 or 10, wherein the grip includes an operation unit configured to accept user settings for the electronic device.

12. The electronic device according to any one of claims 1 to 11, wherein the reflective medium is provided, on the surface of the diaphragm opposite the contact surface, in a region including a center of the diaphragm.

13. The electronic device according to any one of claims 1 to 12, wherein an incident angle $\theta$ of light incident on a surface of the reflective medium satisfies $45° < \theta < 90°$.

14. The electronic device according to any one of claims 1 to 13, wherein the reflective medium is a sheet member affixed to the surface of the diaphragm opposite the contact surface.

15. The electronic device according to any one of claims 1 to 14, wherein the housing is made of metal and an areal density of the housing is greater than an areal density of the diaphragm.

16. An electronic device comprising:

a diaphragm having a contact surface configured to contact a subject and a reflective medium on a surface of the diaphragm opposite the contact surface, the diaphragm being configured to elastically deform when pressed by the subject at the contact surface;

a light-emitting diode;

an aperture configured to narrow light emitted by the light-emitting diode;

a photodetector having a light-receiving surface configured to receive light that passes through the aperture and is specularly reflected by the reflective medium;

an output unit configured to output a signal based on light in an illuminated region formed on the light-receiving surface by the specularly reflected light; and

a housing enclosing the light-emitting diode, the aperture, and the photodetector,

wherein the diaphragm is configured to form part of an exterior of the electronic device together with the housing.

17. An electronic device comprising:

a diaphragm having a contact surface configured to contact a subject and a reflective medium on a surface of the diaphragm opposite the contact surface, the diaphragm being configured to elastically deform when pressed by the subject at the contact surface;

a light-emitting diode;

an aperture configured to narrow light emitted by the light-emitting diode;

a photodetector having a light-receiving surface configured to receive light that passes through the aperture and is specularly reflected by the reflective medium, the photodetector generating a signal based on light reaching the light-receiving surface;

a signal extraction module configured to extract a biosignal in a specific frequency band from the signal generated by the photodetector;

an audio output unit configured to output sound based on the biosignal; and

a controller configured to increase or reduce a volume of the audio output unit based on the signal generated by the photodetector before extraction by the signal extraction module.

18. The electronic device according to claim 17, wherein the controller determines whether the diaphragm is being pressed by the subject based on the signal generated by the photodetector, increases the volume from a first volume to a second volume greater than the first volume when the diaphragm is determined to be pressed by the subject, and reduces the volume from the second volume to the first volume when the diaphragm is determined not to be pressed by the subject.

19. The electronic device according to claim 18, wherein a time required to reduce the volume from the second volume to the first volume is shorter than a time required to increase the volume from the first volume to the second volume.

20. The electronic device according to any one of claims 17 to 19, further comprising an indicator configured to notify a user of information regarding a pressing state of the diaphragm determined based on the signal generated by the photodetector.

21. The electronic device according to any one of claims 17 to 20, wherein the specific frequency band includes a frequency range from 10 Hz to 1 kHz.

22. An electronic device comprising:

a diaphragm having a contact surface configured to contact a subject and a reflective medium on a surface of the diaphragm opposite the contact surface, the diaphragm being configured to elastically deform when pressed by the subject at the contact surface;

a light-emitting diode;

an aperture configured to narrow light emitted by the light-emitting diode;

a photodetector having a light-receiving surface configured to receive light that passes through the aperture and is specularly reflected by the reflective medium, the photodetector generating a signal based on light reaching the light-receiving surface;

a high-pass filter having a cutoff frequency greater than or equal to 10 Hz and less than 20 Hz and configured to pass frequency components of the signal generated by the photodetector higher than the cutoff frequency;

an amplifier circuit configured to amplify the signal processed by the high-pass filter; and

a transmitter configured to transmit, to an external audio output device, an audio signal based on the signal amplified by the amplifier circuit.

23. The electronic device according to claim 22, further comprising a low-pass filter configured to pass a frequency component of the signal processed by the high-pass filter lower than a cutoff frequency of at least 1 kHz and less than 2 kHz.

24. An electronic device operable in a plurality of modes including a first mode and a second mode having lower power consumption than the first mode, the electronic device comprising:

a diaphragm having a contact surface configured to contact a subject and a reflective medium on a surface of the diaphragm opposite the contact surface;

a light-emitting diode;

an aperture configured to narrow light emitted by the light-emitting diode;

a photodetector having a light-receiving surface configured to receive light that passes through the aperture and is specularly reflected by the reflective medium, the photodetector generating a signal based on light reaching the light-receiving surface;

an output unit configured to output the signal generated by the photodetector; and

a controller configured to transition the electronic device to the first mode or the second mode based on an output of the photodetector.

25. The electronic device according to claim 24, wherein, in the first mode, the controller transitions the electronic device to the second mode when the output of the photodetector does not change for a set period of time.

26. The electronic device according to claim 24 or 25, wherein, in the second mode, the controller transitions the electronic device to the first mode when the output of the photodetector exceeds a threshold.

27. The electronic device according to any one of claims 24 to 26, further comprising an accelerometer configured to detect motion of the electronic device, wherein the controller transitions the electronic device to the second mode or the first mode based on the output of the photodetector and an output of the accelerometer.

28. The electronic device according to any one of claims 24 to 27, further comprising an input interface configured to accept a user instruction regarding a mode transition of the electronic device, wherein the controller transitions the electronic device from the second mode to the first mode based on the output of the photodetector and the user instruction.

29. The electronic device according to any one of claims 24 to 28, further comprising a display device configured to display a state of the electronic device, wherein, in the first mode, power is supplied to the display device, and, in the second mode, supply of power to the display device is stopped.

30. An electronic device comprising:

   a diaphragm having a contact surface configured to contact a subject and a reflective medium on a surface of the diaphragm opposite the contact surface, the diaphragm being configured to elastically deform when pressed by the subject at the contact surface;
   a light-emitting diode;
   an aperture configured to narrow light emitted by the light-emitting diode;
   a photodetector having a light-receiving surface configured to receive light that passes through the aperture and is specularly reflected by the reflective medium; and
   an output unit configured to output a signal based on light in an illuminated region formed on the light-receiving surface by the specularly reflected light,
   wherein a boundary of the illuminated region, the boundary being defined by light narrowed by the aperture and specularly reflected by the reflective medium, moves in accordance with displacement of the contact surface caused by elastic deformation of the diaphragm, thereby changing an area of the illuminated region on the light-receiving surface and changing an output of the output unit, and
   wherein a movement amount of the boundary is greater than a displacement amount of the contact surface caused by the elastic deformation of the diaphragm.

31. An electronic device comprising:
   a chest piece configured to measure vibration of a subject and a grip operable by a user, wherein the chest piece includes:

   a diaphragm having a contact surface configured to contact the subject and a reflective medium on a surface of the diaphragm opposite the contact surface;
   a light-emitting diode;
   an aperture configured to narrow light emitted by the light-emitting diode; and
   a photodetector having a light-receiving surface configured to receive light that passes through the aperture and is specularly reflected by the reflective medium, wherein the grip includes an output unit configured to output, to an external audio output device, a signal based on light reaching the light-receiving surface, and
   wherein the chest piece is pivotably coupled to the grip.

32. An electronic device comprising a base unit and a replacement unit, wherein the replacement unit includes:

   a diaphragm having a contact surface configured to contact a subject and a reflective medium on a surface of the diaphragm opposite the contact surface;
   a light-emitting diode;

an aperture configured to narrow light emitted by the light-emitting diode;

a photodetector configured to receive light that passes through the aperture and is specularly reflected by the reflective medium; and

a holding member that holds the light-emitting diode and the photodetector, wherein the base unit includes:

> a housing enclosing the light-emitting diode, the aperture, the photodetector, and the holding member; and
> an output unit configured to output, to an external audio output device, a signal based on an output of the photodetector, and

wherein the electronic device further comprises an attachment/removal mechanism configured to couple the holding member to the housing so as to be attachable and removable by a user operation

**33.** An electronic device comprising:

> a diaphragm having a contact surface configured to contact a subject and a reflective medium on a surface of the diaphragm opposite the contact surface;
> a light-emitting diode;
> an aperture configured to narrow light emitted by the light-emitting diode;
> a photodetector having a light-receiving surface configured to receive light that passes through the aperture and is specularly reflected by the reflective medium;
> a vibration detector configured to detect air vibrations within a space facing the reflective medium, the air vibrations being produced by displacement of the contact surface of the diaphragm; and
> an output unit configured to output, to an external audio output device, a signal based on light reaching the light-receiving surface and a signal based on the air vibrations detected by the vibration detector.

# F I G. 1A

# F I G. 1B

FIG. 2A

# FIG. 2B

# FIG. 2C

# F I G. 3A

# F I G. 3B

# F I G.  4A

# F I G.  4B

# F I G.  4C

# F I G. 4D

# F I G. 4E

# F I G. 4F

# FIG. 5

**100**

ELECTRONIC AUSCULTATION APPARATUS

**110**~ CHEST PIECE

**610**

SOUND OUTPUT UNIT

**618**

FILTER/AMP

**611**

A/D CONVERTER

**615**

AMP

**612**

AMP

**616**

VOLUME ADJUSTMENT UNIT

**613**

ENCODER

**614**

WIRELESS COMMUNICATION UNIT

**617**

WIRED COMMUNICATION UNIT

**630**

COMPUTER

**620**

SOUND OUTPUT DEVICE

# FIG. 6

# FIG. 7A

# FIG. 7B

# FIG. 7C

# FIG. 7D

FIG. 7E

FIG. 8A

FIG. 8B

FIG. 8C

FIG. 8D

FIG. 8E

F I G. 9A

F I G. 9B

# F I G. 9C

| | CROSS-SECTIONAL CONFIGURATION | CHANGE DISPLACEMENT SIGNAL | UPPER SIDE OF OPENING 706 | LOWER SIDE OF OPENING 706 | UPPER SIDE OF OPENING 707 | LOWER SIDE OF OPENING 707 |
|---|---|---|---|---|---|---|
| FIRST EMBODIMENT | | | DEFINE | DO NOT DEFINE | DEFINE | DO NOT DEFINE |
| FIRST VARIATION | | | DEFINE | DO NOT DEFINE | NOT PRESENT | NOT PRESENT |
| SECOND VARIATION | | | DO NOT DEFINE | DEFINE | DO NOT DEFINE | DEFINE |
| THIRD VARIATION | | | DO NOT DEFINE | DEFINE | NOT PRESENT | NOT PRESENT |
| FOURTH VARIATION | | | NOT PRESENT | NOT PRESENT | DEFINE | DO NOT DEFINE |

EP 4 736 775 A1

**1000**

ELECTRONIC AUSCULTATION APPARATUS

**1010**

CHEST PIECE

**1011**

DISPLACEMENT DETECTION UNIT

**1012**

VIBRATION DETECTION UNIT

**1020**

SOUND OUTPUT UNIT

**611**

A/D CONVERTER

**615**

AMP

**612**

AMP

**616**

VOLUME ADJUSTMENT UNIT

**613**

ENCODER

**1021**

OUTPUT SELECTION UNIT

**614**

WIRELESS COMMUNICATION UNIT

**617**

WIRED COMMUNICATION UNIT

**630**

COMPUTER

**620**

SOUND OUTPUT DEVICE

F I G.  10

FIG. 11A

FIG. 11B

FIG. 11C

FIG. 12A

**F I G.  12B**

**F I G.  13A**

**F I G.  13B**

**F I G.  13C**

**F I G. 13D**

**F I G. 13E**

**F I G. 13F**

**F I G. 13G**

F I G. 13H

1106c
1106a
1105
1105c
706
1106d
1106b
1106

z x
CS
y

F I G. 13I

1106c
1106a
1106e
707
1105
1105e
1106b
1106

x z
CS
y

F I G. 13J

1106c
1106a
1105
706
1105c
1106d
1106
1106b

CS
z x
y

F I G. 13K

1106a
1106c
1105
707
1105e
1106
1106e
1106b

CS
z x
y

86

FIG. 14A

FIG. 14B

FIG. 14C

F I G. 15A

F I G. 15B

F I G. 15C

F I G. 16A

F I G. 16B

F I G. 16C

# FIG. 17A

# F I G. 17B

# F I G. 17C

# F I G. 17D

1773

LIGHT-RECEIVING ELEMENT OUTPUT SIGNAL [V]

TIME [sec]

# F I G. 17E

1774

LIGHT-RECEIVING ELEMENT OUTPUT SIGNAL [V]

TIME [sec]

# F I G. 17F

# F I G. 17G

# F I G. 17H

**F I G. 18**

# F I G.   19A

```
            ┌─────────┐
            │  START  │
            └────┬────┘
                 │                    S1901
                 ▼
      ┌────────────────────────┐
      │   OBTAIN DIAPHRAGM      │
      │  DISPLACEMENT SIGNAL    │
      └───────────┬────────────┘
                  │                   S1902
                  ▼
              ╱───────╲         YES
          ╱   UNUSED    ╲──────────────┐
          ╲   STATE?    ╱               │
              ╲───────╱                 │
             NO  │                      │
        S1903    ▼                 S1904 ▼
   ┌───────────────────────┐   ┌───────────────────────┐
   │  SET NORMAL LEVEL AND │   │   SET MUTE LEVEL AND   │
   │ NOTIFY THAT IT IS IN- │   │  NOTIFY THAT IT IS MUTE│
   │       USE STATE       │   │                        │
   └───────────┬───────────┘   └───────────┬───────────┘
```

# F I G.   19B

```
            ┌─────────┐
            │  START  │
            └────┬────┘
                 │                    S1911
                 ▼
      ┌────────────────────────────┐
      │  A/D CONVERT SOUND SIGNAL   │
      └─────────────┬──────────────┘
                    │                 S1912
                    ▼
      ┌────────────────────────────┐
      │ PERFORM REDUCTION PROCESSING│
      │   AND SMOOTHING PROCESSING  │
      └─────────────┬──────────────┘
                    │                 S1913
                    ▼
      ┌────────────────────────────┐
      │     OUTPUT SOUND SIGNAL     │
      └─────────────┬──────────────┘
```

# F I G. 20

# FIG. 21

```
              ( START )
                  │
                  │         S1901
                  ▼
      ┌──────────────────────────┐
      │   OBTAIN DIAPHRAGM        │
      │   DISPLACEMENT SIGNAL     │
      └──────────────────────────┘
                  │
                  │         S1902
                  ▼
            ╱────────────╲         YES
           ╱    UNUSED     ╲──────────────────────┐
           ╲    STATE?     ╱                       │
            ╲────────────╱                         │
                  │ NO                             │
                  │         S2101                  │
                  ▼                                │
            ╱────────────╲         YES             │
           ╱ OVERPRESSURE ╲──────────┐             │
           ╲    STATE?     ╱         │             │
            ╲────────────╱          │             │
             │ NO    S1903          │      S1904  │
             ▼                      │             ▼
  ┌────────────────────┐            │   ┌────────────────────┐
  │ SET NORMAL LEVEL AND│           │   │ SET MUTE LEVEL AND  │
  │ NOTIFY THAT IT IS   │           │   │ NOTIFY THAT IT IS   │
  │ IN-USE STATE        │           │   │ MUTE                │
  └────────────────────┘            │   └────────────────────┘
             │              S2102   │             │
             │             ▼         │             │
             │   ┌────────────────────┐           │
             │   │ SET MUTE LEVEL AND │           │
             │   │ NOTIFY THAT IT IS  │           │
             │   │ MUTE AND           │           │
             │   │ OVERPRESSURE       │           │
             │   └────────────────────┘           │
             │             │                       │
             └─────────────┴───────────────────────┘
```

# F I G. 22

# F I G. 23

2400

POWER SUPPLY UNIT

1711 BATTERY

1712 CHARGING IC

1715 LOAD SW

1713 DC/DC

1716 LDO

1714 LDO

VBAT

VBUS

125 CONNECTOR

V2

V1

1700 MICROCONTROLLER

1750 3G SENSOR

202 LIGHT-EMITTING ELEMENT

204 LIGHT-RECEIVING ELEMENT

1101 MICROPHONE

1730 DIAPHRAGM DISPLACEMENT SIGNAL PROCESSING UNIT

1740 MICROPHONE SIGNAL PROCESSING UNIT

F I G. 24A

**F I G. 24B**

POWER SUPPLY UNIT — 2410

- 1711 BATTERY
- 1712 CHARGING IC
- 1713 DC/DC
- 1714 LDO
- 1715 LOAD SW
- 1716 LDO
- 2411 LOAD SW

VBAT, V0, V1, V2

VBUS

- 125 CONNECTOR
- 1700 MICROCONTROLLER
- 1750 3G SENSOR
- 202 LIGHT-EMITTING ELEMENT
- 204 LIGHT-RECEIVING ELEMENT
- 1730 DIAPHRAGM DISPLACEMENT SIGNAL PROCESSING UNIT
- 1101 MICROPHONE
- 1740 MICROPHONE SIGNAL PROCESSING UNIT

F I G. 24C

EP 4 736 775 A1

# F I G. 25A

**617**

WIRED COMMUNICATION UNIT

**125**

CONNECTOR

VBUS | D+ | D-

CHARGING IC

**1712**

**1700**

MICROCONTROLLER

**1711**

BATTERY

# F I G. 25B

**617**

WIRED COMMUNICATION UNIT

**125**

CONNECTOR

VBUS | D+ | D-

**1700**

MICROCONTROLLER

**1712**

CHARGING IC

# F I G. 26A

# F I G. 26B

```
                    ┌─────────┐
                    │  START  │
                    └─────────┘
                         │
                         ▼                    S2611
         ┌───────────────────────────────┐
         │          START TIMER          │
         └───────────────────────────────┘
                         │
     ┌───────────────────┤
     │                   ▼                    S2612
     │            ╱─────────────╲      NO
     │          ╱    IN USE?      ╲──────────┐
     │           ╲               ╱           │
     │             ╲───────────╱             │
     │                  │ YES                │
     │                  ▼              S2613  │
     │        ┌─────────────────────┐        │
     │        │      RESET TIMER     │        │
     │        └─────────────────────┘        │
     │                  │                     │
     │                  │◄────────────────────┘
     │                  ▼              S2614
     │            ╱─────────────╲      YES
     │          ╱   TRANSITION    ╲──────────┐
     │         ╱    INSTRUCTION     ╲         │
     │          ╲       ?          ╱          │
     │            ╲───────────────╱           │
     │                  │ NO                   │
     │                  ▼              S2615   │
     │    NO    ╱─────────────╲                │
     └─────────╱   DID TIMER     ╲             │
              ╱  EXCEED THRESHOLD  ╲           │
               ╲       ?          ╱            │
                 ╲───────────────╱             │
                      │ YES                    │
                      │◄──────────────────────┘
                      ▼              S2616
         ┌───────────────────────────────┐
         │  TRANSITION TO POWER-SAVING MODE │
         └───────────────────────────────┘
                      │
                      ▼
                 ┌─────────┐
                 │   END   │
                 └─────────┘
```

# F I G. 26C

START

S2621

IS RETURN
CONDITION SATISFIED
?

NO

YES

S2622

RETURN FROM POWER-SAVING MODE

END

# F I G. 27A

# F I G. 27B

# FIG. 28A

# FIG. 28B

# FIG. 28C

# FIG. 28D

# FIG. 29A

# FIG. 29B

# F I G. 29C

# F I G. 29D

# F I G. 30

# FIG. 31A

2800
2820
3100
3101
2810

# FIG. 31B

2800
2820
3100
3101
3101
2810

# FIG. 31C

2820
3100
3101
3101
2810
2820
3100
3101
3101
2810

# F I G. 32A

# F I G. 32B

# F I G. 32C

FIG. 32D

2811
2811a 2811b
3210

z
x CS
y

3220

208a
208c
208
208d
208b

3234 3231 3232
3230 3233 1104e
1106 206 1104f

FIG. 33A

y z
CS
x

3220

208a
3223
3208e
3221
3222
208d
208b
208c

F I G. 33B

3220

3221

208a

208c

CS

z

x

y

208b   208e   3223   3222   208d

F I G. 33C

3230

z

x

CS

y

3231

3234   3232

3233   1104e

1106   206   1104f

F I G. 33D

3230

z

CS

x   y

3232   3231

1104e

203   1104   206   205

1106   213

# F I G. 34A

# F I G. 34B

# F I G.   34C

# F I G.   34D

# FIG. 35A

3420

y z CS
x

3422

3221

208a

3223

208d

208c

208e 208b

# FIG. 35B

z
CS
x y

3221

3420

208a

208c

3223 3422 208d

208b 208e

# FIG. 35C

z
x
CS
y

3430

3231

3234

3432

3233

1106

206

1104f

1104e

# FIG. 35D

z
CS
x
y

3430

3231  3432

1104e

203  1104

1106

206  213

205

# F I G. 36A

# F I G. 36B

# F I G. 36C

# F I G. 36D

# F I G. 37A

# F I G. 37B

# F I G. 37C

# F I G. 37D

**EP 4 736 775 A1**

## INTERNATIONAL SEARCH REPORT

| International application No. |
|---|
| **PCT/JP2024/023412** |

**A. CLASSIFICATION OF SUBJECT MATTER**

*A61B 7/04*(2006.01)i; *A61B 5/00*(2006.01)i; *A61B 7/02*(2006.01)i; *G01H 17/00*(2006.01)i
FI: A61B7/04 T; A61B7/04 M; G01H17/00 Z; A61B5/00 101R; A61B7/04 J; A61B7/04 C; A61B7/04 P; A61B7/04 Z; A61B7/04 R; A61B7/02 F; A61B7/04 N; A61B7/04 L

According to International Patent Classification (IPC) or to both national classification and IPC

**B. FIELDS SEARCHED**

Minimum documentation searched (classification system followed by classification symbols)

A61B7/00-7/04; A61B5/02; G01H17/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | CN 113616233 A (BEIJING GOLDEN CHANNEL TECHNOLOGY CO., LTD.) 09 November 2021 (2021-11-09)<br>paragraphs [0007]-[0008], [0055]-[0056], fig. 5 | 1-33 |
| Y | JP 4-230820 A (DYNISCO INC.) 19 August 1992 (1992-08-19)<br>paragraph [0008] | 1-33 |
| Y | JP 2002-153459 A (TESSHOKAI) 28 May 2002 (2002-05-28)<br>paragraph [0077] | 17-21, 24-29 |
| Y | JP 2022-88956 A (ONE A KK) 15 June 2022 (2022-06-15)<br>paragraphs [0049]-[0052] | 17-21, 24-29 |
| Y | JP 2020-124446 A (OMRON HEALTHCARE CO., LTD.) 20 August 2020 (2020-08-20)<br>paragraph [0010] | 17-21, 24-29 |

☑ Further documents are listed in the continuation of Box C.　　☑ See patent family annex.

| | |
|---|---|
| \* Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **16 July 2024** | **17 September 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT

| | International application No. |
|---|---|
| | **PCT/JP2024/023412** |

**C. DOCUMENTS CONSIDERED TO BE RELEVANT**

| Category* | Citation of document, with indication, where appropriate, of the relevant passages | Relevant to claim No. |
|---|---|---|
| Y | JP 2020-531119 A (VITALCHAINS CORPORATION) 05 November 2020 (2020-11-05) paragraphs [0083]-[0087] | 32 |
| A | JP 2008-79688 A (CITIZEN HOLDINGS CO., LTD.) 10 April 2008 (2008-04-10) paragraph [0016], fig. 5 | 1-33 |

Form PCT/ISA/210 (second sheet) (July 2022)

## INTERNATIONAL SEARCH REPORT
### Information on patent family members

International application No.

**PCT/JP2024/023412**

| Patent document cited in search report | | | Publication date (day/month/year) | Patent family member(s) | | | Publication date (day/month/year) |
|---|---|---|---|---|---|---|---|
| CN | 113616233 | A | 09 November 2021 | (Family: none) | | | |
| JP | 4-230820 | A | 19 August 1992 | US | 5127269 | A | |
| | | | | column 1, lines 8-13 | | | |
| | | | | EP | 455241 | A2 | |
| | | | | KR 10-1991-0020424 | | A | |
| JP | 2002-153459 | A | 28 May 2002 | (Family: none) | | | |
| JP | 2022-88956 | A | 15 June 2022 | (Family: none) | | | |
| JP | 2020-124446 | A | 20 August 2020 | US | 2021/0361255 | A1 | |
| | | | | paragraph [0016] | | | |
| | | | | WO | 2020/162105 | A1 | |
| | | | | CN | 113301852 | A | |
| JP | 2020-531119 | A | 05 November 2020 | US | 2019/0053778 | A1 | |
| | | | | paragraphs [0083]-[0087] | | | |
| | | | | WO | 2019/036554 | A1 | |
| | | | | EP | 3668406 | A1 | |
| | | | | CN | 111031922 | A | |
| JP | 2008-79688 | A | 10 April 2008 | (Family: none) | | | |

Form PCT/ISA/210 (patent family annex) (July 2022)

**EP 4 736 775 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022119446 A
- JP 2017047095 A
- JP 2023106205 A
- JP 2023206277 A
- JP 2023213194 A
- JP 2023207172 A
- JP 2023214037 A
- JP 2023213195 A
- JP 2023210285 A
- JP 2023198526 A
- JP 2024057616 A
- JP 2024063542 A
- JP 2024065612 A